# EUROPEAN PATENT SPECIFICATION

(11) **EP 1 157 000 B1**
(45) Date of publication and mention of the grant of the patent: **02.11.2005**
(21) Application number: 00977094.2
(22) Date of filing: 08.11.2000
(51) Int. Cl.: C07C 229/28, C07C 59/11, C07C 59/62, A61K 31/19, A61P 27/02

(54) **BRANCHED CHAIN AMINO ACID-DEPENDENT AMINOTRANSFERASE INHIBITORS AND THEIR USE IN THE TREATMENT OF DIABETIC RETINOPATHY**
INHIBITORE DER VERZWEIGTE-AMINOSÄURE-ABHÄNGIGEN AMINOTRANSFERASE UND DEREN VERWENDUNG IN DER BEHANDLUNG DER ZUCKERKRANKE RETINOPATHIE
INHIBITEURS D'AMINOTRANSFERASE ACIDES AMINES-DEPENDANTE A CHAINE RAMIFIEE, ET LEUR UTILISATION DANS LE CADRE DU TRAITEMENT DE LA RETINOPATHIE DIABETIQUE

(30) Priority: 08.12.1999 US 169635 P; 11.01.2000 US 175399 P; 05.09.2000 US 230020 P
(43) Date of publication of application: 28.11.2001
(73) Proprietor: Warner-Lambert Company LLC, Morris Plains, NJ 07950 (US); THE PENN STATE RESEARCH FOUNDATION, University Park, PA 16802-7000 (US); WAKE FOREST UNIVERSITY, Winston-Salem, NC 27157 (US)
(72) Inventor: BRYANS, Justin, Stephen, Balsham CB1 6DZ (GB); HU, Lain-Yen, Ann Arbor, MI 48105 (US); HUTSON, Susan, M., Winston-Salem, NC 27101 (US); LANOUE, Kathryn, Foley, Hummelstown, PA 17036 (US); LIETH, Erich, Chapel Hill, CA 27516 (US); RAFFERTY, Michael, Francis, Ann Arbor, MI 48105 (US); RYDER, Todd, Robert, Rochester, NY 14608 (US); SU, Ti-Zhi, Ann Arbor, MI 48105 (US); WELTY, Devin, Franklin, Foothills Ranch, CA 92612 (US); WUSTROW, David, Juergen, Ann Arbor, MI 48103 (US)
(74) Representative: Tesch, Rudolf
(86) International application number: PCT/US2000/030769
(87) International publication number: WO 2001/042191

(56) References cited:
- WO-A-97/33858
- FIELD M J ET AL: "EVALUATION OF GABAPENTIN AND S-(+)-3-ISOBUTYLGABA IN A RAT MODEL OFPOSTOPERATIVE PAIN" JOURNAL OF PHARMACOLOGY AND EXPERIMENTAL THERAPEUTICS,AMERICAN SOCIETY FOR PHARMACOLOGY AND,US, vol. 282, no. 3, September 1997 (1997-09), pages 1242-1246, XP000946613 ISSN: 0022-3565
- BRYANS, J S ET AL: "Identification of Novel Ligands for the Gabpentin Binding Site on the alpha2delta Subunit of a Calcium Channel and Their Evaluation as Anticonvulsant Agents" JOURNAL OF MEDICINAL CHEMISTRY, vol. 41, no. 11, 29 April 1998 (1998-04-29), pages 1838-1845, XP000941901
- GOLDLUST A ET AL: "Effects of anticonvulsant drug gabepentin on the enzymes in metabolic pathways of glutamamte and GABA" EPILEPSY RESEARCH, vol. 22, 1995, pages 1-11, XP000973529
- HUTSON S M ET AL: "Role of Branched-Chain Aminotransferase Isoenzymes and Gabapentin in Neurotransmitter Metabolism" JOURNAL OF NEUROCHEMISTRY, vol. 71, no. 2, 1998, pages 863-874, XP000973532
- TAYLOR C P ET AL: "A summary of mechanistic hypotheses of gabapentin pharmacology" EPILEPSY RESEARCH, vol. 29, no. 3, 1998, pages 233-249, XP000973536

## Description

### BACKGROUND OF THE INVENTION

More than 14 million people in the United States have diabetes. All people with diabetes are at risk of retinal complications. However, people with type I, i.e., insulin-dependent diabetes, face a greater risk of severe vision loss than people with type II, i.e., non-insulin dependent diabetes.

Retinopathy is any non-inflammatory disease of the retina. Diabetic retinopathy is any retinopathy associated with any form of diabetes mellitus.

Initially, the high blood glucose level in diabetic people causes an increase in growth factors in their eyes. This condition is known as the "pre-diabetic retinopathy stage" and can lead to full diabetic retinopathy, if not prophylactically treated.

Retinopathy will affect the majority of diabetic people to some extent during their lifetimes. It is the leading cause of blindness in Americans of age 20 to 74 today, and is expected to impair vision in approximately one-third of diabetic people in the United States. Each year in the United States, as many as 40,000 new cases of blindness occur among diabetic people (CDC, unpublished data, 1993). Diabetic people are 25 times more likely than the general population to become blind due to retinopathy.

Diabetic retinopathy has two stages-a nonproliferative stage, which typically occurs first, and a proliferative stage. The nonproliferative stage, which is also referred to as "background diabetic retinopathy," is characterized by thickening of the basement membrane, loss of retinal pericytes, microvascular abnormalities, intraretinal microaneurysms, retinal hemorrhages (also known as "dot blot" hemorrhages), retinal edema, in particular diabetic macular edema, capillary closure associated with retinal ischemia or poor retinal perfusion (i.e., poor vessel development) and soft and hard exudates. The proliferative stage, which affects an estimated 700,000 Americans (Chen et al., J. Miss. State Med. Assoc. 36(7): 201-208 (1995)), is characterized by neovascularization and fibrovascular growth (i.e., scarring involving glial and fibrous elements) from the retina or optic nerve over the inner surface of the retina or disc or into the vitreous cavity.

The proliferative stage can lead to rubeotic or neovascular glaucoma. Macular edema can occur in either stage and it, along with complications from retinal neovascularization, are the two major retinal problems that cause the diabetes-related vision loss.

While the pathological stages of diabetic retinopathy are well-described, the molecular events underlying diabetic retinopathy are poorly understood. This is due, in part, to the fact that the disease progresses over ten to thirty years, depending on a given individual.

Tight control of glycemia and hypertension and ophthalmic screening of diabetics appears beneficial in preventing the disease. Current treatment consists of regular observation by an ophthalmologist, laser photocoagulation and vitrectomy.

Macular edema threatening or involving the macular center is treated with focal macular photocoagulation. Small (50 mu in diameter), mild-intensity laser burns are targeted at areas of leakage in the macula (Murphy, Amer. Family Physician 51(4): 785-796 (1995)). If the macular edema persists, retreatment may be necessary.

Patients with severe to very severe nonproliferative retinopathy and patients, who are at high risk for proliferative retinopathy or who already have early or advanced proliferative retinopathy, are treated with scatter or panretinal photocoagulation. Panretinal photocoagulation involves 1,500-2,000 laser burns, which are 500 mu in diameter, in the midperipheral and peripheral portion of the retina (Murphy (1995), supra).

The best-documented biochemical mechanism for the development of microvascular complications of diabetes is the sorbitol pathway. In the sorbitol pathway, the enzyme aldose reductase catalyzes the conversion of glucose to sorbitol, and of galactose to galactitol. Aldose reductase has a low substrate affinity for glucose. Accordingly, when glucose concentrations are normal, the pathway is inactive. During hyperglycemia, the sorbitol pathway becomes active. Activation of the sorbitol pathway is important for retinal pericytes, for example, which do not require insulin for glucose penetration. Similarly, retinal capillary cells appear to contain substantial amounts of aldose reductase (Ferris, Hospital Practice: 79-89 (May 15, 1993).

WO 97/33858 relates to substituted cyclic amino acid derivatives that should be considered as useful in the treatment of various neurological disorders.

The Journal of Pharmacology and Experimental Therapeutics of 1997, vol 282, pages 1242 to 1246 by Field M.J. and al. relates to gabapentin that may be effective in the treatment of postoperative pain.

Bryans J.S. and al in J. Med. Chem. vol 41, pages 1838-1845 identify Gabapentin binding sites on the calcium channel in an animal model of epilepsy.

Given the prevalence of diabetic retinopathy, there remains a need for an effective prophylactic and therapeutic treatment of the disease. Accordingly, it is a principal object of the present invention to provide an use of inhibiting compounds for manufacture of pharmaceutical for prophylactically and therapeutically treatment of diabetic retinopathy, including treatment at the pre-diabetic retinopathy stage, the nonproliferative diabetic retinopathy stage, and the proliferative diabetic retinopathy stage. This and other objects of the present invention will become apparent from the detailed description provided herein.

### SUMMARY OF THE INVENTION

The present-invention is directed to the use of an inhibitor of the branched chain amino acid-dependent aminotransferase pathway for the manufacture of pharmaceuticals for the prophylactic and therapeutic treatment of diabetic retinopathy, including treatment at the pre-diabetic retinopathy stage, the nonproliferative diabetic retinopathy stage, and the proliferative diabetic retinopathy stage. The use comprises the administration of an inhibitor of the branched chain amino acid-dependent aminotransferase pathway. Preferably, the inhibitor of the branched chain amino acid-dependent aminotransferase pathway (BCAT) is a compound of Formulas I, II, and/or III: 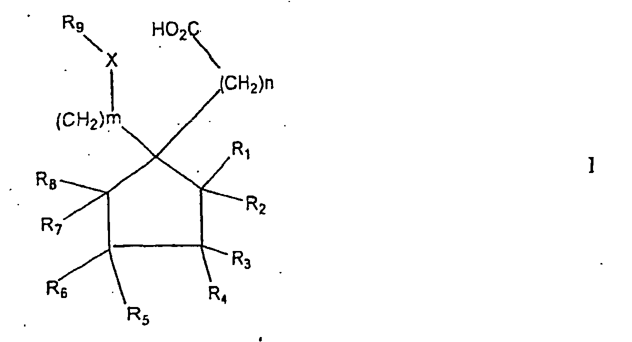 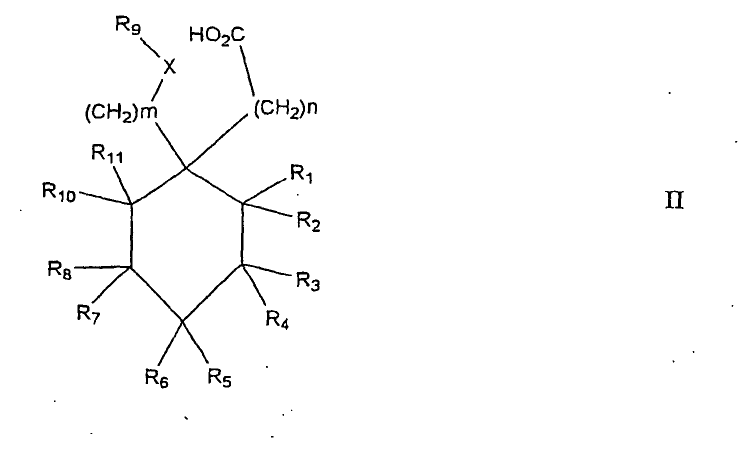 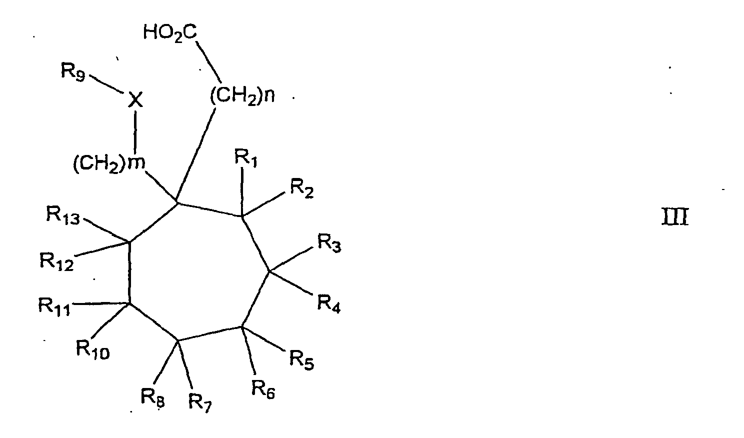 wherein:
R9 is H; alkyl; cycloalkyl; substituted alkyl containing halogen, amine, alkoxy, cycloalkyl, or hydroxy; allyl; alkynyl; alkanoyl; alkoxyalkanoyl; sulfonyl; phenyl; benzyl; or arylalkyl;
m and n are independently an integer of 1-3;
R₁ - R₈ and R₁₀ - R₁₄ are independently H, alkyl, or substituted alkyl; and
X = NR₁₄, O, or S;
or a pharmaceutically acceptable salt, ester, prodrug, or amide thereof

The compounds can have one or more asymmetric carbon atoms and each asymmetric center can be racemic (R & S) or chiral (R or S).

Preferred compounds are those of Formula I, II, or III wherein m and n are 1; X is N_{R14}; R₉ is H; R₄ is methyl; R₄ and R₅ are methyl; R₈ is methyl; R₁₀ is methyl; R₇ and R₈ are methyl; R₄ and R₈ are methyl; R₁-R₈ and R₁₀-R₁₃ are H; R₉ is alkyl; R₉ is benzyl; R₁₄ is alkyl; R₉ is arylalkyl; R₉ is cycloalkyl; R₁-R₈ are H; R₁-R₈ and R₁₀-R₁₁ are H; R₁-R₂ and R₇-R₈ are H; or R₂ is methyl.

More preferred compounds are those of Formula II wherein R₃ is alkyl, R₁-R₂ and R₄-R₁₁ and R₁₄ are hydrogen, and m and n are 1, and X is NR₁₄; R₃ and R₁₁ are alkyl, R₁-R₂ and R₄-R₁₀ and R₁₄ are hydrogen, m and n are 1, and X is NR₁₄; R₃ and R₁₁ are alkyl, R₁-R₂ and R₄-R₁₀ and R₁₄ are hydrogen, m and n are 1, R₉ is alkyl, and X is NR₁₄; and R₁-R₁₁ and R₁₄ are hydrogen, m and n are 1, and X is O.

Especially preferred compounds are selected from:
(1-Allylaminomethyl-cyclohexyl)-acetic acid;
(1-Prop-2-ynylaminomethyl-cyclohexyl)-acetic acid;
{1-[(2,2,2-Trifluoro-ethylamino)-methyl]-cyclohexyl}-acetic acid;
{1-[(3,3,3-Trifluoro-propylamino)-methyl]-cyclohexyl}-acetic acid;
1α,3β,5β- (1-Allylaminomethyl-3,5-dimethyl-cyclohexyl)-acetic acid;
1α,3β,5β-(3,5-Dimethyl-1-prop-2-ynylaminomethyl-cyclohexyl)-acetic acid;
1α,3β,5β-{3,5-Dimethyl-1-[(2,2,2-trifluoro-ethylamino)-methyl]-cyclohexyl}-acetic acid;
1α,3β,5β-{3,5-Dimethyl-1-[(3,3,3-trifluoro-propylamino)-methyl]-cyclohexyl}-acetic acid;
trans-((1R,3R)-1-Allylaminomethyl-3-methyl-cyclohexyl)-acetic acid;
trans-((1R,3R)-3-Methyl-1-prop-2-ynylaminomethyl-cyclohexyl)-acetic acid;
trans-{(1R,3R)-3-Methyl-1-[(2,2,2-trifluoro-ethylamino)-methyl]-cyclohexyl}-acetic acid;
trans-{(1R,3R)-3-Methyl-1-[(3,3,3-trifluoro-propylamino)-methyl]-cyclohexyl}-acetic acid;
trans-{(1R,3R)-3-Methyl-1-[(4,4,4-trifluoro-butylamino)-methyl]-cyclohexyl}-acetic acid;
1α,3β,5β-{3,5-Dimethyl-1-[(4,4,4-trifluoro-butylamino)-methyl]-cyclohexyl}-acetic acid;
1α,3β,5β-{1-[(Cyclopropylmethyl-amino)-methyl]-3,5-dimethyl-cyclohexyl}-acetic acid;
trans-{(1R,3R)-1-[(Cyclopropylmethyl-amino)-methyl]-3-methyl-cyclohexyl}-acetic acid;
trans-((1R,3R)-3-Methyl-1-methylaminomethyl-cyclohexyl)-acetic acid;
trans-((1R,3R)-1-Ethylaminomethyl-3-methyl-cyclohexyl)-acetic acid;
trans-((1R,3R)-3-Methyl-1-propylaminomethyl-cyclohexyl)-acetic acid;
trans-((1R,3R)-1-Butylaminomethyl-3-methyl-cyclohexyl)-acetic acid;
trans-((1R,3R)-1-Hydroxymethyl-3-methyl-cyclohexyl)-acetic acid;
1α,3β,5β-{1-[(Hydroxymethyl-amino)-methyl]-3,5-dimethyl-cyclohexyl}-acetic acid;
1α,3β,5β-(1-Aminomethyl-3,5-diethyl-cyclohexyl)-acetic acid, hydrochloride;
trans-(1R,3R)(1-Aminomethyl-3-methyl-cyclohexyl)-acetic acid, hydrochloride;
(1-Aminomethyl-2-methyl-cyclohexyl)-acetic acid, hydrochloride;
(1-Aminomethyl-3,3-dimethyl-cyclohexyl)-acetic acid, hydrochloride;
(±)-(trans)-(1-Aminomethyl-3,4-dimethyl-cyclopentyl)-acetic acid, hydrochloride;
(cis/trans)-(3R)-(1-Aminomethyl-3-methyl-cyclopentyl)-acetic acid, hydrochloride;
(+)-(trans)-(1-Aminomethyl-3,4-dimethyl-cyclopentyl)-acetic acid, hydrochloride;
(+)-(trans)-(1-Aminomethyl-3,4-dimethyl-cyclopentyl)-acetic acid, hydrochloride;
1α,3β,5β-(1-Aminomethyl-3,5-dimethyl-cyclohexyl)-acetic acid, hydrochloride;
1α,3β,5β-(3,5-Dimethyl-1-propylaminomethyl-cyclohexyl)-acetic acid, hydrochloride salt;
1α,3β,5β-(1-Ethylaminomethyl-3,5-dimethyl-cyclohexyl)-acetic acid, hydrochloride salt;
1α,3β,5β-(1-Benzylamino-methyl)-3,5-dimethyl-cyclohexyl]-acetic acid, hydrochloride salt;
1α,3β,5β-(1-Dimethylaminomethyl-3,5-dimethyl-cyclohexyl)-acetic acid, hydrochloride salt;
1α,3β,5β-(1-Butylaminomethyl-3,5-dimethyl-cyclohexyl)-acetic acid, hydrochloride salt;
1α,3β,5β-{1-[(Benzyl-methyl-amino)-methyl]-3,5-dimethyl-cyclohexyl}-acetic acid, hydrochloride salt;
1α,3β,5β-(3,5-Dimethyl-1-methylaminomethyl-cyclohexyl)-acetic acid, hydrochloride salt;
1α,3β,5β-[1-(Acetylamino-methyl)-3,5-dimethyl-cyclohexyl]-acetic acid;
1α,3β,5β-(1-(Isobutylamino-methyl)-3,5-dimethyl-cyclohexyl]-acetic acid, hydrochloride salt;
1α,3β,5β-[3,5-Dimethyl-1-(phenethylamino-methyl)-cyclohexyl]-acetic acid, hydrochloride salt;
1α,3β,5β-{3,5-Dimethyl-1-[(3-phenyl-propylamino)-methyl]-cyclohexyl}-acetic acid, hydrochloride salt;
{1-[(Cyclobutylmethyl-amino)-methyl]-cyclohexyl}-acetic acid, hydrochloride salt;
1α,3β,5β-[1-(Isopropylamino-methyl)-3,5-dimethyl-cyclohexyl]-acetic acid, hydrochloride salt;
1-Aminomethyl-1-cyclohexane-acetic acid;
1-Aminomethyl-1-cyclopentane-acetic acid;
1-Aminomenthyl-1-cyclopentane-acetic acid, sodium salt;
1-(hydroxymethyl)cyclohexane-acetic acid, sodium salt;
{1-[(2-Methyl-butylamino)-methyl]-cyclohexyl}-acetic acid, hydrochloride salt;
{1-[(4,4,4-Trifluoro-butylamino)-methyl]-cyclohexyl}-acetic acid, hydrochloride salt;
(1-Ethylaminomethyl-cyclohexyl)-acetic acid, hydrochloride salt;
{1-[(Cyclopropylmethyl-amino)-methyl]-cyclohexyl}-acetic acid, hydrochloride salt;
{1-[(2-Hydroxy-1-methyl-ethylamino)-methyl]-cyclohexyl}-acetic acid, hydrochloride salt;
[1-(Isobutylamino-methyl)-cyclohexyl]-acetic acid, hydrochloride salt;
(1-Propylaminomethyl-cyclohexyl)-acetic acid, hydrochloride salt;
[1-(Isopropylamino-methyl)-cyclohexyl]-acetic acid, hydrochloride salt;
(1-Cyclohexylaminomethyl-cyclohexyl)-acetic acid, hydrochloride salt;
[1-(Benzylamino-methyl)-cyclohexyl]-acetic acid, hydrochloride salt;
((1R,3R)-3-Methyl-1-propylaminomethyl-cyclohexyl)-acetic acid, hydrochloride salt;
{1-[(Cyclohexylmethyl-amino)-methyl]-cyclohexyl}-acetic acid, hydrochloric salt;
{1-[(Cyclohexylmethyl-amino)-methyl]-cyclohexyl}-acetic acid, hydrochloric salt;
[1-(*tert*-Butoxycarbonylamino-methyl)-cyclohexyl]-acetic acid;
[1-(Acetylamino-methyl)-cyclohexyl]-acetic acid;
((3R, 5S)-1-Cyclobutylaminomethyl-3,5-dimethyl-cyclohexyl)-acetic acid, hydrochloride salt;
{(3R, 5S)-3,5-Dimethyl-1-[(2-methyl-butylamino)-methyl]-cyclohexyl}-acetic acid, hydrochloride salt;
{(3R, 5S)-1-[(2-Hydroxy-1-methyl-ethylamino)-methyl]-3,5-dimethyl-cyclohexyl}-acetic acid, hydrochloride salt;
{(3R, 5S)-1-[(2,2-Dimethoxy-ethylamino)-methyl]-3,5-dimethyl-cyclohexyl}-acetic acid, hydrochloride salt;
{(3R, 5S)-1-[(Cyclopentylmethyl-amino)-methyl]-3,5-dimethyl-cyclohexyl}-acetic acid, hydrochloride salt;
{(3R,5S)-1-[(Cyclohexylmethyl-amino)-methyl]-3,5-dimethyl-cyclohexyl}-acetic acid, hydrochloride salt;
((3R, 5S)-1-Cyclohexylaminomethyl-3,5-dimethyl-cyclohexyl)-acetic acid, hydrochloride salt;
((3R,5S)-1-Carboxymethyl-3,5-dimethyl-cyclohexyl)-acetic acid;
*trans*-((3R, 5S)-1-Hydroxymethyl-3,5-dimethyl-cyclohexyl)-acetic acid, hydrochloride salt;
*cis*-((3R, 5S)-1-Hydroxymethyl-3,5-dimethyl-cyclohexyl)-acetic acid, hydrochloride salt;
(1-Dimethylaminomethyl-cyclohexyl)-acetic acid, hydrochloride salt;
(1-Butylaminomethyl-cyclohexyl)-acetic acid, hydrochloride salt;
{1-[2,2-Dimethoxy-ethylamino)-methyl]-cyclohexyl}-acetic acid, hydrochloride salt;
(1-methylaminomethyl-cyclohexyl)-acetic acid, hydrochloride salt;
{1-[(Benzyl-methyl-amino)-methyl]-cyclohexyl}-acetic acid, hydrochloride salt;
[1-(Phenethylamino-methyl)-cyclohexyl]-acetic acid, hydrochloride salt;
{1-[(3-Phenyl-propylamino)-methyl]-cyclohexyl}-acetic acid, hydrochloride salt;
((3R, 5S)-1-Hydroxymethyl-3,5-dimethyl-cyclohexyl)-acetic acid, sodium salt;
((3R, 5S)-1-Ethylaminomethyl-3,5-dimethyl-cyclohexyl)-acetic acid, hydrochloride salt;
(1-Aminomethyl-4-ethyl-cyclohexyl)-acetic acid, hydrochloric salt;
(1-Aminomethyl-4-propyl-cyclohexyl)-acetic acid, hydrochloric salt;
((3R, 5S)-3,5-Dimethyl-1-propylaminomethyl-cyclohexyl)-acetic acid, hydrochloride salt;
[(1R, 3R)-1-(Benzylamino-methyl)-3-methyl-cyclohexyl]-acetic acid, hydrochloride salt;
{(1R, 3R)-1-[(Benzyl-methyl-amino)-methyl]-3-methyl-cyclohexyl}-acetic acid, hydrochloride salt; and
((1R, 3R)-3-Methyl-1-methylaminomethyl-cyclohexyl)-acetic acid, hydrochloride salt;

Several of the compounds described by Formulas I, II, and III are known.
Compounds of Formula A: 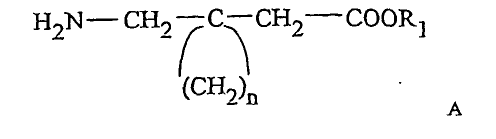 wherein R₁ is hydrogen or a lower alkyl radical and n is 4, 5, or 6 are known in United States Patent Number 4,024,175 and its divisional United States Patent Number 4,087,544. The uses disclosed are: protective effect against cramp induced by thiosemicarbazide; protective action against cardiazole cramp; the cerebral diseases, epilepsy, faintness attacks, hypokinesia, and cranial traumas; and improvement in cerebral functions. The compounds are useful in geriatric patients. The patents are hereby incorporated by reference.
Compounds of Formula B: 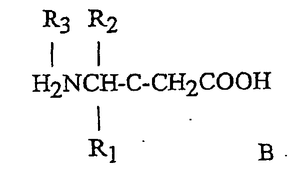 or a pharmaceutically acceptable salt thereof wherein R₁ is a straight or branched alkyl group having from 1 to 6 carbon atoms, phenyl or cycloalkyl having from 3 to 6 carbon atoms; R₂ is hydrogen or methyl; and R₃ is hydrogen, or carboxyl are known in United States Patent Number 5,563,175 and its various divisionals. These patents are hereby incorporated by reference.

The present invention also discloses novel compounds of the Formula IV 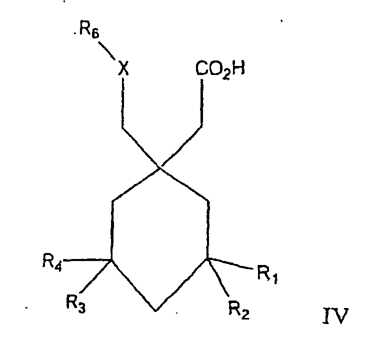 R₁-R₄ are hydrogen or alkyl;
X is NR₅ or O;
R₅ is hydrogen or alkyl;
R₆ is alkyl, benzyl, alkoxyalkanoyl, arylalkyl, alkoxy, cycloalkyl, allyl, alkylcycloalkyl, trisubstituted halogenalkyl, and wherein R1-R4 are each hydrogen then R₆ is not hydrogen or methyl; or a pharmaceutically acceptable salt, ester, prodrug, or amide thereof . Like the comounds of Formulas I, II, and III, the compounds are useful for the prophylactic and therapeutic treatment of diabetic retiriopathy, including treatment at the pre-diabetic retinopathy stage, the nonproliferative diabetic retinopathy stage, and the proliferative diabetic retinopathy stage.

Preferred compounds are those of Formula IV wherein R₂ and R₄ are hydrogen and R₁ and R₃ are alkyl; R₂ and R₄ are hydrogen and R₁ and R₃ are methyl; R₁-R₄ are hydrogen; R₁ is alkyl and R₂-R₄ are hydrogen; R₁ is methyl and R₂-R₄ are hydrogen; R₅ is hydrogen; X is O; R₆ is alkyl; R₆ is benzyl; R₆ is phenylalkyl; R₆ is cycloalkyl; R₆ is trifluoroalkyl; R₆ is alkylcycloalkyl; R₆ is alkoxy; and R₆ is allyl.

Other preferred compounds are those of Formula IV wherein R₂ and R₄ are hydrogen and R₁ and R₃ are methyl; R₁-R₄ are hydrogen; R₁ is methyl and R₂-R₄ are hydrogen; R₅ is hydrogen; R₆ is alkyl; R₆ is benzyl; R₆ is phenylalkyl; R₆ is cycloalkyl; R₆ is trifluoroalkyl; R₆ is alkylcycloalkyl; R₆ is alkoxy; and R₆ is allyl.

Especially preferred compounds are selected from:
(1-Allylaminomethyl-cyclohexyl)-acetic acid;
(1-Prop-2-ynylaminomethyl-cyclohexyl)-acetic acid;
{1-[(2,2,2-Trifluoro-ethylamino)-methyl]-cyclohexyl}-acetic acid;
{1-[(3,3,3-Trifluoro-propylamino)-methyl]-cyclohexyl}-acetic acid;
1α,3β,5β-(1-Allylaminomethyl-3,5-dimethyl-cyclohexyl)-acetic acid;
1α,3β,5β-(3,5-Dimethyl-1-prop-2-ynylaminomethyl-cyclohexyl)-acetic acid;
1α,3β,5β-{3,5-Dimethyl-1-[(2,2,2-trifluoro-ethylamino)-methyl]-cyclohexyl}-acetic acid;
1α,3β,5β-{3,5-Dimethyl-1-[(3,3,3-trifluoro-propylamino)-methyl]-cyclohexyl}-acetic acid;
trans-((1R,3R)-1-Allylaminomethyl-3-methyl-cyclohexyl)-acetic acid;
trans-((1R,3R)-3-Methyl-1-prop-2-ynylaminomethyl-cyclohexyl)-acetic acid;
trans-{(1R,3R)-3-Methyl-1-[(2,2,2-tifluoro-ethylamino)-methyl]-cyclohexyl}-acetic acid;
trans-{(1R,3R)-3-Methyl-1-[(3,3,3-trifluoro-propylamino)-methyl]-cyclohexyl}-acetic acid;
trans-{(1R,3R)-3-Methyl-1-[(4,4,4-trifluoro-butylamino)-methyl]-cyclohexyl}-acetic acid;
1α,3β,5β-{3,5-Dimethyl-1-[(4,4,4-trifluoro-butylamino)-methyl]-cyclohexyl}-acetic acid;
1α,3β,5β-{1-[(Cyclopropylmethyl-amino)-methyl]-3,5-dimethyl-cyclohexyl}-acetic acid;
trans-{(1R,3R)-1-[(Cyclopropylmethyl-amino)-methyl]-3-methyl-cyclohexyl}-acetic acid;
trans-((1R,3R)-3-Methyl-1-methylaminomethyl-cyclohexyl)-acetic acid;
trans-((1R,3R)-1-Ethylaminomethyl-3-methyl-cyclohexyl)-acetic acid;
trans-((1R,3R)-3-Methyl-1-propylaminomethyl-cyclohexyl)-acetic acid;
trans-((1R,3R)-1-Butylaminomethyl-3-methyl-cyclohexyl)-acetic acid;
trans-((1R,3R)-1-Hydroxymethyl-3-methyl-cyclohexyl)-acetic acid;
1α,3β,5β-{1-[(Hydroxymethyl-amino)-methyl]-3,5-dimethyl-cyclohexyl}-acetic acid;
1α,3β,5β-(1-Aminomethyl-3,5-diethyl-cyclohexyl)-acetic acid, hydrochloride;
1α,3β,5β-(3,5-Dimethyl-1-propylaminomethyl-cyclohexyl)-acetic acid, hydrochloride salt;
1α,3β,5β-(1-Ethylaminomethyl-3,5-dimethyl-cyclohexyl)-acetic acid, hydrochloride salt;
1α,3β,5β-[(1-Benzylamino-methyl)-3,5-dimethyl-cyclohexyl]-acetic acid, hydrochloride salt;
1α,3β,5β-(1-Dimethylaminomethyl-3,5-dimethyl-cyclohexyl)-acetic acid, hydrochloride salt;
1α,3β,5β-(1-Butylaminomethyl-3,5-dimethyl-cyclohexyl)-acetic acid, hydrochloride salt;
1α,3β,5β-{1-[(Benzyl-methyl-amino)-methyl]-3,5-dimethyl-cyclohexyl}-acetic acid, hydrochloride salt;
1α,3β,5β-(3,5-Dimethyl-1-methylaminomethyl-cyclohexyl)-acetic acid, hydrochloride salt;
1α,3β,5β-[1-(Acetylamino-methyl)-3,5-dimethyl-cyclohexyl]-acetic acid;
1α,3β,5β-[1-(Isobutylamino-methyl)-3,5-dimethyl-cyclohexyl]-acetic acid, hydrochloride salt;
1α,3β,5β-[3,5-Dimethyl-1-(phenethylamino-methyl)-cyclohexyl]-acetic acid, hydrochloride salt;
1α,3β,5β-{3,5-Dimethyl-1-[(3-phenyl-propylamino)-methyl]-cyclohexyl}-acetic acid, hydrochloride salt;
{1-[(Cyclobutylmethyl-amino)-methyl]-cyclohexyl}-acetic acid, hydrochloride salt;
1α,3β,5β-[1-(Isopropylamino-methyl)-3,5-dimethyl-cyclohexyl]-acetic acid, hydrochloride salt;
{1-[(2-Methyl-butylamino)-methyl]-cyclohexyl}-acetic acid, hydrochloride salt;
{1-[(4,4,4-Trifluoro-butylamino)-methyl]-cyclohexyl}-acetic acid, hydrochloride salt;
(1-Ethylaminomethyl-cyclohexyl)-acetic acid, hydrochloride salt;
{1-[(Cyclopropylmethyl-amino)-methyl]-cyclohexyl}-acetic acid, hydrochloride salt;
{1-[(2-Hydroxy-1-methyl-ethylamino)-methyl]-cyclohexyl}-acetic acid, hydrochloride salt;
[1-(Isobutylamino-methyl)-cyclohexyl]-acetic acid, hydrochloride salt;
(1-Propylaminomethyl-cyclohexyl)-acetic acid, hydrochloride salt;
[1-(Isopropylamino-methyl)-cyclohexyl]-acetic acid, hydrochloride salt;
(1-Cyclohexylaminomethyl-cycloheayl)-acetic acid, hydrochloride salt;
[1-(Benzylamino-methyl)-cyclohexyl]-acetic acid, hydrochloride salt;
((1R,3R)-3-Methyl-1-propylaminomethyl-cyclohexyl)-acetic acid, hydrochloride salt;
{1-[Cyclopentylmethyl-amino)-methyl]-cyclohexyl}-acetic acid, hydrochloric salt;
{1-[(Cyclohexylmethyl-amino)-methyl]-cyclohexyl}-acetic acid, hydrochloric salt;
[1-(*tert*-Butoxycarbonylamino-methyl)-cyclohexyl]-acetic acid;
[1-(Acetylamino-methyl)-cyclohexyl]-acetic acid;
((3R, 5S)-1-Cyclobutylaminomethyl-3,5-dimethyl-cyclohexyl)-acetic acid, hydrochloride salt;
{(3R, 5S)-3,5-Dimethyl-1-[(2-methyl-butylamino)-methyl]-cyclohexyl}-acetic acid, hydrochloride salt;
{(3R, 5S)-1-[(2-Hydroxy-1-methyl-ethylamino)-methyl]-3,5-dimethyl-cyclohexyl}-acetic acid, hydrochloride salt;
{(3R, 5S)-1-[(2,2-Dimethoxy-ethylamino)-methyl]-3,5-dimethyl-cyclohexyl}-acetic acid, hydrochloride salt;
{(3R, 5S)-1-[(Cyclopentylmethyl-amino)-methyl]-3,5-dimethyl-cyclohexyl}-acetic acid, hydrochloride salt;
{(3R,5S)-1-[(Cyclohexylmethyl-amino)-methyl]-3,5-dimethyl-cyclohexyl}-acetic acid, hydrochloride salt;
((3R, 5S)-1-Cyclohexylaminomethyl-3,5-dimethyl-cyclohexyl)-acetic acid, hydrochloride salt;
((3R,5S)-1-Carboxymethyl-3,5-dimethyl-cyclohexyl)-acetic acid;
*trans*-((3R, 5S)-1-Hydroxymethyl-3,5-dimethyl-cyclohexyl)-acetic acid, hydrochloride salt;
*cis*-((3R, 5S)-1-Hydroxymethyl-3,5-dimethyl-cyclohexyl-acetic acid, hydrochloride salt;
(1-Dimethylaminomethyl-cyclohexyl)-acetic acid, hydrochloride salt;
(1-Butylaminomethyl-cyclohexyl)-acetic acid, hydrochloride salt;
{1-[2,2-Dimethoxy-ethylamino)-methyl]-cyclohexyl}-acetic acid, hydrochloride salt;
(1-methylaminomethyl-cyclohexyl)-acetic acid, hydrochloride salt;
{1-[(Benzyl-methyl-amino)-methyl]-cyclohexyl}-acetic acid, hydrochloride salt;
[1-(Phenethylamino-methyl)-cyclohexyl]-acetic acid, hydrochloride salt;
{1-[(3-Phenyl-propylamino)-methyl]-cyclohexyl}-acetic acid, hydrochloride salt;
((3R, 5S)-1-Hydroxymethyl-3,5-dimethyl-cyclohexyl)-acetic acid, sodium salt;
((3R, 5S)-1-Ethylaminomethyl-3,5-dimethyl-cyclohexyl)-acetic acid, hydrochloride salt;
(1-Aminomethyl-4-ethyl-cyclohexyl)-acetic acid, hydrochloric salt;
(1-Aminomethyl-4-propyl-cyclohexyl)-acetic acid, hydrochloric salt;
((3R, 5S)-3,5-Dimethyl-1-propylaminomethyl-cyclohexyl)-acetic acid, hydrochloride salt;
[(1R, 3R)-1-(Benzylamino-methyl)-3-methyl-cyclohexyl]-acetic acid, hydrochloride salt;
{(1R, 3R)-1-[(Benzyl-methyl-amino)-methyl]-3-methyl-cyclohexyl}-acetic acid, hydrochloride salt;
   and
((1R, 3R)-3-Methyl-1-methylaminomethyl-cyclohexyl)-acetic acid, hydrochloride salt;

The present invention is also a pharmaceutical composition comprising a therapeutically effective amount of one or more compounds of Formula IV and a pharmaceutically acceptable carrier.

Compounds of Formula II, such as gabapentin, are useful for treating various conditions such as neurodegenerative disorder (US 5,084.479), depression (US 5,025,035), anxiety/panic (US 5,792,796), mania/bipolar disorders (US 5,510,381), anti-inflammatory diseases (WO 98 158641), gastrointestinal damage (WO 99 108670), glaucoma, and pain. Because the compounds of Formula II and Formula IV both operate to affect the BCAT pathway and affect the synthesis of glutamate, which is involved in each of the aforementioned indications, the compounds of Formula IV will also be useful for treating the indications. Thus the present invention is also directed to the use of the compounds of Formula IV for the manufacture of pharmaceuticals to treat each or more than one of neurodegenerative disorders (including but not limited to ALS, Parkinson's disease and Alzheimer's disease), depression, anxiety/panic, mania/bipolar disorders, anti-inflammatory diseases, glaucoma pain, or gastrointestinal damage.

The present invention also provides the use of a compound of formula I, II and/or III: 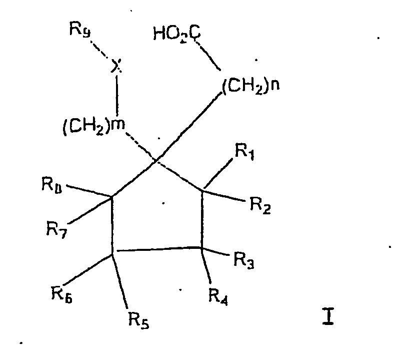 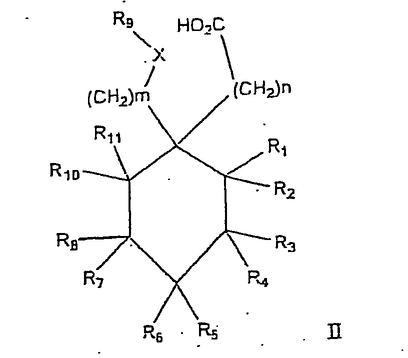 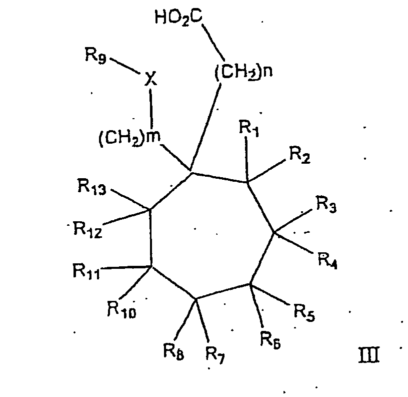 wherein:
R₉ is H; alkyl; cycloalkyl; substituted alkyl containing halogen, amine, alkoxy, cycloalkyl, or hydroxy; allyl; alkynyl; alkanoyl; alkoxyalkanoyl; sulfonyl; phenyl; benzyl; or arylalkyl;
and the proviso that R₉ cannot be hydrogen or alkanoyl for compounds of formula II; m and n are independently an integer of 1-3;
R₁ - R₈ and R₁₀ - R₁₄ are independently H, alkyl, or substituted alkyl, and
X = NR₁₄, O, or S
where there is more than one stereoisomer, each chiral center may be independently R or S; or a pharmaceutically acceptable salt ester, prodrug, or amide thereof for the manufacture of a pharmaceutical for inhibiting the branch chain amino acid-dependent aminotransferase.

### BRIEF DESCRIPTION OF THE DRAWINGS

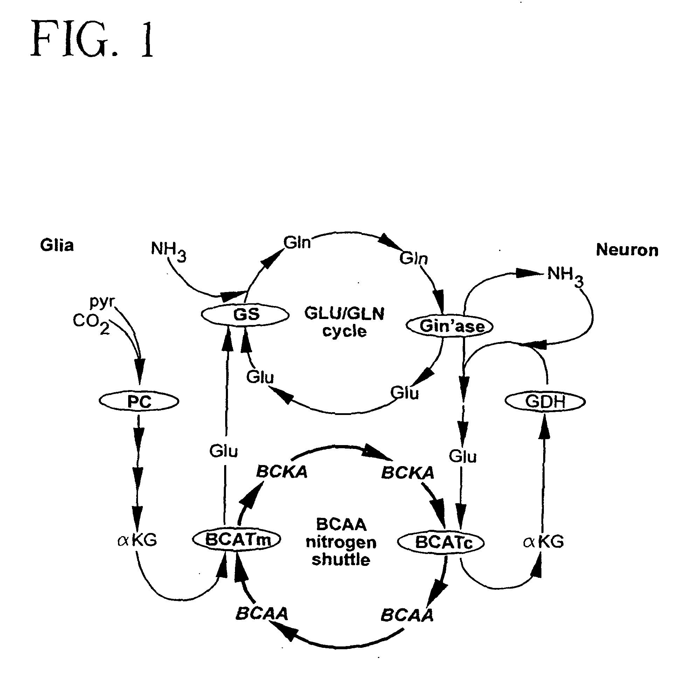

**Figure 1.** Scheme showing the BCAT shuttle in the retina. The shuttle proper is highlighted and shows how branched chain amino acids in the glia can transaminate α-ketoglutarate to form glutamate. The BCKA product diffuses across the interstitium (shown as grey) to the neurons where it is converted back to the branched chain amino acid by transamination with glutamate via BCATc. Abbreviations are as follows: BCAA=branched chain amino acid; GLU=glutamate; GLN=glutamine; Gln'ase=glutaminase; pyr=pyruvate; PC=pyruvate carboxylase, αKG=α-ketoglutarate, BCATm=mitochondrial branched chain aminotransferase; BCATc=cytosolic branclied chain aminotransferase; BCKA=branched chain keto acid; GDH=glutamate dehydrogenase.

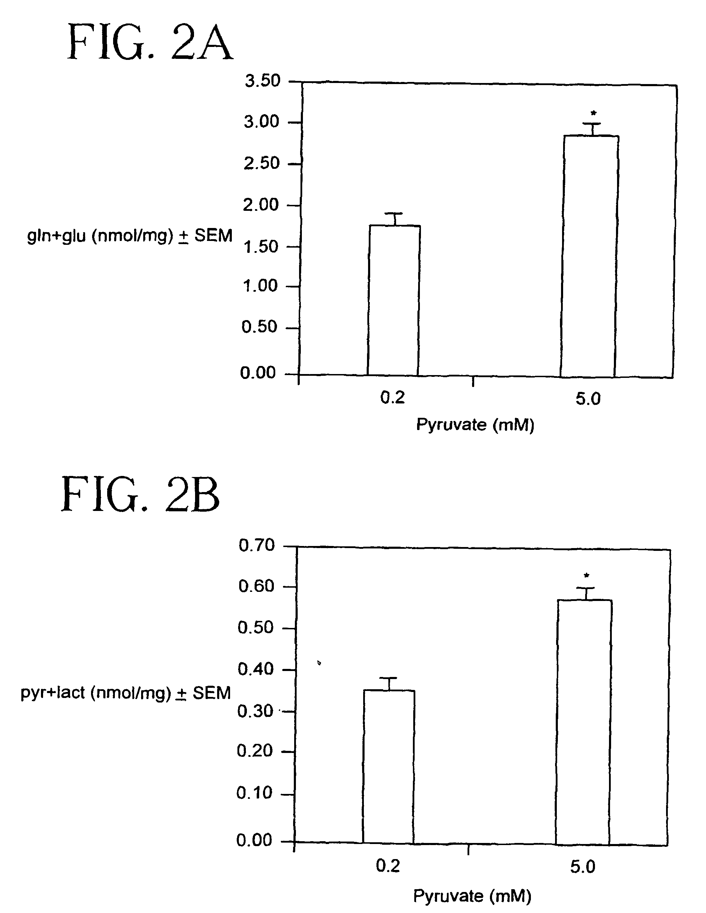

**Figure 2.** Effect ofpyruvate concentration on incorporation of H¹⁴CO₃ into glutamate plus glutamine (A) and pyruvate plus lactate (B) by *ex vivo* retinas. Hemi-retinas were incubated at 37°C under standard conditions with 25 mM H¹⁴CO₃ for 20 minutes. Pyruvate in the medium was either 0.2 or 5 mM. Values shown are mean ± SEM (n=4, * p<0.05). Abrevs: gln+glu = sum of labeled glutamine plus glutamate; pyr+lact = sum of labeled pyruvate plus lactate.

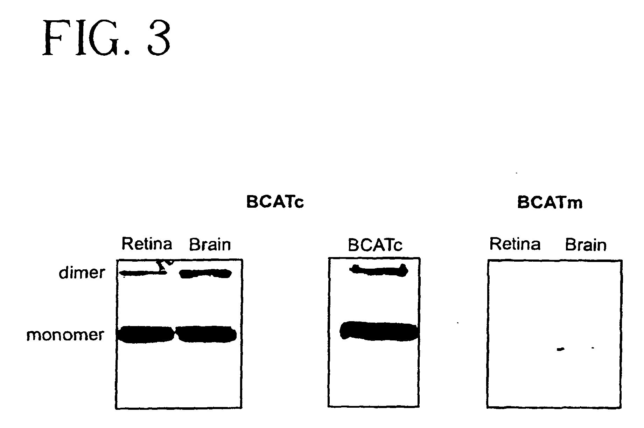

**Figure 3.** Expression of BCAT isoenzymes in rat retina. Tissue extraction, SDS-PAGE, and immunoblotting were performed as described using the ECL detection kit. Immunoaffinity purified rat anti-BCATc peptide antibody (1:1000) and human anti-BCATm antibody (1:1000) were used.

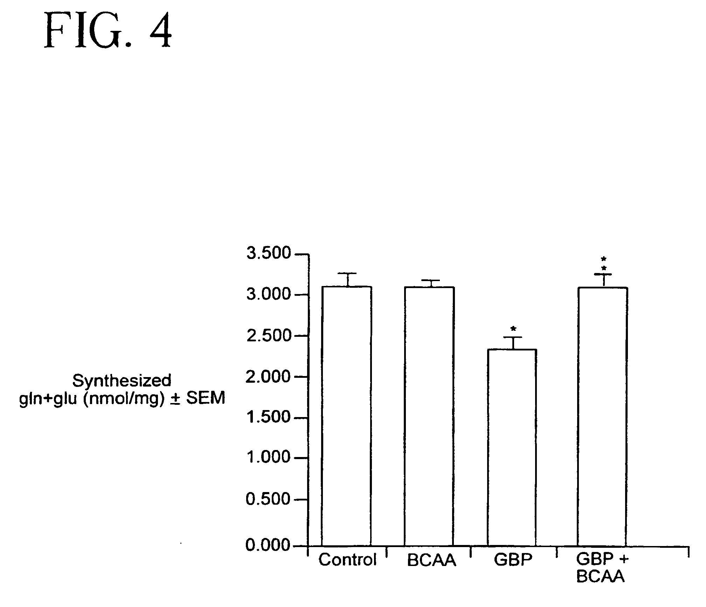

**Figure 4.** The influence of gabapentin (GBP) and branched chain amino acids on incorporation of H¹⁴CO₃ into glutamate plus glutamine by *ex vivo* retinas. Half retinas were incubated at 37° C under standard conditions with 25 mM H¹⁴CO₃ and 0.2 mM pyruvate for 20 minutes. In some instances gabapentin (1 mM) was included in the incubation medium and in others GBP (1 mM) plus all three branched amino acids (0.2 mM) each were included. Values shown are means ± SEM of HCO₃ incorporated into glutamate plus glutamine in 20 minutes (n=4, * p<0.001, ** p<0.01). Abrevs: gln+glu = sum of labeled glutamine plus glutamate.

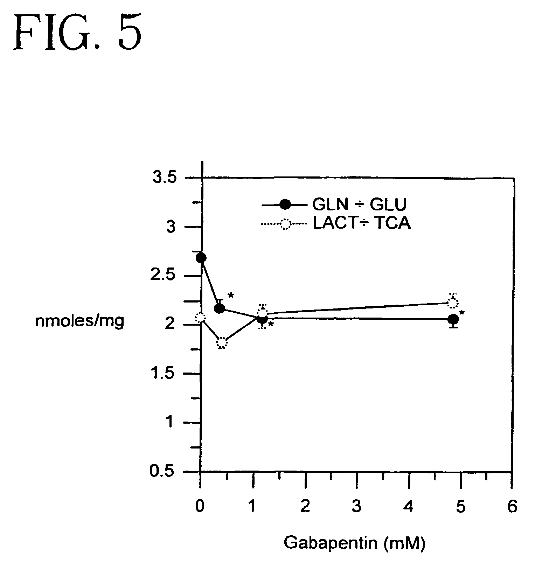

**Figure 5.** Influence of gabapentin (GBP) concentration on incorporation of H¹⁴CO₃ into glutamate and glutamine in *ex vivo* retinas. Conditions were similar to those of Figure 4 except for the concentration of GBP. Values shown are means ± SEM (n=6-7, * p<0.05). Abrevs: GLN+GLU = sum of glutamine plus glutamate; LACT + TCA = sum of lactate plus TCA cycle intermediates.

### DETAILED DESCRIPTION OF THE INVENTION

The present invention is predicated on the discovery that intraocular glutamate injection causes ganglion cell loss which is related to the early stages of diabetic retinopathy (Vorwerk et al. IOVS 37:1618-1624 (1996)) and that an inhibitor of the branched chain amino acid-dependent aminotransferase pathway, such as gabapentin for example, is effective in inhibiting the synthesis of glutamate, thus preventing diabetic retinopathy. Accordingly, the present invention provides the use of inhibiting compounds for the manufacture of pharmaceuticals for the prophylactic and therapeutic treatment of diabetic retinopathy, including treatment at the pre-diabetic retinopathy stage, the nonproliferative diabetic retinopathy stage, and the proliferative diabetic retinopathy stage. By "prophylactic" is meant the protection, in whole or in part, against diabetic retinopathy, in particular diabetic macular edema. By "therapeutic" is meant the amelioration of diabetic retinopathy, itself, and the protection, in whole or in part, against further diabetic retinopathy, in particular diabetic macular edema.

The use comprises the administration of an inhibitor of the branched chain amino acid-dependent aminotransferase pathway in an amount sufficient to treat the retina for retinopathy prophylactically or therapeutically. Any inhibitor of the branched chain amino acid-dependent aminotransferase pathway can be used in the use of the present invention as long as it is safe and efficacious. Herein, "branch chain amino acid-dependent aminotransferase (BCAT) inhibitor" will be used to refer to such compounds and is intended to encompass all compounds that affect the branch chain amino acid-dependent aminotransferase pathway at any and all points in the pathway.

The BCAT inhibitor is a compound of Formulas I, II, and/or III, as described above, or a pharmaceutically acceptable BCAT pathway-inhibiting analogue or prodrug thereof, or a pharmaceutically acceptable salt, ester, or amide of any of the foregoing.

Unless otherwise expressly stated, the following definitions are adhered to throughout this disclosure.

"Alkyl" means a straight or branched hydrocarbon radical having from 1 to 10 carbon atoms (unless stated otherwise) and includes, for example, methyl, ethyl, n-propyl, isopropyl, n-butyl, sec-butyl, isobutyl, tert-butyl, n-pentyl, iso-pentyl, n-hexyl, and the like.

"Halogen" includes fluoro, chloro, bromo, and iodo.

"Alkenyl" means straight and branched hydrocarbon radicals having from 2 to 6 carbon atoms and one double bond and includes ethenyl, 3-buten-1-yl, 2-ethenylbutyl, 3-hexen-1-yl, and the like.

"Alkynyl" means straight and branched hydrocarbon radicals having from 2 to 6 carbon atoms and one triple bond and includes ethynyl, 3-butyn-1-yl, propynyl, 2-butyn-1-yl, 3-pentyn-1-yl, and the like.

"Cycloalkyl" means a monocyclic or polycyclic hydrocarbyl group such as cyclopropyl, cycloheptyl, cyclooctyl, cyclodecyl, cyclobutyl, adamantyl, norpinanyl, decalinyl, norbornyl, cyclohexyl, and cyclopentyl. Such groups can be substituted with groups such as hydroxy, keto, and the like. Also included are rings in which 1 to 3 heteroatoms replace carbons. Such groups are termed "heterocyclyl", which means a cycloalkyl group also bearing at least one heteroatom selected from O, S, or NR₂, examples being substituted or unsubstituted oxiranyl, pyrrolidinyl, piperidyl, tetrahydropyran, piperazinyl, acylpiperazinyl, pyrrolidinyl, and morpholine.

"Alkoxy" refers to the alkyl groups mentioned above bound through oxygen, examples of which include methoxy, ethoxy, isopropoxy, tert-butoxy, and the like. In addition, alkoxy refers to polyethers such as -O-(CH₂)₂-O-CH₃, and the like.

"Alkanoyl" groups are alkyl linked through a carbonyl, ie, C₁-C₅-C(O)-. Such groups include formyl, acetyl, propionyl, butyryl, and isobutyryl.

"Acyl" means an alkyl or aryl (Ar) group bonded through a carbonyl group, ie, R-C(O)-. For example, acyl includes a C₁-C₆ alkanoyl, including substituted alkanoyl, wherein the alkyl portion can be substituted by NR⁴R⁵ or a carboxylic or heterocyclic group. Typical acyl groups include acetyl, benzoyl, and the like.

The alkyl, alkenyl, alkoxy, and alkynyl groups described above are optionally substituted, preferably by 1 to 3 groups selected from NR⁴R⁵, phenyl, substituted phenyl, thio C₁-C₆ alkyl, C₁-C₆ alkoxy, hydroxy, carboxy, C₁-C₆ alkoxycarbonyl, halo, nitrile, cycloalkyl, and a 5- or 6-membered carbocyclic ring or heterocyclic ring having 1 or 2 heteroatoms selected from nitrogen, substituted nitrogen, oxygen, and sulfur. "Substituted nitrogen" means nitrogen bearing C₁-C₆ alkyl or (CH₂)ₙPh where n is 1, 2, or 3. Perhalo and polyhalo substitution is also embraced.

Examples of substituted alkyl groups include 2-aminoethyl, pentachloroethyl, trifluoromethyl, 2-diethylaminoethyl, 2-dimethylaminopropyl, ethoxycarbonylmethyl, 3-phenylbutyl, methanylsulfanylmethyl, methoxymethyl, 3-hydroxypentyl, 2-carboxybutyl, 4-chlorobutyl, 3-cyclopropylpropyl, pentafluoroethyl, 3-morpholinopropyl, piperazinylmethyl, and 2-(4-methylpiperazinyl)ethyl.

Examples of substituted alkynyl groups include 2-methoxyethynyl, 2-ethylsulfanyethynyl, 4-(1-piperazinyl)-3-(butynyl), 3-phenyl-5-hexynyl, 3-diethylamino-3-butynyl, 4-chloro-3-butynyl, 4-cyclobutyl-4-hexenyl, and the like.

Typical substituted alkoxy groups include aminomethoxy, trifluoromethoxy, 2-diethylaminoethoxy, 2-ethoxycarbonylethoxy, 3-hydroxypropoxy, 6-carboxhexyloxy, and the like.

Further, examples of substituted alkyl, alkenyl, and alkynyl groups include dimethylaminomethyl, carboxymethyl, 4-dimethylamino-3-buten-1-yl, 5-ethylmethylamino-3-pentyn-1-yl, 4-morpholinobutyl, 4-tetrahydropyrinidylbutyl, 3-imidazolidin-1-ylpropyl, 4-tetrahydrothiazol-3-yl-butyl, phenylmethyl, 3-chlorophenylmethyl, and the like.

The terms "Ar" and "aryl" refer to unsubstituted and substituted aromatic groups. Heteroaryl groups have from 4 to 9 ring atoms, from 1 to 4 of which are independently selected from the group consisting of O, S, and N. Preferred heteroaryl groups have 1 or 2 heteroatoms in a 5- or 6-membered aromatic ring. Mono and bicyclic aromatic ring systems are included in the definition of aryl and heteroaryl. Typical aryl and heteroaryl groups include phenyl, 3-chlorophenyl, 2,6-dibromophenyl, pyridyl, 3-methylpyridyl, benzothienyl, 2,4,6-tribromophenyl, 4-ethylbenzothienyl, furanyl, 3,4-diethylfuranyl, naphthyl, 4,7-dichloronaphthyl, pyrrole, pyrazole, imidazole, thiazole, and the like.

Preferred Ar groups are phenyl and phenyl substituted by 1, 2, or 3 groups independently selected from the group consisting of alkyl, alkoxy, thio, thioalkyl, hydroxy, carbonyl groups, amino of the formula -NR⁴R⁵, and T(CH₂)ₘQR₄ or T(CH₂)ₘCO₂R⁴ wherein m is 1 to 6, T is O, S, NR⁴, N(O)R⁴, NR⁴R⁶Y, or CR⁴R⁵, Q is O, S, NR⁵, N(O)R⁵, or NR⁵R⁶Y wherein R⁴ and R⁵ are as described above, and R⁷ is alkyl or substituted alkyl, for example, methyl, trichloroethyl, diphenylmethyl, and the like. The alkyl and alkoxy groups can be substituted as defined above. For example, typical groups are carboxyalkyl, alkoxycarbonylalkyl, hydroxyalkyl, hydroxyalkoxy, and alkoxyalkyl.

The symbol "-" means a bond.

The term "patient" means all animals including humans. Examples of patients include humans, cows, dogs, cats, goats, sheep, and pigs.

The compounds of the present invention can exist in unsolvated forms as well as solvated forms, including hydrated forms. In general, the solvated forms, including hydrated forms, are equivalent to unsolvated forms and are intended to be encompassed within the scope of the present invention.

Certain of the compounds of the present invention possess one or more chiral centers and each center may exist in the R(D) or S(L) configuration. The present invention includes all enantiomeric and epimeric forms as well as the appropriate mixtures thereof

The compounds of Formulas I, II, and III are capable of further forming both pharmaceutically acceptable analogs comprising salts, esters, amides, and prodrugs. As used herein, the term "pharmaceutically acceptable salts, esters, amides, and prodrugs" refers to those carboxylate salts, amino acid addition salts, esters, amides, and prodrugs of the compounds of the present invention which are, within the scope of sound medical judgment, suitable for use in contact with the tissues of patients without undue toxicity, irritation, allergic response, and the like, commensurate with a reasonable benefit/risk ratio, and effective for their intended use, as well as the zwitterionic forms, where possible, of the compounds of the invention. The term "salts" refers to the relatively non-toxic, inorganic and organic acid addition salts of compounds of the present invention. These salts can be prepared *in situ* during the final isolation and purification of the compounds or by separately reacting the purified compound in its free base form with a suitable organic or inorganic acid and isolating the salt thus formed and including, but not limited to, acid addition and/or base salts, solvents and N-oxides of a compound of Formulas I, II, and III. This invention also provides pharmaceutical formulations comprising a compound of Formulas I, II, or III together with a pharmaceutically acceptable carrier, diluent, or excipient therefor. All of these forms are within the present invention.

Pharmaceutically acceptable acid addition salts of the compounds of Formulas I, II and III include salts derived form inorganic acids such as hydrochloric, nitric, phosphoric, sulfuric, hydrobromic, hydriodic, phosphorus, and the like, as well as the salts derived from organic acids, such as aliphatic mono- and dicarboxylic acids, phenyl-substituted alkanoic acids, hydroxy alkanoic acids, alkanedioic acids, aromatic acids, aliphatic and aromatic sulfonic acids, etc. Such salts thus include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, acetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, toluenesulfonate, phenylacetate, citrate, lactate, maleate, tartrate, methanesulfonate, and the like. Also contemplated are the salts of amino acids such as arginate, gluconate, galacturonate, and the like; see, for example, Berge, et al., "Pharmaceutical Salts," *J. of Pharmaceutical Science*, 1977;66:1-19.

The acid addition salts of the basic compounds are prepared by contacting the free base form with a sufficient amount of the desired acid to produce the salt in the conventional manner. The free base form may be regenerated by contacting the salt form with a base and isolating the free base in the conventional manner. The free base forms differ from their respective salt forms somewhat in certain physical properties such as solubility in polar solvents, but otherwise the salts are equivalent to their respective free base for purposes of the present invention.

Pharmaceutically acceptable base addition salts are formed with metals or amines, such as alkali and alkaline earth metal hydroxides, or of organic amines. Examples of metals used as cations are sodium, potassium, magnesium, calcium, and the like. Examples of suitable amines are N,N'-dibenzylethylenediamine, chloroprocaine, choline, diethanolamine, ethylenediamine, N-methylglucamine, and procaine; see, for example, Berge, et al., supra.

The base addition salts of acidic compounds are prepared by contacting the free acid form with a sufficient amount of the desired base to produce the salt in the conventional manner. The free acid form may be regenerated by contacting the salt form with an acid and isolating the free acid in a conventional manner. The free acid forms differ from their respective salt forms somewhat in certain physical properties such as solubility in polar solvents, but otherwise the salts are equivalent to their respective free acid for purposes of the present invention.

Examples of pharmaceutically acceptable, non-toxic esters of the compounds of this invention include C₁-C₆alkyl esters wherein the alkyl group is a straight or branched chain. Acceptable esters also include C₅-C₇cycloalkyl esters as well as arylalkyl esters such as, but not limited to benzyl. C₁-C₄alkyl esters are preferred. Esters of the compounds of the present invention may be prepared according to conventional methods.

Examples of pharmaceutically acceptable, non-toxic amides of the compounds of this invention include amides derived from ammonia, primary C₁-C₆alkyl amines and secondary C₁-C₆dialkyl amines wherein the alkyl groups are straight or branched chain. In the case of secondary amines the amine may also be in the form of a 5- or 6-membered heterocycle containing one nitrogen atom. Amides derived from ammonia, C₁-C₃alkyl primary amines, and C₁-C₂dialkyl secondary amines are preferred. Amides of the compounds of the invention may be prepared according to conventional methods.

The term "prodrug" refers to compounds that are rapidly transformed *in vivo* to yield the parent compound of the above Formulae, for example, by hydrolysis in blood. A thorough discussion is provided in T. Higuchi and V Stella, "Pro-drugs as Novel Delivery Systems," Vol 14 of the A.C.S. Symposium Series, and in *Bioreversible Carriers in Drug Design*, ed. Edward B. Roche, American Pharmaceutical Association and Pergamon Press, 1987, both of which are incorporated herein by reference. In general, a prodrug is a drug which has been chemically modified and may be biologically inactive at its site of action, but which may be degraded or modified by one or more enzymatic or other in vivo processes to the parent bioactive form.

A therapeutically effective amount is an amount of a compound of Formulas I, II, or III, that when administered to a patient, ameliorates a symptom of the disease.

The BCAT inhibitor, which is preferably a compound of Formulas I, II, and/or III, a BCAT pathway-inhibiting analogue of Formulas I, II, and/or III, a BCAT pathway-inhibiting prodrug of Formulas I, II, and/or III, or a pharmaceutically acceptable salt of any of the foregoing, can be administered in accordance with the present inventive method by any suitable route. Suitable routes of administration include systemic, such as orally or by injection, topical, intraocular, periocular (e.g., subTenon's), subconjunctival, subretinal, suprachoroidal and retrobulbar. The manner in which the BCAT inhibitor is administered is dependent, in part, upon whether the treatment of retinopathy is prophylactic or therapeutic. The manner in which the BCAT inhibitor is administered for therapeutic treatment of retinopathy is dependent, in part, upon the cause of the retinopathy.

For example, given that diabetes is the leading cause of retinopathy, the BCAT inhibitor can be administered prophylactically as soon as the pre-diabetic retinopathy state is detected. For the prophylactic treatment of retinopathy that can result from diabetes, the BCAT inhibitor is preferably administered systemically, e.g., orally or by injection. For the therapeutic treatment of nonproliferative diabetic retinopathy, the BCAT inhibitor can be administered systemically, e.g., orally or by injection, or intraocularly. Proliferative diabetic retinopathy can be therapeutically treated by the administration of the BCAT inhibitor intraocularly, topically, subconjunctivally or periocularly (e.g., subTenon's), for example. The BCAT inhibitor is preferably administered intraocularly, topically, subconjunctivally or periocularly (e.g., subTenon's) for the prophylactic or therapeutic treatment of retinopathy before, during or after surgical removal from an eye of scar tissue generated during neovascularization during the proliferative diabetic stage.

The BCAT inhibitor is preferably administered as soon as possible after it has been determined that an animal, such as a mammal, specifically a human, is at risk for retinopathy (prophylactic treatment) or has begun to develop retinopathy (therapeutic treatment). Treatment will depend, in part, upon the particular BCAT inhibitor used, the amount of the BCAT inhibitor administered, the route of administration, and the cause and extent, if any, of retinopathy realized.

One skilled in the art will appreciate that suitable methods of administering a BCAT inhibitor, which is useful in the present inventive method, are available. Although more than one route can be used to administer a particular BCAT inhibitor, a particular route can provide a more immediate and more effective reaction than another route. Accordingly, the described routes of administration are merely exemplary and are in no way limiting.

The dose administered to an animal, particularly a human, in accordance with the present invention should be sufficient to effect the desired response in the animal over a reasonable time frame. One skilled in the art will recognize that dosage will depend upon a variety of factors, including the strength of the particular BCAT inhibitor employed, the age, species, condition or disease state, and body weight of the animal, as well as the amount of the retina about to be affected or actually affected by retinopathy. The size of the dose also will be determined by the route, timing and frequency of administration as well as the existence, nature, and extent of any adverse side effects that might accompany the administration of a particular BCAT inhibitor and the desired physiological effect. It will be appreciated by one of ordinary skill in the art that various conditions or disease states, in particular, chronic conditions or disease states, may require prolonged treatment involving multiple administrations.

Suitable doses and dosage regimens can be determined by conventional range-finding techniques known to those of ordinary skill in the art. Generally, treatment is initiated with smaller dosages, which are less than the optimum dose of the compound. Thereafter, the dosage is increased by small increments until the optimum effect under the circumstances is reached. The present inventive method will typically involve the administration of from about 1 mg/kg/day to about 100 mg/kg/day, preferably from about 15 mg/kg/day to about 50 mg/kg/day, if administered systemically. Intraocular administration typically will involve the administration of from about 0.1 mg total to about 5 mg total, preferably from about 0.5 mg total to about I mg total. A preferred concentration for topical administration is 100 mu M.

Compositions for use in the present inventive method preferably comprise a-pharmaceutically acceptable carrier and an amount of a BCAT inhibitor sufficient to treat retinopathy prophylactically or therapeutically. The carrier can be any ofthose conventionally used and is limited only by chemico-physical considerations, such as solubility and lack of reactivity with the compound, and by the route of administration. It will be appreciated by one of ordinary skill in the art that, in addition to the following described pharmaceutical compositions, the BCAT inhibitor can be formulated as polymeric compositions, inclusion complexes, such as cyclodextrin inclusion complexes, liposomes, microspheres, microcapsules and the like (see, e.g., U.S. Pat. Nos. *4,997,652, 5,185,152* and *5,718,922*).

The BCAT inhibitor can be formulated as a pharmaceutically acceptable acid addition salt. Examples of pharmaceutically acceptable acid addition salts for use in the pharmaceutical composition include those derived from mineral acids, such as hydrochloric, hydrobromic, phosphoric, metaphosphoric, nitric and sulfuric acids, and organic acids, such as tartaric, acetic, citric, malic, lactic, fumaric, benzoic, glycolic, gluconic, succinic, and arylsulphonic, for example p-toluenesulphonic, acids.

The pharmaceutically acceptable excipients described herein, for example, vehicles, adjuvants, carriers or diluents, are well-known to those who are skilled in the art and are readily available to the public. It is preferred that the pharmaceutically acceptable carrier be one which is chemically inert to the BCAT inhibitor and one which has no detrimental side effects or toxicity under the conditions of use.

The choice of excipient will be determined in part by the particular BCAT inhibitor, as well as by the particular method used to administer the composition. Accordingly, there is a wide variety of suitable formulations of the pharmaceutical composition of the present invention. The following formulations are merely exemplary and are in no way limiting.

Injectable formulations are among those that are preferred in accordance with the present inventive method. The requirements for effective pharmaceutically carriers for injectable compositions are well-known to those of ordinary skill in the art (see Pharmaceutics and Pharmacy Practice, J. B. Lippincott Co., Philadelphia, Pa., Banker and Chalmers, eds., pages 238-250 (1982), and ASHP Handbook on Injectable Drugs, Toissel, 4th ed., pages 622-630 (1986)). It is preferred that such injectable compositions be administered intramuscularly, intravenously, or intraperitoneally.

Topical formulations are well-known to those of skill in the art. Such formulations are suitable in the context of the present invention for application to the skin. The use of patches, corneal shields (see, e.g., U.S. Pat. No. *5,185,152*), and ophthalmic solutions (see, e.g., U.S. Pat. No. *5,710,182*) and ointments, e.g., eye drops, is also within the skill in the art.

Formulations suitable for oral administration can consist of (a) liquid solutions, such as an effective amount of the compound dissolved in diluents, such as water, saline, or orange juice; (b) capsules, sachets, tablets, lozenges, and troches, each containing a predetermined amount of the active ingredient, as solids or granules; (c) powders; (d) suspensions in an appropriate liquid; and (e) suitable emulsions. Liquid formulations may include diluents, such as water and alcohols, for example, ethanol, benzyl alcohol, and the polyethylene alcohols, either with or without the addition of a pharmaceutically acceptable surfactant, suspending agent, or emulsifying agent. Capsule forms can be of the ordinary hard- or soft-shelled gelatin type containing, for example, surfactants, lubricants, and inert fillers, such as lactose, sucrose, calcium phosphate, and corn starch. Tablet forms can include one or more of lactose, sucrose, mannitol, corn starch, potato starch, alginic acid, microcystalline cellulose, acacia, gelatin, guar gum, colloidal silicon dioxide, croscarmellose sodium, talc, magnesium stearate, calcium stearate, zinc stearate, stearic acid, and other excipients, colorants, diluents, buffering agents, disintegrating agents, moistening agents, preservatives, flavoring agents, and pharmacologically compatible excipients. Lozenge forms can comprise the active ingredient in a flavor, usually sucrose and acacia or tragacanth, as well as pastilles comprising the active ingredient in an inert base, such as gelatin and glycerin, or sucrose and acacia, emulsions, gels, and the like containing, in addition to the active ingredient, such excipients as are known in the art.

The effectiveness of an orally administered drug is dependent upon the drug's efficient transport across the mucosal epithelium and its stability in entero-hepatic circulation. Drugs that are effective after parenteral administration but less effective orally, or whose plasma half-life is considered too short, may be chemically modified into a prodrug form.

Formulations suitable for parenteral administration include aqueous and non-aqueous, isotonic sterile injection solutions, which can contain anti-oxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. The inhibitor can be administered in a physiologically acceptable diluent in a pharmaceutical carrier, such as a sterile liquid or mixture of liquids, including water, saline, aqueous dextrose and related sugar solutions, an alcohol, such as ethanol, isopropanol, or hexadecyl alcohol, glycols, such as propylene glycol or polyethylene glycol, dimethylsulfoxide, glycerol ketals, such as 2,2-dimethyl-1,3-dioxolane-4-methanol, ethers, such as poly(ethyleneglycol) 400, an oil, a fatty acid, a fatty acid ester or glyceride, or an acetylated fatty acid glyceride, with or without the addition of a pharmaceutically acceptable surfactant, such as a soap or a detergent, suspending agent, such as pectin, carbomers, methylcellulose, hydroxypropylmethylcellulose, or carboxymethylcellulose, or emulsifying agents and other pharmaceutical adjuvants. Oils, which can be used in parenteral formulations include petroleum, animal, vegetable, or synthetic oils. Specific examples of oils include peanut, soybean, sesame, cottonseed, corn, olive, petrolatum, and mineral.

Suitable fatty acids for use in parenteral formulations include oleic acid, stearic acid, and isostearic acid. Ethyl oleate and isopropyl myristate are examples of suitable fatty acid esters.

Suitable soaps for use in parenteral formulations include fatty alkali metals, ammonium, and triethanolamine salts, and suitable detergents include (a) cationic detergents such as, for example, dimethyl dialkyl ammonium halides, and alkyl pyridinium halides, (b) anionic detergents such as, for example, alkyl, aryl, and olefin sulfonates, alkyl, olefin, ether, and monoglyceride sulfates, and sulfosuccinates, (c) nonionic detergents such as, for example, fatty amine oxides, fatty acid alkanolamides, and polyoxyetbylenepolypropylene copolymers, (d) amphoteric detergents such as, for example, alkyl-p-aminopropionates, and 2-alkyl-imidazoline quaternary ammonium salts, and (e) mixtures thereof.

The parenteral formulations will typically contain from about 0.5 to about 25% by weight of the active ingredient in solution. Preservatives and buffers may be used. In order to minimize or eliminate irritation at the site of injection, such compositions may contain one or more nonionic surfactants having a hydrophile-lipophile balance (HLB) of from about 12 to about 17.

The quantity of surfactant in such formulations will typically range from about 5 to about 15% by weight. Suitable surfactants include polyethylene sorbitan fatty acid esters, such as sorbitan monooleate and the high molecular weight adducts of ethylene oxide with a hydrophobic base, formed by the condensation of propylene oxide with propylene glycol. The parenteral formulations can be presented in unit-dose or multi-dose sealed containers, such as ampules and vials, and can be stored in a freeze-dried (lyophilized) condition requiring only the addition of the sterile liquid excipient, for example, water, for injections, immediately prior to use. Extemporaneous injection solutions and suspensions can be prepared from sterile powders, granules, and tablets of the kind previously described.

Such compositions can be formulated as intraocular formulations, sustained-release formulations or devices (see, e.g., U.S. Pat. No. *5,378,475*). For example, gelantin, chondroitin sulfate, a polyphosphoester, such as bis-2-hydroxyethyl-terephthalate (BHET), or a polylactic-glycolic acid (in various proportions) can be used to formulate sustained-release formulations. Implants (see, e.g., U.S. Pat. Nos. *5,443,505, 4,853,224* and *4,997,652*), devices (see, e.g., U.S. Pat. Nos. *5,554,187, 4,863,457,* *5,098,443* and *5,725,493*), such as an implantable device, e.g., a mechanical reservoir, an intraocular device or an extraocular device with an intraocular conduit (e.g., 100 mu -1 mm in diameter), or an implant or a device comprised of a polymeric composition as described above, can be used.

The present inventive use also can involve the co-administration of other pharmaceutically active compounds. By "co-administration" is meant administration before, concurrently with, e.g., in combination with the BCAT inhibitor in the same formulation or in separate formulations, or after administration of a BCAT inhibitor as described above. For example, corticosteroids, e.g., prednisone, methylprednisolone, dexamethasone, or triamcinalone acetinide, or noncorticosteroid anti-inflammatory compounds, such as ibuprofen or flubiproben, can be co-administered. Similarly, vitamins and minerals, e.g., zinc, anti-oxidants, e.g., carotenoids (such as a xanthophyll carotenoid like zeaxanthin or lutein), and micronutrients can be co-administered.

General synthetic schemes for preparing compounds of Formula I- IV.

### EXAMPLE 1: trans-(1R,3R)(1-Aminomethyl-3-methyl-cyclohexyl)-acetic acid hydrochloride

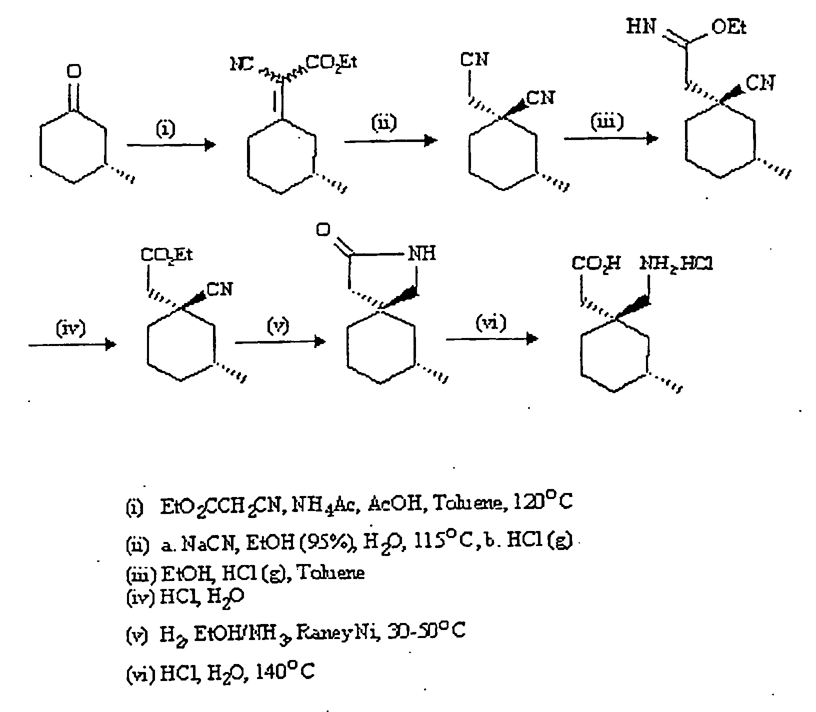

### Step i: 2-Cyano-((R)-3-methyl-cyclohexylidene)-acetic acid ethyl ester

A mixture of 3-(R)-Methylcyclohexanone (125mmol), Ethyl cyanoacetate (124mmol), Ammonium acetate (12.5mmol) and glacial Acetic acid (24mmnol) were refluxed with a Dean Stark trap for 24 Hours. The mixture was cooled and washed with H₂O. The H₂O washes were extracted with Toluene. The Toluene extracts were combined with the original organic layer, dried over MgSO₄ and the solvent evaporated. The crude oil was purified by Kugelrohr distillation to give an oiL (Bpt oven temp 150-160°C). Yield 86%
¹HNMR (CDCl₃) 400MHz δ: 1.01-1.05 (3H, m), 1.17-1.32 (1H, m), 1.35 (3H, t, J=7Hz), 1.42-2.30 (6H, m), 2.98 (1H, d, J=13Hz), 3.74 (1H, d, J=13Hz), 4.27 (2H, q, J=7Hz)
MS (CI) m/z: 208 (MH⁺)

| | | | | | |
|---|---|---|---|---|---|
| Analysis | C₁₂H₁₇NO₂ | Calc | C 69.54 | H 8.27 | N 6.76; |
| | | Found | C 69.44 | H 8.22 | N 6.76 |

### Step ii: trans-(1R,3R)-1-Cyanomethyl-3-methyl-cyclohexanecarbonitrile

To a solution of NaCN (40mmol) in 6mL H₂O and 160mL Ethanol (95%) was added 2-Cyano-((R)-3-methyl-cyclohexylidene)-acetic acid ethyl ester (40mmol). After 22 Hours at reflux the cooled solution was filtered, the filtrate acidified with gaseous HCl and filtered again. The solvent was removed and the crude oil was purified by column chromatography to give a pale yellow crystalline solid. Yield 88%
¹HNMR (CDCl₃) 400MHz δ: 0.90 (1H, m), 0.98 (3H, d, J=6Hz), 1.11 (1H, t, J=12Hz), 1.38 (1H, dt, J=4, 9Hz), 1.60-1.90 (4H, m), 2.07 (2H, m), 2.68 (2H, s)
MS (CI) m/z: 163 (MH⁺)

| | | | | | |
|---|---|---|---|---|---|
| Analysis | C₁₀H₁₄N₂ | Calc | C 74.04 | H 8.70 | N 17.27 |
| | | Found | C 74.05 | H 8.71 | N 17.25 |

### Step iii: trans-2-((1R,3R)-1-Cyano-3-methyl-cyclohexyl)-acetimidic acid ethyl ester

To a solution of trans-(1R,3R)-1-Cyanomethyl-3-methyl-cyclohexanecarbonitrile (6.2mmol) in 30mL Ethanol (absolute) was added 30 mL dried Toluene. The solution was chilled in ice whilst saturating with gaseous HCl. The stoppered solution was then left to stand at room temperature for 24 Hours. The solvent was removed and the solid residue was triturated with Diethyl Ether to obtain a ppt which was dried to give a white crystalline solid. Yield 50%.
Mp 118-120°C
¹HNMR (DMSO) 400MHz δ: 0.8-0.89 (1H, m), 0.91 (3H, d, J=6.3Hz), 1.06-1.12 (1H, m), 1.24-1.35 (1H, m), 1.37 (3H, t, J=7Hz), 1.41-1.95 (6H, m), 3.02 (2H, s), 4.49 (2H, q, J=7Hz) MS (CI) m/z: 195 (MH⁺)

| | | | | | |
|---|---|---|---|---|---|
| Analysis | C₁₂H₂₀N₂O. 1.08M HCl | Calc | C 58.19 | H 8.58 | N 11.31 |
| | | Found | C 58.25 | H 8.59 | N 11.59 |

### Step iv: trans-((1R,3R)-1-Cyano-3-methyl-cyclohexyl)-acetic acid ethyl ester

trans-2-((1R,3R)-1-Cyano-3-methyl-cyclohexyl)-acetimidic acid ethyl ester (1.1mmol) was dissolved in ice cold H₂O (40mL) and the pH adjusted with 1N HCl to pH 1.5. The solution was stirred at room temperature for 20 Hours. Ethylacetate was added (30mL) and the organic layer was washed with H₂O, dried and the solvent removed to leave a clear oil. Yield 82%
¹HNMR (CDCl₃) 400MHz δ: 0.78-0.90 (1H, m), 0.93 (3H, d, J=6Hz), 0.97-1.00 (1H, m), 1.23-1.25 (1H, m), 1.29 (3H, t, J=7.2Hz), 1.59-1.80 (4H, m), 2.05-2.08 (2H, Br t), 2.54 (2H, s), 4.20 (2H, q, J=7.2Hz)
MS (CI) m/z: 210 (MH⁺)

| | | | | | |
|---|---|---|---|---|---|
| Analysis | C₁₂H₁₉NO₂ | Calc | C 68.87 | H 9.15 | N 6.69 |
| | | Found | C 68.87 | H 9.11 | N 6.90 |

### Step v: trans-(5R,7R)-7-Methyl-2-aza-spiro[4.5]decan-3-one

trans-((1R,3R)-1-Cyano-3-methyl-cyclohexyl)-acetic acid ethyl ester (8.9mmol) was dissolved in NH₃/EtOH (7%, 40mL) along with prewashed Raney Nickel (H₂O followed by EtOH) in a 250mL Parr flask.. The solution was hydrogenated at 30°C, 46psi i.e. 317.158 Kilopascals for 24 Hours.

The cooled solution was filtered through a pad of celite, washing with Ethyl acetate. The solvent was removed from the filtrate to leave a.white solid. Yield 30%.
Mp 92-98°C
¹HNMR (DMSO) 400MHz δ: 0.75-0.82 (1H, m), 0.84 (3H, d, J=6.4Hz), 0.88-0.94 (1H, m), 1.14-1.19 (1H, m), 1.20-1.50 (2H, m), 1.50-1.63 (4H, m), 1.91 (2H, s), 3.03 (2H, s), 7.42 (1H, s)
MS (CI) m/z: 168 (MH⁺)

| | | | | | |
|---|---|---|---|---|---|
| Analysis | C₁₀H₁₇NO | Calc | C 71.81 | H 10.25 | N 8.37 |
| | | Found | C 71.80 | H 10.29 | N 8.31 |

### Step vi: trans-((1R,3R)-1-Aminomethyl-3-methyl-cyclohexyl)-acetic acid hydrochloride

trans-(5R,7R)-7-Methyl-2-aza-spiro[4.5]decan-3-one (2.17mmol) was dissolved in a solution of 10M HCl (5mL) and H₂O (5mL) and the mixture was refluxed at approx 140°C for 5 Hours. The cooled solution was diluted with 10 mL H₂O and 10 mL DCM and the aqueous layer was further washed with 2x15 mL DCM. The aqueous layer was then reduced to dryness to leave a white solid. Yield 76%.
Mp 148-155°C. [α]_{D} = -2.5 (T=20°C, c=1, MeOH). One isomer (RR).
¹HNMR (CDCl₃) 400MHz δ: 0.69-0.79 (1H, m), 0.82 (3H, d, J=6Hz), 0.87-0.90 (1H, m), 1.12-1.20 (1H, dt, J=4.5, 13.3Hz), 1.34-1.50 (3H, m), 1.60-1.63 (3H, m), 2.30 (2H, s), 3.01 (2H, s), 7.93 (3H, Br s)
MS (CI) m/z: 186 (MH⁺)

| | | | | | |
|---|---|---|---|---|---|
| Analysis | C₁₀H₁₉NO₂. 1.1 HCl | Calc | C 53.29 | H 8.99 | N 6.21 |
| | | Found | C 53.23 | H 8.99 | N 6.45 |

### EXAMPLE 2: (1-Aminomethyl-2-methyl-cyclohexyl)-acetic acid hydrochloride

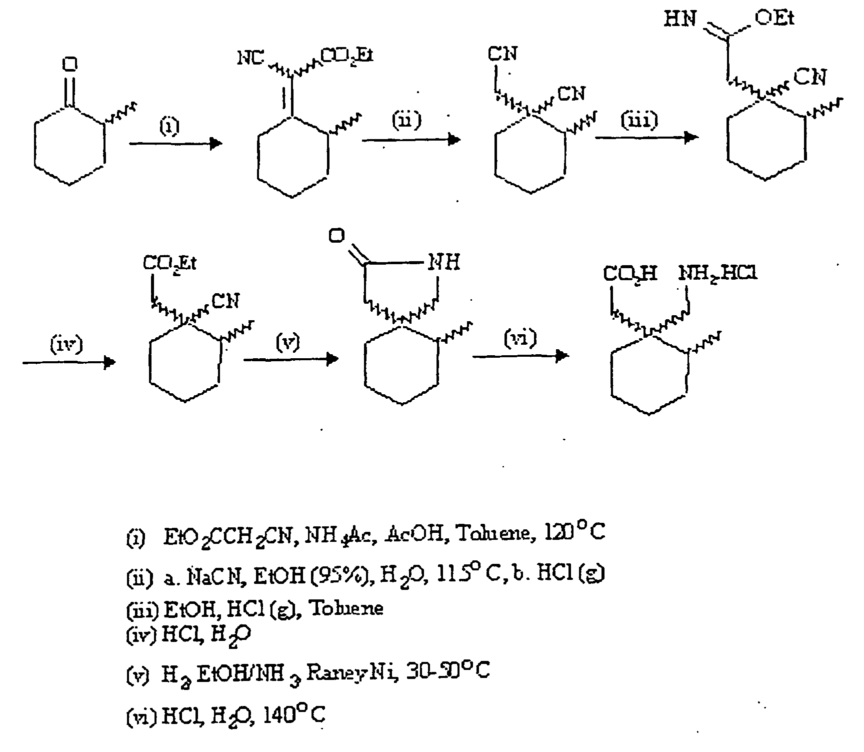

### Step i: 2-Cyano-(2-methyl-cyclohexylidene)-acetic acid ethyl ester

(+/-) 2-Methyl cyclohexanone (80mmol), Ethyl cyanoacetate (80mmol), Ammonium acetate (8mmol) and glacial Acetic acid (16mmol) were reacted as in the General method (Example 1) step i to give a clear oil. Yield 76%.
¹HNMR (CDCl₃) 400MHz δ: 1.23 (3H, dd, J=7, 10Hz), 1.35 (3H, t, J=7Hz), 1.55-1.82 (5H, m), 1.93-2.05 (1H, m), 2.17 (1H, dt, J=5, 14Hz), 2.47 (1H, dt, J=5, 9Hz), 2.92-2.97 (1H, Br d, J=15Hz), 3.30-3.35 (1H, m), 3.81-3.86 (1H, Br d, J=15 Hz), 4.06-4.14 (1H, m), 4.23-4.30 (3H, dq, J=1, 6Hz)
MS (CI) m/z: 208 (MH+)

| | | | | | |
|---|---|---|---|---|---|
| Analysis | C₁₂H₁₇NO₂ | Calc | C 69.54 | H 8.27 | N 6.76 |
| | | Found | C 69.26 | H 8.26 | N 6.66 |

### Step ii: 1-Cyanomethyl-2-methyl-cyclohexanecarbonitrile

2-Cyano-(2-methyl-cyclohexylidene)-acetic acid ethyl ester (37mmol), NaCN (37mmol) were reacted as in the General method (Example 1) step ii. The crude solid was purified by column chromatography (3:1, Heptane : Ethylacetate), to give a clear oil. Yield 76%
¹HNMR (CDCl₃) 400MHz δ: 1.06 (3H, d, J=6.8Hz), 1.11 (3H, d, J=6.8Hz), 1.20-2.20 (18H, m), 2.77 (2H, dd, J=16.8Hz), 2.63 (2H, dd, J=16.8Hz)
MS (CI) m/z: 163 (MH⁺)

| | | | | | |
|---|---|---|---|---|---|
| Analysis | C₁₀H₁₄N₂. 0.1 H₂O | Calc | C 73.49 | H 8.69 | N 16.86 |
| | | Found | C 73.24 | H 8.73 | N 17.08 |

### Step iii: 2-(1-Cyano-2-methyl-cyclohexyl)-acetimidic acid ethyl ester

1-Cyanomethyl-2-methyl-cyclohexanecarbonitrile(7.3mmol) was reacted as in the General method (Example 1) Step (iii) to give a white solid. Yield 70%.
Mpt 107-114°C.
¹HNMR (DMSO) 400MHz δ: 1.00-1.06 (3H, 2xt, J=6.4Hz), 1.10-1.38 (2H, m), 1.38 (3H, t, J=6.8Hz), 1.40-2.10 (7H, m), 2.86, 2.92, 3.10, 3.28 (2H, 4xd, J=14,14.4, 14.8, 14Hz resp.), 4.48 (2H, q, J=6.8Hz)
MS (CI) m/z: 209 (MH⁺)

| | | | | | |
|---|---|---|---|---|---|
| Analysis | C₁₂H₂₀N₂ O . 1.06 HCl | Calc | C 58.37 | H 8.60 | N 11.34 |
| | | Found | C 58.15 | H 8.63 | N 11.60 |

### Step iv: (1-Cyano-2-methyl-cyclohexyl)-acetic acid ethyl ester

2-(1-Cyano-2-methyl-cyclohexyl)-acetimidic acid ethyl ester (4.1mmol) was reacted as in the General method (Example 1) step (iv) to give a clear oil. Yield 82%
¹HNMR (CDCl₃) 400MHz δ: 1.03, 1.09 (3H, 2xd, J=7Hz), 127-1.30 (3H, m), 1.32-2.00 (8H, m) 2.10-2.20 (1H, m), 2.44, 2.82 (3H, 2xd, J=14.8Hz), 2.54 (1H, m), 4.16-4.22 (2H, m)
MS (CI) m/z: 210 (M⁺)

| | | | | | |
|---|---|---|---|---|---|
| Analysis | C₁₂H₁₉NO₂ | Calc | C 68.87 | H 9.15 | N 6.69 |
| | | Found | C 68.84 | H 9.13 | N 6.75 |

### Step v: 6-Methyl-2-aza-spiro[4,5]decan-3-one

(1-Cyano-2-methyl-cyclohexyl)-acetic acid ethyl ester (8.4mmol) was reacted as in the General method (Example 1) Step (v) for 24 Hours at 10°C, 50 psi i.e. 344.737 Kilopascals

The crude oil was purified by column chromatography (Ethylacetate), to give a white solid. Yield 34%.
Mp: 85-90 °C
¹HNMR (CDCl₃) 400MHz δ: 0.88-0.91 (3H, dd, J=4, 6.8Hz), 1.41-1.78 (9H, m), 2.00-2.30 (2H, m), 3.06-3.23 (2H, m), 7.27 (1H, Br s)
MS (CI) m/z: 168 (MH⁺)

| | | | | | |
|---|---|---|---|---|---|
| Analysis | C₁₀H₁₇NO | Calc | C 71.81 | H 10.24 | N 8.37 |
| | | Found | C 71.83 | H 10.19 | N 8.27 |

### Step vi: (1-Aminomethyl-2-methyl-cyclohexyl)-acetic acid hydrochloride

6-Methyl-2-aza-spiro[4.5]decan-3-one (2.5mmol) was reacted as in the General method (Example 1) step vi to give a white solid. Yield 42%.
Mp: 108-110° C.
[α]_{D} = 0 (T=20.5° C, C=1, MeOH). Two Diastereomers 3:1
¹HNMR (DMSO + D₂O) 400MHz δ: 0.79, 0.85 (3H, 2xd, J=6.8Hz), 1.21-1.65 (9H, m), 2.22, 2.43 (1H, 2xd, J=15Hz), 2.46, 2.49 (1H, 2xd, J=15Hz), 2.83-2.92 (1H, 2xd, J=13.6Hz), 3.05, 3.15 (1H, 2xd, J=13.6Hz).
MS (CI) m/z: 186 (MH⁺)

| | | | | | |
|---|---|---|---|---|---|
| Analysis | C₁₀H₁₉NO₂. 1.3 HCl | Calc | C 51.64 | H 8.79 | N 6.02 |
| | | Found | C 51.66 | H 8.91 | N 6.16 |

### EXAMPLE 3 (1-Aminomethyl-3,3-dimethyl-cyclohexyl)-acetic acid hydrochloride

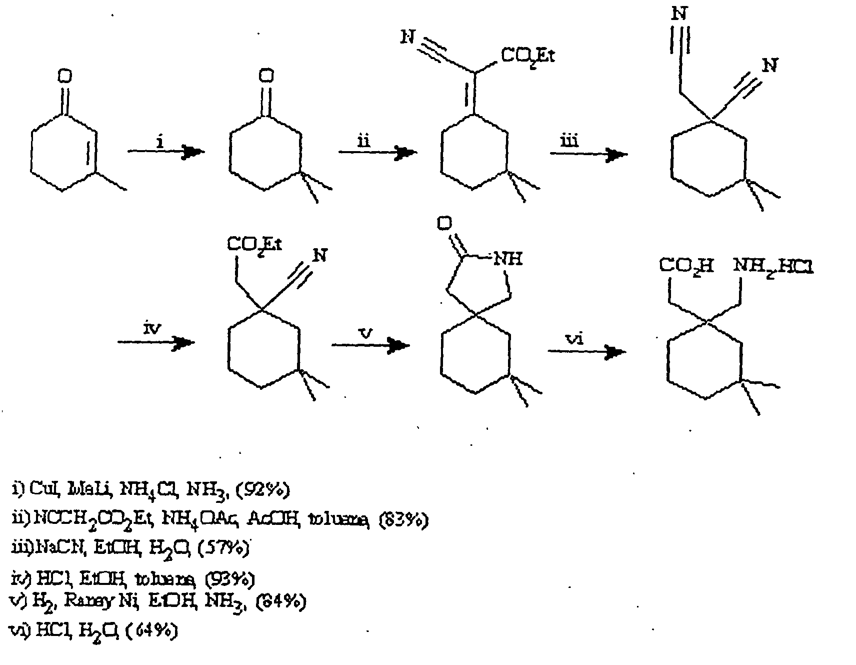

### Step i: 3,3-Dimethyl-cyclohexanone:

Synthesised via the method outlined by Pelletier, S.W., Mody, N.V., *J. Org. Chem*., 1976, 41, 1069.

A solution of lithium dimethyl cuprate was prepared by the addition of methyl lithium (1.4M in ether, 77.25mL, 2.45mol) to copper (I) iodide (8.8g, 0.046mol) under argon. The solution was cooled to 0°C, and 3-methyl-cyclohexen-1-one (5mL, 0.044mol) was added dropwise, with stirring, and a deep yellow precipitate was formed. The suspension was stirred at room temperature for one hour before being poured into a solution of aqueous ammonia (100mL) and ammonium acetate (ca. 5g). The layers were separated and the aqueous layer washed with diethyl ether (3x 50mL). The combined organics were washed with saturated brine (3x 100mL), dried (MgSO₄), and the solvent removed in vacuo to leave a dark yellow liquid.
¹H NMR (400MHz. CDCl₃) 0.98 (6H, s), 1.59 (2H, m), 1.88 (2H, m), 2.14 (2H, m), 2.26 (2H, m).
IR (film) νₘₐₓcm⁻¹ 2956, 1711 (C=O), 1457, 1368, 1292, 1226, 1076.

### Step ii: 2-Cyano-5,5-dimethyl-3-propyl-hex-2-enoic acid ethyl ester

To a solution of 3,3-dimethylcyclohexanone (4g, 0.032mol) in toluene (25mL) was added ethyl cyanoacetate (3.37mL, 0.032mol, 1 eq.), ammonium acetate (0.24g, 0.003mol, 0.1eq.), and acetic acid (0.36mL, 0.006mol, 0.2eq.). The yellow solution was heated to reflux whilst attached to a Dean-Stark trap, and heating was continued until no more water condensed in the trap. After cooling, the now orange solution was washed with water (3x 25mL) and the organic layer dried (MgSO₄). Filtration and removal of the solvent *in vacuo* gave the crude product as a deep orange liquid. Purification was achieved by Kugelrohr distillation to leave the mixture of *cis* and *trans* products as a pale yellow liquid, bp 160-170°C, 4 mbar (5.83g, 83%).
¹H NMR (400MHz. CDCl₃) 0.96 (6H, s, 2x Me), 0.99 (6H, s, 2x Me),1.34 (6H, m, 2x Me of ester), 1.49 (4H, m), 1.75 (2H, quin, *J* 6.4), 1.82 (2H, quin, *J* 6.4), 2.46 (2H, s), 2.60 (2H, t, *J* = 6.4 Hz), 2.80 (2H, s), 2.93 (2H, t, *J* = 6.4 Hz), 4.27 (4H, m).
MS (CI) z/e 222 (M⁺+1)
IR (film) νₘₐₓcm⁻¹ 2958, 2870, 2224 (CN), 1731 (C=O), 1606 (C=C), 1277, 1223.

| | | | | | |
|---|---|---|---|---|---|
| Analysis | C₁₃H₁₉O₂N | Calc | C 70.56 | H 8.65 | N 6.32 |
| | | Found | C 70.35 | H 8.79 | N 6.25 |

### Step iii: 1-Cyanomethyl-3,3-dimethyl-cyclohexanecarbonitrile

To a solution of the 2-Cyano-5,5-dimethyl-3-propyl-hex-2-enoic acid ethyl ester (1.26g, 0.006mol) in ethanol (100ml) and water (4mL) was added sodium cyanide (0.28g, 0.006mol, 1 eq.). The yellowish solution was heated to reflux for 8 hours and then cooled, during which time an off-white precipitate was formed.
The suspension was filtered under vacuum and the filtrate acidified with HCl gas until the pH was approximately 2. The mixture was then filtered a second time and then the solvent removed *in vacuo* to leave the crude product as a pale green solid.
Flash column chromatography, after adsorption of the crude product on to silica, eluting with 0-50% EtOAc in heptane gave the binitrile as a colourless solid (0.57g, 57%).
¹H NMR (400MHz, CDCl₃) 0.99 (3H, s, Me), 1.13 (1H, td, J 13.2, 4.2), 1.21 (3H, s, Me), 1.32 (2H, m), 1.54 (1H, m), 1.82 (3H, m), 2.15 (1H, m), 2.65 (2H, s, CH₂CN).
¹³C IVMR (400MHz, CDCl₃) 19.61, 25.17, 30.79, 31.18, 33.77, 34.79, 35.37, 37.92, 46.26, 115.06, 122.19.
MS (CI) z/e 177 (M⁺+1)
IR (film) νₘₐₓcm⁻¹ 2988, 2937, 2869, 2237 (2x CN), 1749, 1456, 1423, 1369, 1202, 1180, 1031, 972.

| | | | | | |
|---|---|---|---|---|---|
| Analysis | C₁₁H₁₆N₂ | Calc | C74.96 | H 9.15 | N 15.89 |
| | | Found | C 75.08 | H 9.32 | N 15.80 |

### Step iv: (1-Cyano-3,3-dimethyl-cyclohexyl)-acetic acid ethyl ester:

1-Cyanomethyl-3,3-dimethyl-cyclohexanecarbonitrile (0.50g, 2.84mmol) was dissolved in absolute ethanol (20mL) at room temperature and then cooled to 0°C. Toluene (20mL) was added to the solution and then the reaction mixture was acidified by passing HCl gas through it at a gentle rate for *ca*. 45 mins. The flask was then stoppered and left to stand at room temperature for 24 hours.

The yellow solution was partitioned between ethyl acetate and water and the layers separated. The aqueous layer was extracted with ethyl acetate (3x 30mL), and the combined organics washed with aq. sat. sodium hydrogen carbonate solution (3x 50mL), brine (3x 50mL), dried (MgSO₄), and the solvent removed under reduced pressure to leave a pale yellow liquid (0.59g, 93%).
¹H NMR (400MHz, CDCl₃) 0.94 (3H, s, Me), 1.16 (3H,m), 1.21 (3H, s, Me), 1.29 (3H, t, *J* 7.2, CH₂CH₃), 1.50 (1H, m), 1.65 (1H, dt, *J* 14.4, 7.6), 1.84 (1H, qt, *J* 13.3, 3.2),1.96 (1H, dt, *J* 13.7, 2.2), 2.16 (1H, m), 2.48, (1H, d, *J* 15.6, C-2H), 2.54 (1H, d, *J* 15.6, C-2H), 4.20 (2H, q, J 7.2, CH₂CH₃).
¹³C NMR (400MHz, CDCl₃) 14.21, 19.65, 25.42, 31.03, 34.04, 34.14, 36.08, 38.44, 46.14, 46.80, 61.02, 123.67, 169.00.
MS (CI) z/e 224 (M⁺+1).
IR (film) νₘₐₓcm⁻¹ 2998, 2937, 2868, 2234 (CN), 1738 (C=O), 1457, 1372, 1217, 1181, 1154, 1026.

| | | | | | |
|---|---|---|---|---|---|
| Analysis | C₁₃H₂₁NO₂ | Calc | C 69.92 | H 9.48 | N 6.27 |
| | | Found | C 69.63 | H9.45 | N 6.15 |

### Step v 7,7-Dimethyl-2-aza-spiro[4.5]decan-3-one:

(1-Cyano-3,3-dimethyl-cyclohexyl)-acetic acid ethyl ester (0.5g, 2.23mmol) was hydrogenated in ethanolic ammonia (60mL) with Raney nickel as catalyst (*ca*. 0.25g) at 50°C and 50psi i.e. 344.737 Kilopascals for 49 hours.

The catalyst was then removed by filtration through Celite, and the solvent removed *in vacuo* to leave a greenish crystalline solid.

Flash column chromatography, eluting with 0-100% ethyl acetate in heptane, gave the pure lactam as a colourless solid (340 mg, 84%).
¹H NMR (400MHz, CDCl₃) 0.89 (3H, s, Me), 0.92 (3H, s, Me), 1.25 (2H, m), 1.36 (2H, m), 1.51 (3H, m), 1.68 (1H, s), 2.18 (1H, d, *J* 16.4, CH₂NH), 2.24 (1H, d, *J* 16.8, CH₂NH), 3.15 (2H, s, CH₂CO).
¹³C NMR (400MHz, CDCl₃) 19.16, 29.88, 30.36, 31.28, 36.57, 39.05, 39.61, 44.58, 49.54, 54.79, 177.72.
MS (CI) z/e 182 (M⁺+1).
IR (film) νₘₐₓcm⁻¹ 3203, 3100 (NH), 2914, 2860, 1698 (C=O), 1486, 1374, 1317, 1289, 1257, 1076.

| | | | | | |
|---|---|---|---|---|---|
| Analysis | C₁₁H₁₉NO | Calc | C 72.88 | H 10.56 | N 7.73 |
| | | Found | C 72.38 | H 10.47 | N 7.56 |

### Step vi (1-Aminomethyl-3,3-dimethyl-cyclohexyl)-acetic acid hydrochloride:

7,7-Dimethyl-2-aza-spiro[4.5]decan-3-one (0.3g, 1.66mmol) was dissolved in a mixture of HCl (conc., 5mL) and water (5mL), and the resultant colourless solution heated to reflux for 20 hours. The solution was cooled and then partitioned between water and dichloromethane, and the layers separated. The aqueous layer was washed with dichloromethane (3x 20mL) and the water/HCl removed by rotary evaporation to leave the crude product as an off-white solid. Trituration of this solid with ethyl acetate and filtration of the product gave (1-Aminomethyl-3,3-dimethyl-cyclohexyl)-acetic acid, hydrochloride salt as a colourless solid (140mg, 42%, 64% based on recovered starting material).
¹H NMR (400MHz, DMSO) 0.90 (3H, s, Me), 0.92 (3H, s, Me), 1.15-1.49 (8H, m), 2.45 (2H, s, CH₂CO₂H), 2.90 (2H, br.q, *J* 13.5, CH₂NH₃), 7.96 (3H, br.s, NH₃), 12.36 (1H, br.s, OH).
IR (film) νₘₐₓcm⁻¹ 2930, 1728 (C=O), 1272, 1123.

| | | | | | |
|---|---|---|---|---|---|
| Analysis | C₁₁H₂₂NO₂Cl | Calc | C 56.04 | H 9.41 | N 5.94 |
| | | Found | C 55.79 | H 9.61 | N 6.23 |

### EXAMPLE 4: (±)-(trans)-(1-Aminomethyl-3,4-dimethyl-cyclopentyl)-acetic acid hydrochloride

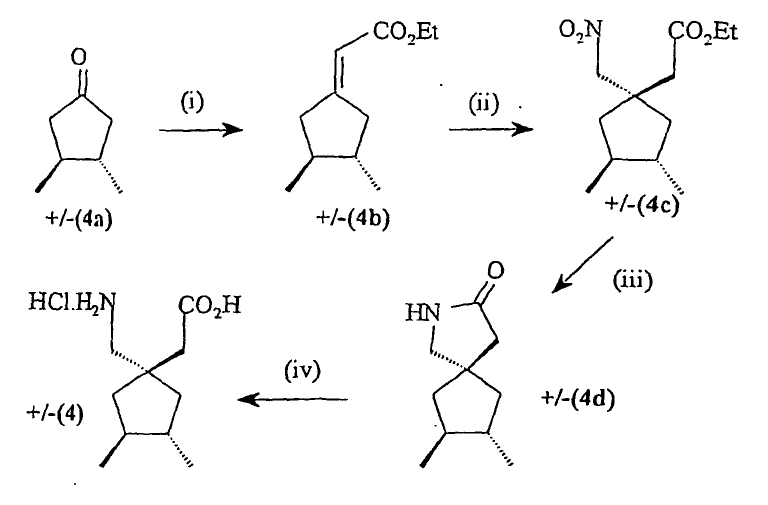

Reagents: (i) Triethylphosphonoacetate, NaH; (ii) MeNO₂,Bu₄N⁺F⁻; (iii) H₂, Ni; (iv) HCl

### Step i: synthesis of (±) (trans)-(3,4-Dimethyl-cyclopentylidene)-acetic acid ethyl ester (4b)

NaH (60% dispersion in oil, 737 mg, 18.42 mmol) was suspended in dry tetrahydrofuran (50 mL) and cooled to 0°C. Triethylphosphonoacetate (3.83 mL, 19.30 mmol) was added and the mixture stirred at 0°C for 15 minutes. The ketone (+/-)-(**4a**) (1.965 g, 17.54 mmol) in THF (10 mL) was then added and the mixture allowed to warm to room temperature. After 2 hours, the mixture was partitioned between diethyl ether (200 mL) and water (150 mL). The organic phase was separated, washed with brine, dried (MgSO₄) and the solvent removed in vacuo. The residue was purified by flash chromatography (silica, ethyl acetate:heptane 1:9) to give 3.01 g (94%) of (**4b**) as a colorless oil.
¹H NMR 400 MHz (CDCl₃): δ 1.01 (3H, d, J = 6 Hz), 1.03 (3H, d, J = 6 Hz), 1.26 (3H, t, J = 7 Hz), 1.49 (2H, m), 2.07 (1H, m), 2.24 (1H, m), 2.61 (1H, m), 4.13 (2H, q, J = 7 Hz), 5.72 (1H, s).
MS (CI+) m/e: 183 (M+1).

### Step ii: synthesis of (±)(trans)-(3,4-Dimethyl-1-nitromethyl-cyclopentyl)-acetic acid ethyl ester (4c)

(±)(trans)-(3,4-Dimethyl-cyclopentylidene)-acetic acid ethyl ester (**4b**) (2.95 g, 16.2 mmol) was dissolved in tetrahydrofuran (10 mL) and stirred at 70°C with nitromethane (1.9 mL, 35.2 mmol) and tetrabutylammonium fluoride (1.0 M in tetrahydrofuran, 22 mL, 22.0 mmol). After 6 hours, the mixture was cooled to room temperature, diluted with ethyl acetate (50 mL), and washed with 2N HCl (30 mL) followed by brine (50 mL). The organic phase was collected, dried (MgSO₄) and the solvent removed in vacuo. The residue was purified by flash chromatography (silica, ethyl acetate:heptane 1:9) to give 1.152 g (29%) of a clear oil.
¹H NMR 400 MHz (CDCl₃): δ 0.98 (6H, d, J = 6 Hz), 1.10-1.39 (5H, m), 1.47 (2H, m), 1.87 (1H, m), 2.03 (1H, m), 2.57 (2H, ABq, J= 16, 38 Hz), 4.14 (2H, q, J = 7 Hz), 4.61 (2H, ABq, J = 12, 60 Hz).
MS (ES+) m/e: 244 (M+1).
IR (film) ν cm⁻¹: 1186, 1376, 1549, 1732, 2956.

### Step iii: Synthesis of (±)-(trans)-7,8-Dimethyl-spiro[4.4]nonan-2-one (4d)

(±)(trans)-(3,4-Dimethyl-1-nitromethyl-cyclopentyl)-acetic acid ethyl ester (**4c**) (1.14 g. 4.7 mmol) was dissolved in methanol (50 mL) and shaken over Raney nickel catalyst under an atmosphere of hydrogen (40 psi i.e. 275.790 Kilopascals) at 30°C. After 5 hours, the catalyst was removed by filtration through celite. The solvent was removed in vacuo to give 746 mg (95%) of a pale yellow oil which solidified on standing.
¹H NMR 400 MHz(CDCl₃): δ 0.98 (6H, d, J = 6 Hz), 1.32 (2H, m), 1.46 (2H, m), 1.97 (2H, m), 2.27 (2H, ABq, J = 16, 27 Hz), 3.23 (2H, s), 5.62 (1H, br s).
MS (ES+) m/e: 168 (M+1).
IR (film) ν cm⁻¹: 1451, 1681, 1715, 2948, 3196.

### Step iv: Synthesis of (±)-(trans)-(1-Aminomethyl-3,4-dimethyl-cyclopentyl)-acetic acid hydrochloride (4)

(±)-(trans)-7,8-Dimethyl-spiro[4.4]nonan-2-one (**4d**) (734 mg, 4.40 mmol) was heated to reflux in a mixture of 1,4-dioxan (5 mL) and 6N HCl (15 mL). After 4 hours, the mixture was cooled to room temperature, diluted with water (20 mL), and washed with dichloromethane (3 × 30 mL). The aqueous phase was collected and the solvent removed in vacuo. The residue was triturated with ethyl acetate to give 675 mg (69%) of a white solid after collection and drying.
¹H NMR 400 MHz (d₆-DMSO): δ 0.91 (6H, d, J = 6 Hz), 1.18 (2H, m), 1.42 (2H, m), 1.72 (1H, m), 1.87 (1H, m), 2.42 (2H, ABq, J = 16, 24Hz), 2.90 (2H, ABq, J = 12, 34 Hz), 8.00 (3H, br s), 12.34 (1H, br s).
MS (ES+) m/e: 186 (M+1 for the free base).

### EXAMPLE 5 (cis/trans)-(3R)-(1-Aminomethyl-3-methyl-cyclopentyl)-acetic acid hydrochloride (5)

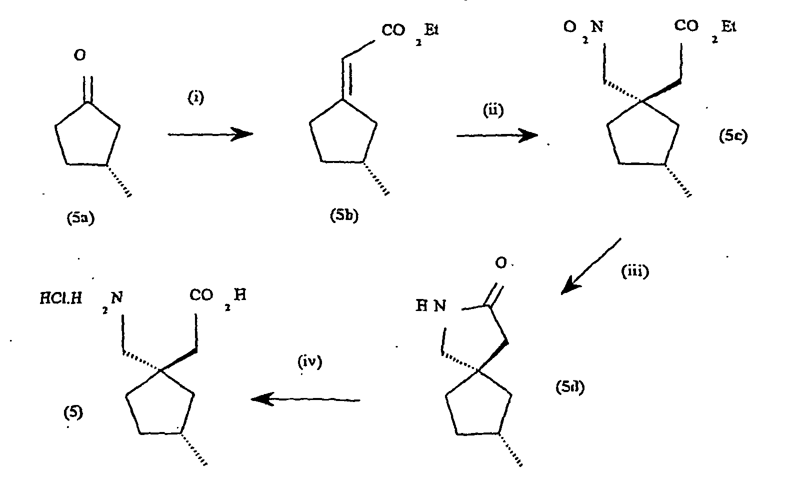

Reagents: (i) Triethylphosphonoacetate, NaH; (ii) MeNO₂, Bu₄N⁺F⁻; (iii) H₂, Ni; (iv) HCl

### Step i: Synthesis of (R)-(3-Methyl-cyclopentylidene)-acetic acid ethyl ester (5b)

NaH (60% dispersion in oil, 1.86 g, 46.5 mmol) was suspended in dry tetrahydrofuran (40 mL) and cooled to 0°C. Triethylphosphonoacetate (9.69 mL, 48.8 mmol) was added and the mixture stirred at 0°C for 15 minutes. (R)-3-Methylcyclopentanone (5a) (5 ml, 46.5 mmol) in THF (10 mL) was then added and the mixture allowed to warm to room temperature. After 2 hours, the mixture was partitioned between diethyl ether (200 mL) and water (150 mL). The organic phase was separated, washed with brine, dried (MgSO₄) and the solvent removed in vacuo. The residue was purified by flash chromatography (silica, ethyl acetate:heptane 1:9) to give 5.45 g (70%) of (2) as a colorless oil.
¹H NMR 400 MHz (CDCl₃): δ 1.04 (3H, m), 1.27 (3H, t, J = 7 Hz), 1.80-2.74 (7H, m), 2.90-3.15 (1H, m), 4.13 (2H, q, J = 7 Hz), 5.76 (1H, s).
MS (CI+) m/e: 169 (M+1).
IR (film) ν cm⁻¹: 1205, 1371, 1653, 1716, 2955.

### Step ii: Synthesis of (cis/trans)-(3R)-(3-Methyl-1-nitromethyl-cyclopentyl)-acetic acid ethyl ester (5c)

(R)-(3-Methyl-cyclopentylidene)-acetic acid ethyl ester **(5b)** (3.0 g, 17.8 mmol) was dissolved in tetrahydrofuran (20 mL) and stirred at 70°C with nitromethane (1.92 mL, 35.6 mmol) and tetrabutylammonium fluoride (1.0 M in tetrahydrofuran, 25 mL, 25.0 mmol). After 18 hours, the mixture was cooled to room temperature, diluted with ethyl acetate (50 mL), and washed with 2N HCl (30 mL) followed by brine (50 mL). The organic phase was collected, dried (MgSO₄), and the solvent removed in vacuo. The residue was purified by flash chromatography (silica, ethyl acetate:heptane 1:9) to give 2.00 g (49%) of a clear oil.
¹H NMR 400 MHz (CDCl₃): δ 1.02 (3H, d, J = 6 Hz), 1.08-1.37 (5H, m), 1.59-2.17 (5H, m), 2.64 (2H, m), 4.15 (2H, q, J = 7 Hz), 4.64 (2H, m).
MS (ES+) m/e: 230 (M+1).
IR (film) ν cm⁻¹: 1183, 1377, 1548, 1732, 2956.

### Step iii:Synthesis of (cis/trans)-(7R)-7-Methyl-spiro[4.4]nonan-2-one (5d)

(cis/trans)-(3R)-(3-Methyl-1-nitromethyl-cyclopentyl)-acetic acid ethyl ester (5c) (1.98 g, 8.66 mmol) was dissolved in methanol (50 mL) and shaken over Raney nickel catalyst under an atmosphere of hydrogen (40 psi i.e. 275.790 Kilopascals) at 30°C. After 18 hours, the catalyst was removed by filtration through celite. The solvent was removed in vacuo and the residue purified by flash chromatography (silica, ethyl acetate:heptane 1:1) to give 1.05 g (79%) of a white solid.
¹H NMR 400 MHz (CDCl₃): δ 1.03 (3H, m), 1.22 (2H, m), 1.60-2.15 (5H, m), 2.22 (2H, m), 3.20 and 3.27 (2H total, 2 × s, cis, and trans), 6.18 (1H, br s).
MS (ES+) m/e: 154 (M+1).
IR (film) ν cm⁻¹: 1695, 2949, 3231.

### Step iv: Synthesis of (cis/trans)-(3R)-(1-Aminomethyl-3-methyl-cyclopentyl)-acetic acid hydrochloride (5)

(cis/trans)-(7R)-7-Methyl-spiro[4.4]nonan-2-one (5d) (746 mg, 4.88 mmol) was heated to reflux in a mixture of 1,4-dioxan (5 mL) and 6N HCl (15 mL). After 4 hours, the mixture was cooled to room temperature, diluted with water (20 mL), and washed with dichloromethane (3 × 30 mL). The aqueous phase was collected and the solvent removed in vacuo. The residue was triturated with ethyl acetate to give a white solid which was collected and dried. This was recrystallized from ethyl acetate/methanol to give 656 mg (65%) of (5) after collection and drying.
¹H NMR 400 MHz (d₆-DMSO): δ 0.96 (3H, m), 1.01-1.24 (2H, m), 1.42-2.10 (5H, m), 2.41 and 2.44 (2H total, 2 × s, cis/trans), 2.94 (2H, m), 7.96 (3H, br s), 12.35 (1H, br s). MS (ES+) m/e: 172 (M+1 for the free base).

### EXAMPLE 6: (+)-(trans)-(1-Aminomethyl-3,4-dimethyl-cyclopentyl)-acetic acid hydrochloride

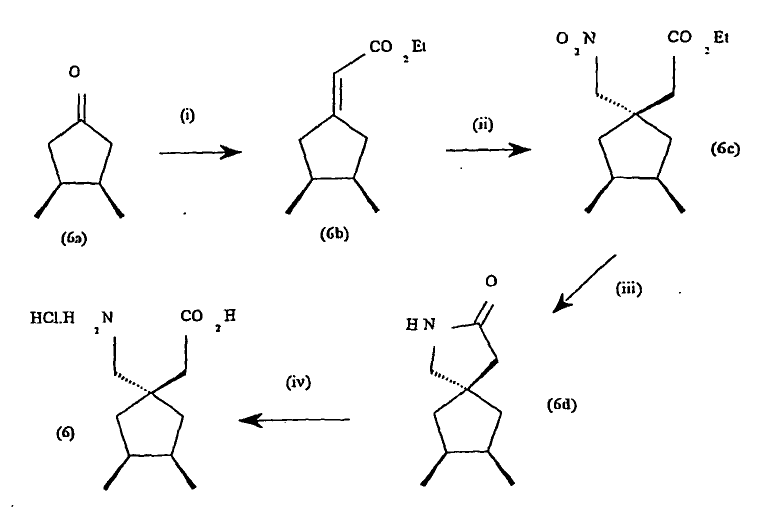

Reagents: (i) Triethylphosphonoacetate, NaH; (ii) MeNO₂, Bu₄N⁺F⁻; (iii) H₂, Ni; (iv) HCl

### Step i: Synthesis of (cis)-(3,4-Dimethyl-cyclopentylidene)-acetic acid ethyl ester (6b)

NaH (60% dispersion in oil, 519 mg, 12.96 mmol) was suspended in dry tetrahydrofuran (30 mL) and cooled to 0°C. Triethylphosphonoacetate (2.68 mL, 13.5 mmol) was added and the mixture stirred at 0°C for 15 minutes. (3S,4R)-3,4-Dimethyl-cyclopentanone **(6a)** (1.21 g, 10.80 mmol) in THF (10 mL) was then added and the mixture allowed to warm to room temperature. After 2 hours, the mixture was partitioned between diethyl ether (200 mL) and water (150 mL). The organic phase was separated, washed with brine, dried (MgSO₄) and the solvent removed in vacuo. The residue was purified by flash chromatography (silica, ethyl acetate:heptane 5:95) to give 1.40 g (71%) of (2) as a colorless oil.
¹H NMR 400 MHz (CDCl 13): δ 0.84 (3H, d, J = 6 Hz), 0.91 (3H, d, J = 6 Hz), 1.26 (3H, t, J = 7 Hz), 2.01-2.95 (6H, m), 4.13 (2H, q, J = 7 Hz), 5.76 (1H, s).
MS (CI+) m/e: 183 (M+I).
IR (film) ν cm⁻¹: 1043, 1125, 1200, 1658, 1715, 2959.

### Step ii: Synthesis of (trans)-(3,4-Dimethyl-1-nitromethyl-cyclopentyl)-acetic acid ethyl ester (6c)

(cis)-(3,4-Dimethyl-cyclopentylidene)-acetic acid ethyl ester (6b) (1.384 g, 7.60 mmol) was dissolved in tetrahydrofuran (10 mL) and stirred at 70°C with nitromethane (0.82 mL, 15.2 mmol) and tetrabutylammonium fluoride (1.0M in tetrahydrofuran, 11.4 mL, 11.4 mmol). After 6 hours, the mixture was cooled to room temperature, diluted with ethyl acetate (50 mL) and washed with 2N HCl (30 mL) followed by brine (50 mL). The organic phase was collected, dried (MgSO₄), and the solvent removed in vacuo. The residue was purified by flash chromatography (silica, ethyl acetate:heptane 5:95) to give 0.837 g (45%) of a clear oil.
¹H NMR 400 MHz (CDCl₃): δ 0.91 (6H, d, J = 6 Hz), 1.21-1.39 (5H, m), 1.98 (2H, m), 2.18 (2H, m), 2.64 (2H, s), 4.15 (2H, q, J = 7 Hz), 4.61 (2H, s).
MS (ES+) m/e: 244 (M+1).
IR (film) ν cm⁻¹: 1184, 1377, 1548, 1732, 2961.

### Step iii: Synthesis of (trans)-7,8-Dimethyl-spiro[4.4]nonan-2-one (6d)

(trans)-(3,4-Dimethyl-1-nitromethyl-cyclopentyl)-acetic acid ethyl ester (6c) (0.83 g, 3.4 mmol) was dissolved in methanol (30 mL) and shaken over Raney nickel catalyst under an atmosphere of hydrogen (40 psi i.e. 275.790 Kilopascals) at 30°C. After 4 hours, the catalyst was removed by filtration through celite. The solvent was removed in vacuo to give 567 mg (99%) of a pale yellow oil which solidified on standing.
¹H NMR 400 MHz (CDCl₃): δ 0.89 (6H, d, J = 6 Hz), 1.38 (2H, m), 1.91 (2H, m), 2.10 (2H, m), 2.32 (2H,s), 3.18 (2H, s), 5.61 (1H, br s).
MS (ES+) m/e: 168 (M+1).
IR (film) ν cm⁻¹: 1304, 1450, 1699, 2871, 3186.

### Step iv: Synthesis of (trans)-(1-Aminomethyl-3,4-dimethyl-cyclopentyl)-acetic acid hydrochloride (6)

(trans)-7,8-Dimethyl-spiro[4.4]nonan-2-one **(6d)** (563 mg, 4.40 mmol) was heated to reflux in a mixture of 1,4-dioxan (5 mL) and 6N HCl (15 mL). After 4 hours, the mixture was cooled to room temperature, diluted with water (20 mL), and washed with dichloromethane (3 × 30 mL). The aqueous phase was collected and the solvent removed in vacuo. The residue was triturated with ethyl acetate to give a white solid which was collected and dried. This was recrystallized from ethyl acetate/methanol to give 440 mg (59%) of (6) after collection and drying.
¹H NMR 400 MHz (d₆-DMSO): δ 0.84 (6H, d, J = 6 Hz), 1.21 (2H; m), 1.81 (2H, m), 2.06 (2H, m), 2.47 (2H, s), 2.59 (2H, s), 7.94 (3H, br s), 12.30 (1H, br s).
MS (ES+) m/e: 186 (M+1 for the free base).

### EXAMPLE 7 (+)-(trans)-(1-Aminomethyl-3,4-dimethyl-cyclopentyl)-acetic acid hydrochloride

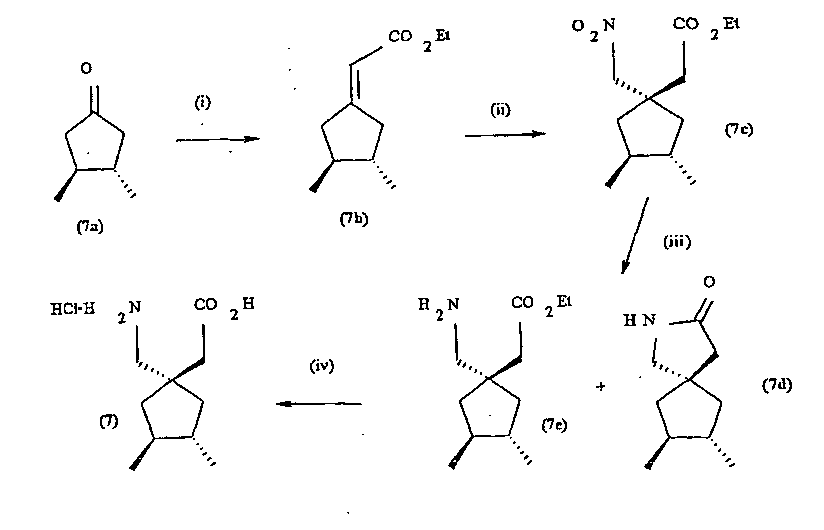

Reagents: (i) Triethylphosphonoacetate, NaH; (ii) MeNO₂, Bu₄N⁺F⁻; (iii) H₂, Ni; (iv) Hcl.

(3S,4S)-3,4-Dimethyl-cyclopentanone (7a) is known in the literature and can be synthesized by the methods outlined therein: Y. Kato, *Chem. Pharm. Bull.,* 1966;14:1438-1439 and related references: W. C. M. C. Kokke, F. A. Varkevisser, *J. Org. Chem*., 1974;39:1535; R. Baker, D. C. Billington, N. Eranayake, *JCS Chem. Comm*., 1981:1234; K. Furuta, K. Iwanaga, H. Yamamoto, *Tet. Lett*., 1986;27:4507; G. Solladie, O. Lohse, *Tet. Asymm*., 1993;4:1547; A. Rosenquist, I. Kvarnstrom, S. C. T. Svensson, B. Classon, B. Samuelsson, *Acta Chem. Scand.,* 1992;46:1127; E. J. Corey, W. Su, *Tet. Lett*., 1988;29:3423; D. W. Knight, B. Ojhara, *Tet. Lett*., 1981;22:5101.

### Step i: Synthesis of (trans)-(3,4-Dimethyl-cyclopentylidene)-acetic acid ethyl ester (7b)

To a suspension of sodium hydride (1.3 g, 32.5 mmol) in THF (60 mL) under nitrogen at 0°C was added triethylphosphonoacetate (6.5 mL, 32.7 mmol) over 5 minutes. After stirring for a further 10 minutes, a solution of (3S,4S)-3,4-Dimethyl-cyclopentanone (**7a**) (approx. 2.68 g, approx. 30 mmol) in THF (2 × 10 mL) was added to the now clear solution and the ice bath removed. After 4 hours the reaction was quenched by pouring into water (100 mL) and the mixture extracted with ether (400 mL). The organic phase was washed with saturated brine (100 mL), dried and concentrated in vacuo. Column chromatography (10:1 heptane/ethyl acetate) gave the product as an oil (4.53 g). (approx. 91 % yield)
¹H NNM 400 MHz (CDCl₃): δ 1.01 (3H, d, J = 6 Hz), 1.03 (3H, d, J = 6 Hz), 1.26 (3H, t, J = 7 Hz), 1.49 (2H, m), 2.07 (1H, m), 2.24 (1H, m), 2.61 (1H, m), 4.13 (2H, q, J = 7 Hz), 5.72 (1H, s).
MS (Cl+) m/e: 183 (M+1).

### Step ii: Synthesis of (trans)-(3,4-Dimethyl-1-nitromethyl-cyclopentyl)-acetic acid ethyl ester (7c)

To a solution of (trans)-(3,4-Dimethyl-cyclopentylidene)-acetic acid ethyl ester (7b) (4.24 g, 23.3 mmol) in THF (15 mL) was added TBAF (32 mL of a 1 M solution in THF, 32 mmol) followed by nitromethane (3 mL) and the reaction heated at 60°C for 8 hours. After cooling, the reaction mixture was diluted with ethyl acetate (150 mL) and washed with 2N HCl (40 mL) then saturated brine (50 mL). Column chromatography (10:1 heptane/ethyl acetate) gave the product as an oil (2.24 g). (40% yield)
¹H NMR 400 MHz (CDCl₃): δ 0.98 (6H, d, J = 6 Hz), 1.10-1.39 (5H, m), 1.47 (2H, m), 1.87 (1H, m), 2.03 (1H, m), 2.57 (2H, ABq, J = 16, 38 Hz), 4.14 (2H, q, J = 7 Hz), 4.61 (2H, ABq, J = 12, 60 Hz).
MS (ES+) m/e: 244 (M+1).
IR (film) ν cm⁻¹: 1186, 1376, 1549, 1732, 2956.

### Step iii: Synthesis of (+)-(trans)-(1-Aminomethyl-3,4-dimethyl-cyclopentyl)-acetic acid hydrochloride (7)

A solution of (trans)-(3,4-Dimethyl-1-nitromethyl-cyclopentyl)-acetic acid ethyl ester (**7c**) (3.5 g, 14.4 mmol) in methanol (100 mL) in the presence of Ni sponge was hydrogenated at 30°C and 50 psi i.e. 344.737 Kilopascals for 4 hours. Filtering off the catalyst and concentrating in vacuo gave a 2:1 mixture of lactam and aminoester, 2.53 g, calculated as 96%, which was used without purification. This mixture (2.53 g, 13.8 mmol) in dioxane (15 mL) and 6N HCl (45 mL) was heated under reflux (oil bath = 110°C) for 4 hours. After cooling and diluting with water (60 mL), the mixture was washed with dichloromethane (3 × 50 mL) and then concentrated in vacuo. The resulting oil was washed with ethyl acetate then dichloromethane to give a sticky foam which was dried to give the product as a white powder (2.32 g). (76% yield)
α_{D} (23 °C) (H₂O) (c = 1.002) = +28.2°.
¹H NMR 400 MHz (d₆-DMSO): δ 0.91 (6H, d, J = 6 Hz), 1.18 (2H, m), 1.42 (2H, m), 1.72 (1H, m), 1.87 (1H, m), 2.42 (2H, ABq, J = 16, 24 Hz), 2.90 (2H, ABq, J = 12, 34 Hz), 8.00 (3H, br s), 12.34 (1H, br s).
MS (ES+) m/e: 186 (M+1 for the free base).

### EXAMPLE 8 1α,3β,5β-(1-Aminomethyl-3,5-dimethyl-cyclohexyl)-acetic acid hydrochloride

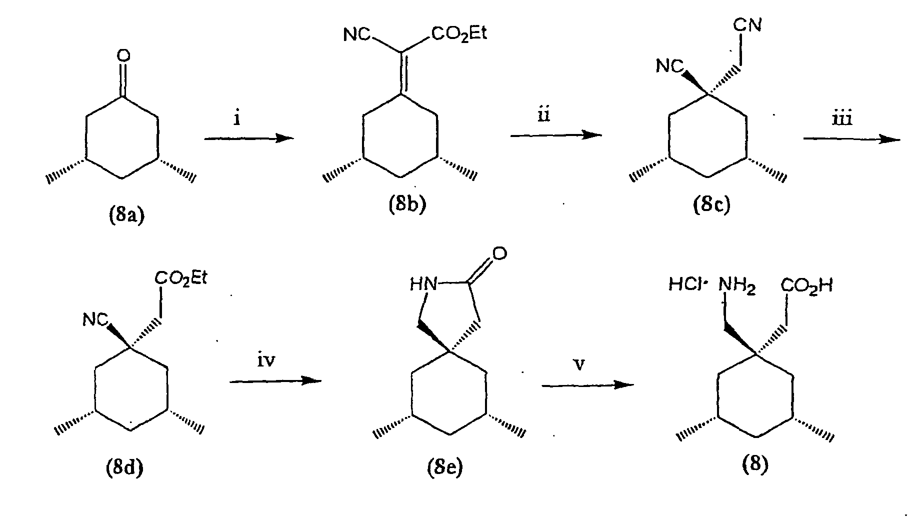

**Step i: Synthesis of 2-Cyano-((3R,5S)-3,5-dimethyl-cyclohexylidene)-acetic acid ethyl ester (8b):** Cis-3,5-dimethylcyclohexanone (8a, 15.78 g, 125.0 mmol) was dissolved in toluene (150 mL), and treated with ammonium acetate (0.96 g, 0.099 mmol), acetic acid (1.4 mL, 0.195 mmol), and ethyl cyanoacetate (14.04 g, 99.2 mmol). The reaction was heated to reflux overnight with a Dean-Stark trap, then cooled to room temperature and washed with H₂O (3 x 50 mL). The aqueous layer was backwashed with toluene (2 x 50 mL). The organic layers were combined, dried over Na₂SO₄, and concentrated. The residue was chromatographed on silica gel eluting with 5 % EtOAc/hexane to give 22.0 g (80%) of the desired product as a clear oil.
MS: 222.1 (M+1 for C₁₃H₁₉N₁O₂); an oil; TLC: SiO₂, R_{f} 0.40 (9:1 hexane/EtOAc); Analysis (C₁₃H₁₉N₁O₂) (Cal.) C: 70.56, H: 8.68, N: 6.33 (found) C: 7.0.74, H: 8.68, N: 6.33. ¹H NMR (CDCl₃) δ 0.83-0.95 (m, 1 H), 1.01 (t, 6 H, J = 6.1 Hz), 1.32 (t, 3 H, J = 7.3 Hz), 1.48-1.85 (m, 5 H), 2.97-3.02 (m, 1 H), 3.92 (dd, 1 H, J = 12.2, 2.2 Hz), 4.24 (q, 2 H, J = 7.1 Hz).

**Step ii: Synthesis of 1α,3β,5β-1-Cyanomethyl-3,5-dimethyl-cyclohexanecarbonitrile (8c):** The 2-Cyano-((3R,5S)-3,5-dimethyl-cyclohexylidene)-acetic acid ethyl ester **(8b,** 6.64 g, 30.0 mmol) was dissolved in EtOH (60 mL) and added to a suspension of NaCN (1.47 g, 30.0 mmol) in EtOH / H₂O (100 mL / 8 mL). The reaction was heated to reflux overnight, then cooled to room temperature, filtered, and concentrated in vacuo to give a clear oil which solidified under vacuum. The residue was chromatographed on silica gel eluting with 4:1 hexane/EtOAc to give 4.05 g (77%) of the desired product as a white, sticky solid.
MS: no M+1 ion observed (C₁₁H₁₆N₂); an oil; TLC: SiO₂, R_{f} 0.31 (4:1 hexane/EtOAc); Analysis (C₁₁H₁₆N₂) (Cal.) C: 74.96, H: 9.15, N: 15.89 (found) C: 74.81, H: 9.25, N: 15.65. ¹H NMR (CDCl₃) δ 0.58 (dd, 1 H, J = 24.9, 12.2 Hz), 0.96 (d, 6 H, J = 6.6 Hz), 1.03 (t, 2 H, J = 12.9 Hz), 1.75-1.90 (m, 3 H), 2.01 (d, 2 H, J = 11.5 Hz), 2.65 (s, 2 H).

**Step iii: Synthesis of 1α,3β,5β-(1-Aminomethyl-3,5-dimethyl-cyclohexyl)-acetic acid hydrochloride (8d):** 1α,3β,5β-1-Cyanomethyl-3,5-dimethyl-cyclohexanecarbonitrile (8c, 5.51 g, 31.26 mmol) was dissolved in EtOH (80 mL) and toluene (80 mL), cooled to 0 °C, and saturated with HCl gas. The solution was stoppered and stirred overnight at room temperature, then concentrated in vacuo to give the crude imino ester as a white solid. This crude material was triturated with Et₂O (150 mL) and dried under vacuum, then treated with H₂O (100 mL) and 1 N HCl (4 mL). The reaction was stirred overnight at room temperature, then extracted with EtOAc (3 x 100 mL). The organic layers were combined, washed with brine, dried over Na₂SO₄, and concentrated. The residue was chromatographed on silica gel eluting with 6:1 hexane/EtOAc to give 5.78 g (83%) of the desired product as a clear oil.
MS: 224.1 (M+1 for C₁₃H₂₁N₁O₂); an oil; TLC: SiO₂, R_{f} 0.32 (6:1 hexane/EtOAc); Analysis (C₁₃H₂₁N₁O₂) (Cal.) C: 69.92, H: 9.48, N: 6.27 (found) C: 70.03, H: 9.45, N: 6.30. ¹H NMR (CDCl₃) δ 0.53 (dd, 1 H, J = 24.9, 12.0 Hz), 0.91 (d, 6 H, J = 6.6 Hz), 1.27 (t, 3 H, J = 7.1 Hz), 1.70-1.76 (m, 1 H), 1.80-1.90 (m, 2 H), 2.02 (d, 2 H, J = 12.9 Hz), 2.51 (s, 2 H), 4.18 (q, 2 H, J = 7.1 Hz).

### Step iv: Synthesis of 5α,7β,9β-7,9-Dimethyl-2-aza-spiro[4.5]decan-3-one (8e):

1α,3β,5β-(1-Cyano-3,5-dimethyl-cyclohexyl)-acetic acid ethyl ester (8d, 5.82 g, 26.1 mmol) was dissolved in MeOH (150 mL), treated with 10% Rh / 2% Pd/C (1.0 g), and shaken under an atmosphere of H₂ (50 psi i.e. 344.737 Kilopascals) for 28 h. The reaction was filtered and concentrated in vacuo. The residue was chromatographed on silica gel eluting with a gradient of 2:3 hexane/EtOAc, EtOAc, 5% MeOH/EtOAc to give 2.21 g (47%) of the desired product.
MS: 182.0 (M+1 for C₁₁H₁₉N₁O₁); mp 116-118 °C; TLC: SiO₂, R_{f} 0.36 (6% MeOH/CH₂Cl₂); Analysis (C₁₁H₁₉N₁O₁) (Cal.) C: 72.88, H: 10.56, N: 7.73 (found) C: 72.84, H: 10.67, N: 7.63. ¹H NMR (CDCl₃) δ 0.49 (dd, 1 H, J = 24.4, 12.0 Hz), 0.86-0.93 (m, 8H), 1.38-1.48 (m, 2 H), 1.60-1.71 (m, 3 H), 2.09 (s, 2 H), 3.16 (s, 2 H), 5.79 (br, 1 H).

**Step v: Synthesis of 1α,3β,5β-(1-Aminomethyl-3,5-dimethyl-cyclohexyl)-acetic acid; hydrochloride salt (8):** 5α,7β,9β-7,9-Dimethyl-2-aza-spiro[4.5]decan-3-one (8e, 0.52 g, 2.87 mmol) was treated with 6 N HCl (19 mL) and heated to reflux overnight. The reaction was cooled to room temperature, upon which a white precipitate formed. The solid was filtered away, and the filtrate was cooled to 0 °C to afford a second crop of crystals, then filtered again. The solids were combined and dried under vacuum to give 0.46 g (68%) of the desired product.
MS: 200.1 (M+1 for C₁₁H₂₁N₁O₂); mp 179-180 °C; Analysis (C₁₁H₂₁N₁O₂.HCl) (Cal.) C: 56.04, H: 9.41, N: 5.94 (found) C: 56.16, H: 9.42, N: 5.73. ¹H NMR (CD₃OD) δ 0.46-0.53 (m, 1 H), 0.77 (t, 1 H, J = 12.5 Hz), 0.85-0.91 (m, 7 H), 1.55-1.67 (m, 5 H), 2.11 (d, 2 H, J = 13.4 Hz), 2.3 6 (s, 2 H), 2.44 (s, I H), 3.14 (s, 2 H).

### EXAMPLE 9 1α,3β,5β-(3,5-Dimethyl-1-propylaminomethyl-cyclohexyl)-acetic acid; hydrochloride salt:

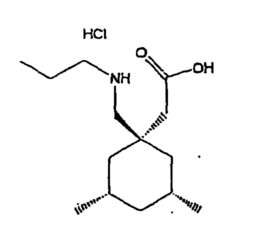

1α,3β,5β-(1-Aminomethyl-3,5-dimethyl-cyclohexyl)-acetic acid; hydrochloride salt (8, 1.0 g, 4.2 mmol) was dissolved in EtOH (100 mL), treated with sodium hydroxide solution (1 N, 5 mL), propionaldehyde (0.9 mL, 12.6 mmol), and 10 % Pd/C (0.3 g), and shaken under an atmosphere of H₂ (50 psi i.e. 344.737 Kilopascals) for 3 h. The reaction was filtered, treated with HCl/Et₂O (1 M, 10 mL), and concentrated in vacuo. The residue was treated with hot isopropanol (100 mL); the NaCl was filtered away, and the clear solution was concentrated in vacuo. The crude product was recrystallized from iPrOH/acetone/hexane (14: 200: 225 mL) and dried under vacuum to give 0.136 g (12%) ofthe.desired product.
MS: 242.1 (M+1 for C₁₄H₂₇N₁O₂); mp 178-180 °C; Analysis (C₁₄H₂₇N₁O₂.HCl.0.27 H₂O) (Cal.) C: 59.48, H: 10.18, N: 4.95 (found) C: 59.53, H: 10.21, N: 4.78. ¹H NMR (CD₃OD) δ 0.52 (dd, 1 H, J = 24.2, 12.0 Hz), 0.83-0.90 (m, 8 H), 1.00 (t, 3 H, J = 7.6 Hz), 1.61-1.80 (m, 7 H), 2.43 (s, 2 H), 2.98 (t, 2 H, J = 7.8 Hz), 3.20 (s, 2 H).

### EXAMPLE 10 1α,3β,5β-(1-Ethylaminomethyl-3,5-dimethyl-cyclohexyl)-acetic acid hydrochloride salt

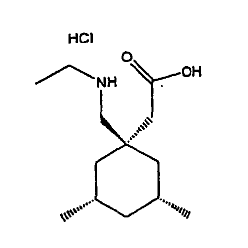

Example 10 was synthesized in accordance with the method of Example 9, except that acetaldehyde was used instead of propionaldehyde and ethyl acetate/heptane was used for the recrystallization (30% yield).
MS: 228 (M+1 for C₁₅H₂₉N₁O₂); mp 188-190 °C; ¹H NMR (DMSO) δ 0.83 (8H, br d), 1.24 (3 H, t, J = 7.1 Hz), 1.60 (6 H, m), 2.38 (d, s), 2.98 (2 H, m), 3.10 (2 H, br s), 8.33 (2 H, br s), 12.42 (1 H, br s).

### EXAMPLE 11 1α,3β,5β-(1-Benzylamino-methyl)-3,5-dimethyl-cyclohexyl]-acetic acid; hydrochloride salt:

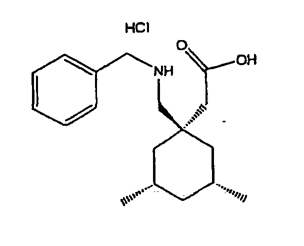

Example 11 was synthesized in accordance with the method of Example 9, except that benzaldehyde was used instead of propionaldehyde and Et₂O was used for the recrystallization (30% yield).
MS: 290.1 (M+1 for C₁₈H₂₇N₁O₂); mp 162-163 °C; Analysis (C₁₈H₂₇N₁O₂.HCl.0.20H₂O) (Cal.) C: 65.62, H: 8.69, N: 4.25 (found) C: 65.60, H: 8.71, N: 4.31. ¹H NMR (CD₃OD) δ 0.48 (dd, 1 H, J = 12.5 Hz), 0.79-0.90 (m, 8 H), 1.46 (br, 2 H), 1.64 (d, 3 H, J = 13.4 Hz), 2.42 (s, 2 H), 3.19 (s, 2 H), 4.23 (s, 2 H), 7.43-7.50 (m, 5 H).

### EXAMPLE 12 1α,3β,5β-(1-Dimethylaminomethyl-3,5-dimethyl-cyclohexyl)-acetic acid; hydrochloride salt:

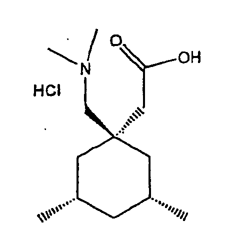

A solution of 1α,3β,5β-(1-Aminomethyl-3,5-dimethyl-cyclohexyl)-acetic acid; hydrochloride salt (8, 0.20 g, 0.59 mmol) in EtOH (15 mL), sodium hydroxide solution (1 N, 0.59 mmol), and B₂O (1 mL) was treated with 20% Pd/C (50 mg) and shaken under an atmosphere of H₂ (50 psi i.e. 344.737 Kilopascals) for 15 hours. The reaction was filtered, treated with 1 N HCl/Et₂O (1 mL), and concentrated in vacuo. The residue was dissolved in hot i-PrOH (4 mL), diluted with acetone (100 mL) and Et₂O (350 mL) and cooled to 0 °C. The solid was filtered and dried under vacuum to give 103 mg (70% yield) of the desired product..
MS: 228.1 (M+1 for C₁₂H₂₃N₁O₂); mp 195-196 °C; Analysis (C₁₂H₂₃N₁O₂.HCl.0.41H₂O) (Cal.) C: 57.58, H: 9.97, N: 5.16 (found) C: 57.73, H: 9.71, N: 4.91. ¹H NMR (CD₃OD) δ 0.56 (dd, 1 H, J = 24.4, 12.0 Hz), 0.89-0.92 (m, 8 H), 1.59-1.75 (m, 8 H), 2.51 (s, 2 H), 2.95 (s, 6 H), 3.44 (s, 2 H).

### EXAMPLE 13 1α,3β,5β-(1-Butylaminomethyl-3,5-dimethyl-cyclohexyl)-acetic acid; hydrochloride salt:

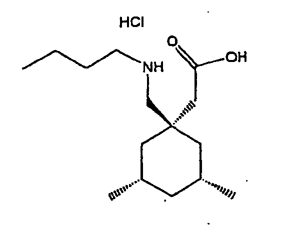

Example 13 was synthesized in accordance with the method of Example 9, except butyraldehyde was used instead of propionaldehyde (48% yield).
MS: 256.1 (M+1 for C₁₅H₂₉N₁O₂); mp 184-185 °C; Analysis (C₁₅H₂₉N₁O₂.HCl) (Cal.) C: 61.73, H: 10.36, N: 4.80 (found) C: 61.71, H: 10.21, N: 4.72. ¹H NMR (CD₃OD) δ 0.53 (dd, 1 H, J = 24.4, 12.0 Hz), 0.86-0.99 (m, 11 H), 1.37-1.47 (m, 2 H), 1.60-1.75 (m, 7 H), 2.43 (s, 2 H), 3.02 (t, 2 H, J = 8.1 Hz), 3.20 (s, 2 H).

### EXAMPLE 14 1α,3β,5β-{1-[(Benzyl-methyl-amino)-methyl]-3,5-dimethylcyclohexyl}-acetic acid; hydrochloride salt:

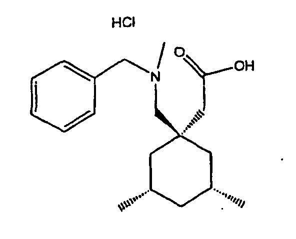

Example 14 was prepared in accordance with the method of Example 9, except that the N-benzyl analog (11) and formaldehyde were used as the starting material and PtO₂ was used as the catalyst instead ofPd/C (70% yield).
MS: 304.1 (M+1 for C₁₉H₂₉N₁O₂); mp 209-210 °C; Analysis (C₁₉H₂₉N₁O₂.HCl.0.21H₂O) (Cal.) C: 66.40, H: 8.92, N: 4.08 (found) C: 66.40, H: 9.05, N: 4.06. ¹H NMR (CD₃OD) δ 0..51 (dd, 1 H, J = 24.4, 12.2 Hz), 0.79-0.92 (m, 9 H), 1.29 (br, 1 H), 1.56-1.82 (m, 4 H), 2.51 (dd, 2 H, J = 18.8, 16.3 Hz) 2.85 (s, 2 H), 3.23-3.49 (m, 2 H), 4.37 (dd, 2 H, J = 31.7, 12.7 Hz), 7.45-7.54 (m, 5 H).

### EXAMPLE 15 1α,3β,5β-(3,5-Dimethyl-1-methylaminomethyl-cyclohexyl)-acetic acid; hydrochloride salt:

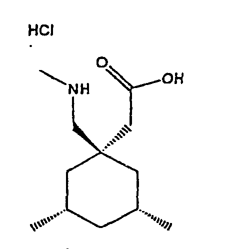

Example 14 (0.14 g, 1.21 mmol) was dissolved in 95% EtOH (50 mL), treated with sodium hydroxide solution (1 N, 1.21 mmol) as well as 20% Pd/C (0.1 g), and shaken under an atmosphere of H₂ (50 psi i.e. 344.737 Kilopascals) for 45 minutes. The reaction was filtered, treated with HCl/Et₂O (1 M, 1 mL), and concentrated in vacuo. The residue was treated with hot iPrOH, the NaCl was filtered away, and the solution was concentrated in vacuo. The crude product was recrystallized from iPrOH/acetone/hexane (7: 150: 110 mL) and dried under vacuum to give 0.189 (63%) of the desired product.
MS: 214.1 (M+1 for C₁₂H₂₃N₁O₂); mp 182-183 °C; Analysis (C₁₂H₂₃N₁O₂.HCl) (Cal.) C: 57.70, H: 9.68, N: 5.61 (found) C: 57.47, H: 9.58, N: 5.35. ¹H NMR (CD₃OD) δ 0.52 (dd, 1 H, J = 24.4, 12.2 Hz), 0.84-0.98 (m, 8 H), 1.57-1.72 (m, 5 H), 2.38 (s, 2 H), 2.72 (s, 3 H), 3.21 (s, 2 H).

### EXAMPLE 16 1α,3β,5β-[1-(Acetylamino-methyl)-3,5-dimethyl-cyclohexyl]-acetic acid:

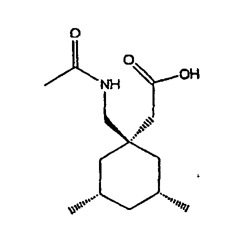

Example 8 (0.5 g, 2.1 mmol) was treated with H₂O (10 mL) and NaOH (0.56 g, 14.0 mmol), cooled to 0 °C, and treated with acetic anhydride. The reaction was stirred overnight at room temperature, then diluted with EtOAc (100 mL) and washed with 1 N HCl (100 mL). The organic layer was dried over Na₂SO₄ and concentrated in vacuo. The crude material was chromatographed on silica gel eluting with 4% MeOH/CH₂Cl₂ to give 0.138 g (27%) of the desired product as an oil which solidified under vacuum.
MS: 242.1 (M+1 for C₁₃H₂₃N₁O₃); mp 122-124 °C; Analysis (C₁₃H₂₃N₁O₃) (Cal.) C: 64.70, H: 9.61, N: 5.80 (found) C: 64.65, H: 9.59, N: 5.63. ¹H NMR (CD₃OD) δ 0.46 (dd, 1 H, J = 23.7, 11.7 Hz), 0.81-0.87 (m, 9 H), 1.56 (d, 2 H, J = 13.4 Hz), 1.65-1.76 (m, 3 H), 1.98 (s, 3 H), 2.18 (s, 2 H), 3.30 (s, 3 H).

### EXAMPLE 17 1α,3β,5β-(1-(Isobutylamino-methyl)-3,5-dimethyl-cyclohexyl]-acetic acid hydrochloride salt

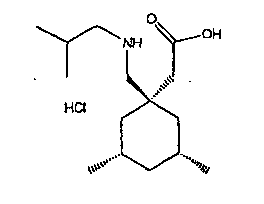

Example 17 was synthesized in accordance with the method of Example 9, except isobutyraldehyde was used instead of propionaldehyde (53% yield).
MS: 256 (M+1 for C₁₅H₂₉N₁O₂); mp 193-194 °C; ¹H NMR (CD₃OD) δ 0.53 (dd, 1 H, J = 24.3, 12.5 Hz), 0.81-0.90 (m, 2 H), 0.89 (d, 6H, J = 6.4 Hz), 1.03 (d, 6H, J = 6.8 Hz), 1.57-1.71 (m, 5H), 2.09 (m, 1H), 2.49 (s, 2H), 2.88 (d, 2H, J = 7.1 Hz), 3.19 (s, 2 H).

### EXAMPLE 18 1α,3β,5β-[3,5-Dimethyl-1-(phenethylamino-methyl)-cyclohexyl]-acetic acid hydrochloride salt:

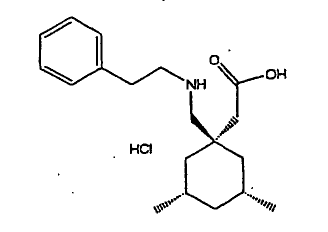

Example 18 was synthesized in accordance with the method of Example 9, except phenylacetaldehyde was used instead of propionaldehyde (16% yield).
MS: 304 (M+1 for C₁₉H₂₉N₁O₂); mp 188-189 °C; Analysis (C₁₉H₂₉N₁O₂.HCl) (Cal.) C: 67.14, H: 8.90, N: 4.12 (found) C: 66.94, H: 8.91, N: 3.98. ¹H NMR (CD₃OD) δ 0.46 (dd, 1 H, J = 24.6, 12.3 Hz), 0.81-0.91 (m, 8 H), 1.58-1.72 (m, 5 H), 2.50 (s, 2H), 3.04-3.08 (m, 2H), 3.26-3.30 (m, 4H), 7.23-7.75 (m, 5H).

### EXAMPLE 19 1α,3β,5β-{3,5-Dimethyl-1-[(3-phenyl-propylamino)-methyl]-cyclohexyl}-acetic acid hydrochloride salt:

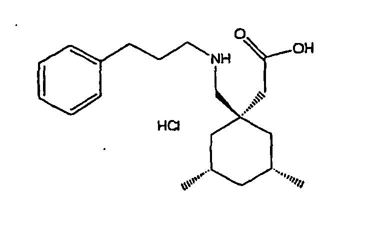

Example 19 was synthesized in accordance with the method of Example 9, except hydrocinnamaldehyde was used instead of propionaldehyde (50% yield).
MS: 318 (M+1 for C₂₀H₃₁N₁O₂); mp 171-172 °C; Analysis (C₂₀H₃₁N₁O₂.HCl) (Cal.) C: 67.87, H: 9.11, N: 3.96 (found) C: 67.83, H: 9.13, N: 3. 78. ¹H NMR (CD₃OD) δ 0.53 (dd, 1 H, J = 24.6, 12.1 Hz), 0.80-0.90 (m, 2 H), 0.90 (d, 6H, J = 6.0 Hz), 1.60-1.72 (m, 5 H), 2.03-2.11 (m, 2H), 2.45 (s, 2H), 2.73 (t, 2H, J = 7.5 Hz), 3.06 (t, 2H, J = 7.7 Hz), 3.3 (s, 2H), 7.20-7.18 (m, 5H).

### EXAMPLE 20 {1-[(Cyclobutylmethyl-amino)-methyl]-cyclohexyl}-acetic acid hydrochloride salt:

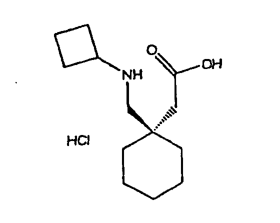

Example 20 was synthesized in accordance with the method of Example 26, except cyclobutanone was used instead of 2-methylbutyraldehyde (70% yield).
MS: 226 (M+1 for C₁₃H₂₃N₁O₂); mp 176-177 °C; Analysis (C₁₃H₂₃N₁O₂.HCl) (Cal.) C: 59.64, H: 9.24, N: 5.35 (found) C: 59.88, H: 9.18, N: 5.19. ¹H NMR (CD₃OD) δ 1.16-1.60 (br., 10 H); 1.80-1.96 (m, 2 H), 2.21-2.40 (m, 4 H), 2.53 (s, 2H), 2.97 (s, 2H), 3.73 (m, 1H).

### EXAMPLE 21 1α,3β,5β-(1-(Isopropylamino-methyl)-3,5-dimethyl-cyclohexyl]-acetic acid hydrochloride salt:

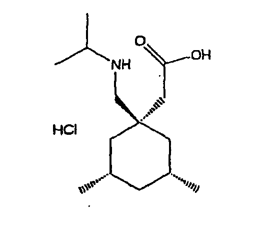

Example 21 was synthesized in accordance with the method of Example 9, except acetone was used instead of propionaldehyde (50% yield).
MS: 242 (M+1 for C₁₄H₂₇N₁O₂); mp 206-207 °C; ¹H NMR (CD₃OD) δ 0.53 (dd, 1 H, J = 24.8, 12.5 Hz), 0.80-0.90 (m, 2 H), 0.99 (d, 6H, J = 6.1 Hz), 1.35 (d, 6H, J = 6.6 Hz), 1.55-1.78 (m, 5H), 2.45 (s, 2H), 3.18 (s, 2H), 3.41 (m, 1H).

### EXAMPLE 22 1-Aminomethyl-1-cyclohexane-acetic acid was previously reported in Satzinger et al. US 4087544, May 2, 1978.

### EXAMPLE 23 1-Aminomethyl-1-cyclopentane-acetic acid was previously reported in Satzinger et al. US 4087544, May 2, 1978.

### EXAMPLE 24 Sodium salt of 1-aminomethyl-1-cyclopentane-acetic acid was previously reported in Satzinger et al. US 4087544, May 2, 1978.

### EXAMPLE 25 Sodium salt of 1-(hydroxymethyl)cyclohexane-acetic acid was previously reported in Satzinger et al. US 2960441, 1960.

### EXAMPLE 26 Synthesis of {1-[(2-Methyl-butylamino)-methyl]-cyclohexyl}-acetic acid; hydrochloride salt :

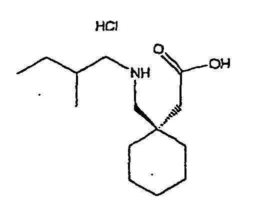

A solution of (1-aminomethyl-cyclohexyl)-acetic acid (Example 22, 2.0 g, 11.7 mmol) in EtOH (100 mL) was treated with 2-methyl-butyraldehyde .(4 mL, 35.1 mmol) and PtO₂ (0.5 g), and shaken under at atmosphere of H₂ (50 psi i.e. 344.737 Kilopascals) for 2 h. The reaction was filtered, treated with 1 N HCl/Et₂O (12 mL), concentrated in vacuo, dissolved in hot i-PrOH (10 mL), diluted with acetone (600 mL), cooled to 0 °C, and filtered to give 1.58 g (49%) of the desired product.
MS: 242.1 (M+1 for C₁₄H₂₇N₁O₂); mp 162-163 °C; Analysis (C₁₄H₂₇N₁O₂.HCl) (Cal.) C: 60.52, H: 10.16, N: 5.04 (found) C: 60.41, H: 10.26, N: 4.95. ¹H NMR (CD₃OD) δ 0.95 (t, 3 H, J = 7.3 Hz), 1.02 (d, 3 H, J= 6.6 Hz), 1.20-1.31 (m, 1 H), 1.42-1.56 (m, 11 H), 1.81-1.89 (m, 1 H), 2.63 (dd, 2 H, J = 18.3, 16.1 Hz), 2.82 (dd, 1 H, J = 12.5, 8.3 Hz), 2.99 (12.5, 6.1 Hz), 3.07 (dd, 2 H, J = 25.6, 12.9 Hz).

### EXAMPLE 27 {1-[(4,4,4-Trifluoro-butylamino)-methyl]-cyclohexyl}-acetic acid;

hydrochloride salt was synthesized in accordance with the method of Example 26, except that 4-trifluorobuyraldehyde was used instead of 2-methylbutyraldehyde (19% yield).
MS: 282.1 (M+1 for C₁₃H₂₂N₁O₂F₃); mp: 163-164 °C; Analysis (C₁₃H₂₂N₁O₂F₃.HCl.0.05H₂O) (Cal.) C: 49.00, H: 7.31, N: 4.40 (found) C: 48.60, H: 7.29, N: 4.19. ¹H NMR (CD₃OD) δ 1.40-1.54 (m, 11 H), 1.96-2.04 (m, 2 H), 2.26-2.38 (m, 2 H), 2.57 (s, 2H), 3.09-3.13 (m, 4 H).

### EXAMPLE 28 (1-Ethylaminomethyl-cyclohexyl)-acetic acid hydrochloride salt

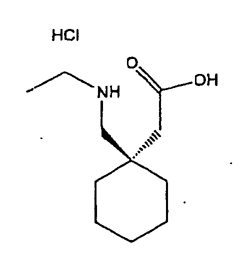

Example 28 was synthesized in accordance with the method of Example 26, except that acetaldehyde was used instead of 2-methylbutyraldehyde (30% yield).
MS: 200.1 (M+1 for C₁₁H₂₁N₁O₂); mp: 160-166 °C; Analysis (C₁₁H₂₁N₁O₂.HCl) (Cal.) C: 56.04, H: 9.41, N: 5.94 (found) C: 55.98, H: 9.36, N: 5.79. ¹H NMR (CD₃OD) δ 1.31-1.54 (m, 13 H), 2.54-2.56 (m, 2 H), 3.06-3.13 (m, 4 H).

### EXAMPLE 29 {1-[(Cyclopropylmethyl-amino)-methyl]-cyclohexyl}-acetic acid hydrochloride salt:

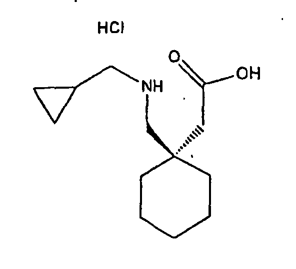

Example 29 was synthesized in accordance with the method of Example 26, except that cyclopropanecarboxaldehyde was used instead of 2-methylbutyraldehyde (62% yield).
MS: 226 (M+1 for C₁₃H₂₃N₁O₂); mp 177-178 °C; Analysis (C₁₃H₂₃N₁O₂.HCl) (Cal.) C: 59.64, H: 9.24, N: 5.35 (found) C: 59.72, H: 9.15, N: 5.27.¹H NMR (CD₃OD) δ 0.50-0.56 (m, 2 H), 0.71-0.74 (m, 2H), 1.05-1.30 (m, 1H), 1.42-1.54 (m, 10H), 2.52 (s, 2H), 2.94 (d, 2H, J = 7.3 Hz), 3.10 (s, 2H).

### EXAMPLE 30 {1-[(2-Hydroxy-1-methyl-ethylamino)-methyl]-cyclohexyl}-acetic acid hydrochloride salt:

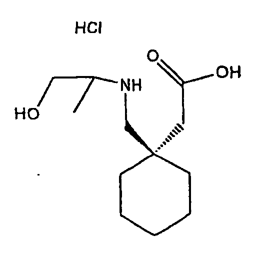

Example 30 was synthesized in accordance with the method of Example 26, except that 1-hydroxy-2-propanone was used instead of 2-methylbutyraldehyde (60% yield).
MS: 230 (M+1 for C₁₂H₂₃N₁O₃); mp 164-165 °C; Analysis (C₁₂H₂₃N₁O₃.HCl) (Cal.) C: 54.23, H: 9.10, N: 5.27 (found) C: 54.23, H: 9.01, N: 5.18.¹H NMR (CD₃OD) δ 1.30-1.32 (m, 3 H), 1.44-1.55 (m, 10H), 2.60 (s; 2H), 3.11 (m, 2H), 3.28 (br., 2H), 3.37 (br., 1H), 3.57-3.61 (m, 1H), 3.78-3.81 (m, 1H).

### EXAMPLE 31 [1-(Isobutylamino-methyl)-cyclohexyl]-acetic acid hydrochloride salt:

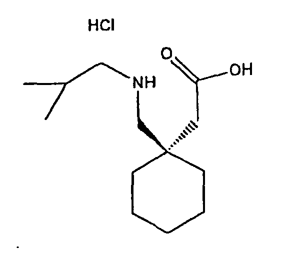

Example 31 was synthesized in accordance with the method of Example 26, except that cyclopropanecarboxaldehyde was used instead of 2-methylbutyraldehyde (35% yield).
MS: 228 (M+1 for C₁₃H₂₅N₁O₂); mp 175-176 °C; Analysis (C₁₃H₂₅N₁O₂.HCl) (Cal.) C: 59.19, H: 9.93, N: 5.51, (found) C: 59.28, H: 9.98, N: 5.24.¹H NMR (CD₃OD) δ 1.03 (d, 6H, J = 6.9 Hz), 1.37-1.60 (m, 10H), 2.10-2.17 (m, 1H), 2.63 (s, 2H), 2.87 (d, 2H, J = 7.4 Hz), 3.07 (s, 2H).

### EXAMPLE 32 (1-Propylaminomethyl-cyclohexyl)-acetic acid hydrochloride salt:

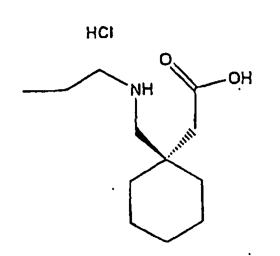

Example 32 was synthesized in accordance with the method of Example 26, except that propionaldehyde was used instead of 2-methylbutyraldehyde.
MS: 214.1 (M+1 for C₁₂H₂₃N₁O₂); mp: 174-177 °C; Analysis (C₁₂H₂₃N₁O₂.HCl) (Cal.) C: 57.70, H: 9.69, N: 5.61 (found) C: 57.55, H: 9.77, N: 5.48. ¹H NMR (CD₃OD) δ 0.93 (t, 1 H, J = 6.3 Hz), 0.99-1.03 (m, 4 H), 1.40-1.54 (m, 13 H), 1.72-1.78 (m, 2 H), 2.96-3.29 (m, 2 H).

### EXAMPLE 33 [1-(Isopropylamino-methyl)-cyclohexyl]-acetic acid hydrochloride salt:

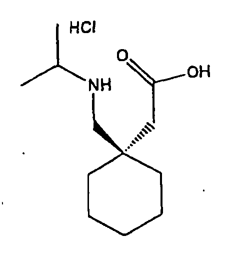

Example 33 was synthesized in accordance with the method of Example 26, except that acetone was used instead of 2-methylbutyraldehyde.
MS: 214.1 (M+1 for C₁₂H₂₃N₁O₂); mp: 193-194 °C; ¹H NMR (CD₃OD) δ 1.29-1.57 (m, 18 H), 2.59 (s, 2 H), 3.07 (s, 2 H), 3.38-3.42 (m, 1 H).

### EXAMPLE 34 (1-Cyclohexylaminomethyl-cyclohexyl)-acetic acid hydrochloride salt:

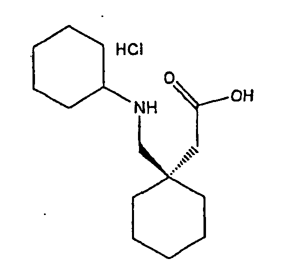

Example 34 was synthesized in accordance with the method of Example 26, except that cyclohexanone was used instead of 2-methylbutyraldehyde (48% yield).
MS: 254 (M+1 for C₁₅H₂₇N₁O₂); mp 198-199 °C; Analysis (C₁₅H₂₇N₁O₂.HCl) (Cal.) C: 62.12, H: 9.74, N: 4.83 (found) C: 62.18, H: 9.84, N: 4.75. ¹H NMR (CD₃OD) δ 1.20-1.60 (m, 16 H), 1.63-1.76 (m, 1H), 1.88 (br., 2H), 2.13 (br., 2H), 2.60 (m., 2H), 3.09 (m., 3H).

### EXAMPLE 35 [1-(Benzylamino-methyl)-cyclohexyl]-acetic acid hydrochloride salt:

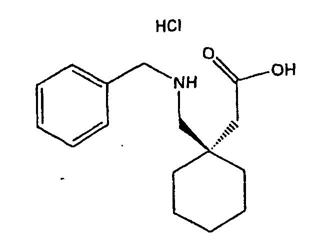

Example 35 was synthesized in accordance with the method of Example 26, except that benzaldebyde was used instead of 2-methylbutyraldehyde.
MS: 262.0 (M+1 for C₁₆H₂₃N₁O₂); mp: 125-135 °C; ¹H NMR (CD₃OD) δ 1.38-1.49 (m, 11 H), 2.55 (s, 2 H), 3.07 (s, 2 H), 4.23 (s, 2 H), 7.43-7.49 (m, 5 H).

### EXAMPLE 36. ((1R,3R)-3-Methyl-1-propylaminomethyl-cyclohexyl)-acetic acid; hydrochloride salt

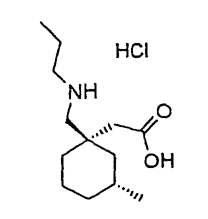

((1R, 3R)-1-Aminomethyl-3-methyl-cyclohexyl)-acetic acid; hydrochloride salt (Example 1, 1.1 g, 4.9 mmol) was dissolved in ethanol (95 mL) and treated with 1 N NaOH (5 mL). Propionaldehyde (4.9 mL) was added followed by 10% Pd/C (0.5 g), and the reaction was shaken under an atmosphere of H₂ (50 psi i.e. 344.737 Kilopascals) for 15.75 h. The reaction was filtered, 1 M HCl (20 mL) in ether was added, and the solution was concentrated in vacuo. The residue was then dissolved in hot isopropanol (100 mL), filtered, and concentrated in vacuo. The residue was then dissolved in isopropanol (10 mL) and recrystallized from acetone. The solid was filtered and the filtrate was cooled overnight to yield a second crop of crystals. The solids were combined to give the desired product (0.78 g, 66%).
MS: 228.1 (M+1 for C₁₃H₂₅NO₂); mp: 179-179.5 °C; Analysis (C₁₃H₂₅NO₂.HCl) (Cal.) C: 59.19, H: 9.93, N: 5.31; (found) C: 59.27, H: 9.97, N: 5.07. IR (KBr, cm⁻¹): 2929 (br), 1717, 1188. ¹H NMR (CD₃OD) δ 0.84-1.03 (m, 8H), 1.12-1.17 (m, 1H) 1.46-1.79 (m, 8H), 2.43 (s, 2H), 3.00 (t, 2H, J = 7.8 Hz) 3.22 (s, 2H).

### EXAMPLE 37. {1-[Cyclopentylmethyl-amino)-methyl]-cyclohexyl}-acetic acid; hydrochloric salt

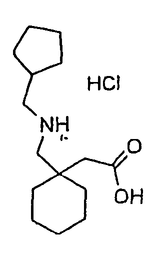

(1-Aminomethyl-cyclohexyl)-acetic acid (3.0 g, 17.5 mmol) was dissolved in ethanol (45 mL). Cyclopentanecarbaldehyde (5 mL, 3 eq) was added and the reaction was treated with PtO₂ (0.25 g). The reaction was then shaken under an atmosphere of H₂ (50 psi i.e. 344.737 Kilopascals) for 16.5 h. The reaction was filtered, 1N HCl (20 mL) in ether was added, and the reaction was concentrated in vacuo. The residue was recrystallized from hot isopropanol and acetone to give the desired product (2.32 g, 52%).
MS: 254.1 (M+1 for C₁₅H₂₇NO₂); mp 172-173 °C; Analysis (C₁₅H₂₇NO₂.HCl) (Cal.) C: 62.16, H: 9.74, N: 4.83; (found) C: 61.92, H: 9.88, N: 4.86. IR (KBr, cm⁻¹): 2930, 2854, 2509, 1689, 1455, 1211. ¹H NMR (CD₃OD) δ 1.23-1.32 (m, 2H), 1.41-1.73 (m, 16H), 1.90 (m, 1H), 2.63 (s, 2H), 3.01 (d, 2H, J = 7.6 Hz), 3.09 (s, 2H).

### EXAMPLE 38. {1-[(Cyclohexylmethyl-amino)-methyl]-cyclohexyl}-acetic acid; hydrochloric salt

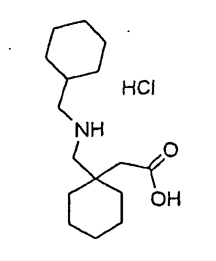

(1-Aminomethyl-cyclohexyl)-acetic acid (3.0 g, 17.5 mmol) was dissolved in ethanol (45 mL). Cyclohexanecarbaldehyde (5 mL, 3 eq) were added and the reaction was treated with PtO₂ (0.25 g). The reaction was then shaken under an atmosphere of H₂ (50 psi i.e. 344.737 Kilopascals) for 16.5 h. The reaction was filtered, 1N HCl (20 mL) in ether was added, and the reaction was concentrated in vacuo. The residue was recrystallized from hot isopropanol and ether to give the desired product (3.57 g, 67%).
MS: 268.1 (M+1 for C₁₆H₂₉NO₂); mp 167.5-168 °C; Analysis (C₁₆H₂₉NO₂.HCl) (Cal.) C: 63.24, H: 9.95, N: 4.61; (found) C: 63.14, H: 10.01, N: 4.45. IR (KBr, cm⁻¹): 2926, 2851, 1718, 1684. ¹H NMR (CD₃OD) δ 1.03 (m, 1H), 1.20-1.85 (m, 20H), 2.63 (s, 2H), 2.88 (d, 2H, J = 6.8 Hz), 3.07 (s, 2H).

### EXAMPLE 39: [1-(tert-Butoxycarbonylamino-methyl)-cyclohexyl]-acetic acid;

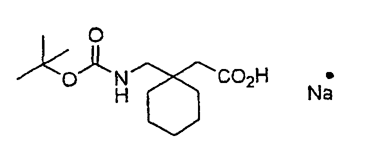

(1-Aminomethyl-cyclohexyl)-acetic acid (10.0 g, 58.4 mmol) was dissolved in THF (280 mL), and it was treated with 1 N NaOH (128.5 mL). The reaction was cooled to 0 °C and 14.0 g (1.1 eq) of di-tert-butyldicarbonate were added portionwise with vigorous stirring. The reaction was allowed to warm to room temperature and stir overnight. The THF was then evaporated, and the aqueous layer was washed with ethyl acetate (3x50 mL). The aqueous layer was acidified with KH₂PO₄ (44 g) and foam was observed. Gradually the foam was replaced with a white ppt. The mixture was stirred for 2h while of KH₂PO₄ (1 g) was added slowly over time. The precipitate was filtered off and washed with water to yield the desired product (15.2 g, 96%).
MS: 272.1 (M+1 for C₁₄H₂₅NO₄); mp: 181 °C (dec); Analysis (C₁₄H₂₅NO₄) (Cal.) C: 59.52, H: 9.36, N: 4.76; (found) C: 59.51, H: 9.03, N: 4.87. IR (KBr, cm⁻¹): 3413, 3054, 2933 (br), 1709, 1666, 1531, 1248; ¹H NMR (CDCl₃) δ 1.27-1.58 (m, 19H), 2.29 (s, 2H), 3.12 (d, 2H, J = 6.8 Hz), 4.97 (br, 1 H).

### EXAMPLE 40: [1-(Acetylamino-methyl)-cyclohexyl]-acetic acid

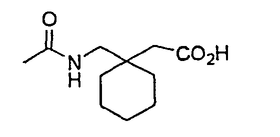

(1-Aminomethyl-cyclohexyl)-acetic acid (2.0 g, 11.7 mmol) was dissolved in H₂O (20 mL), then NaOH (3.1 g, 77.5 mmol, 6.6 eq) and acetic anhydride (1.8 mL, 19.3 mmol, 1.7 eq) were added. The reaction was allowed to stir at room temperature for 2 days. 1N HCl (125 mL) was added and the reaction extracted with EtOAc (125 mL). The organic layer was separated, dried over Na₂SO₄, and concentrated in vacuo. The residue was dissolved in H₂O (100 mL), treated with NaOH (0.12 g), and concentrated in vacuo. The sample was then flash chromatographed with 6% MeOH/ CH₂Cl₂ to give the desired product (0.31 g, 12 %).
MS: 214.1 (M+1 C₁₁H₁₉NO₃); mp: 107-108°C; Analysis (C₁₁H₁₉NO₃) (cal) C: 61.95 H: 8.98 N: 6.57 (found) C: 61.96 H: 9.03 N: 6.54; TLC 0.31 6% MeOH/ CH₂Cl₂; IR (KBr, cm⁻¹): 3286, 3120, 2932, 24401714, 1576, 1419, 1313, 1208. ¹H NMR (CDCl₃) δ 1.25-1.51 (m, 10 H), 2.06 (s, 3H), 2.28 (s, 2H), 3.26-3.29 (m, 2H), 6.32 (br, 1 H).

### EXAMPLE 41: ((3R, 5S)-1-Cyclobutylaminomethyl-3,5-dimethyl-cyclohexyl)-acetic acid; hydrochloride salt

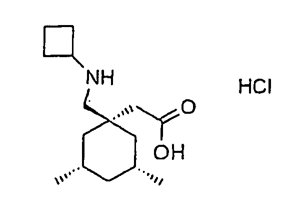

((3R, 5S)-1-Aminomethyl-3,5-dimethyl-cyclohexyl)-acetic acid; hydrochoride salt (0.76 g, 3.2 mmol) was dissolved in ethanol (45 mL) and treated with 1N NaOH (3.2 mL). Cyclobutanone (1 mL, 6 eq) was added followed by PtO₂ (0.2 g). The reaction was shaken under an atmosphere of H₂ (50 psi i.e. 344.737 Kilopascals) for 16 h. The reaction was then filtered, 1N HCl in ether (35 mL) was added, and the reaction was concentrated in vacuo. The residue was dissolved in hot isopropanol (150 mL) and filtered. Then acetone (100 mL) and ether (600 mL) that had been cooled to 0 °C were added to the solution. The solution was allowed to sit at 0 °C for 2 h at which time crystals were observed. The crystals were collected and washed with ether and acetone (0.38 g, 41%).
MS: 254.1 (M+1 for C₁₅H₂₇NO₂); mp 200-201 °C; Analysis (C₁₅H₂₇NO₂.HCl) (Cal.) C: 62.16, H: 9.74, N: 4.83; (found) C: 62.09, H: 9.72, N: 4.68. IR (KBr, cm⁻¹): 2926, 1720, 1189. ¹H NMR (CD₃OD) δ 0.48-0.57 (m, 1H), 0.85-0.92 (m, 8H), 1.60-1.73 (m, 5H), 1.78-1.93 (m, 2H), 2.22-2.38 (m, 4H), 2.42 (s, 2H), 3.11 (s, 2H), 3.72-3.78 (m, 1H).

### EXAMPLE 42: {(3R, 5S)-3,5-Dimethyl-1-[(2-methyl-butylamino)-methyl]-cyclohexyl}-acetic acid; hydrochloride salt

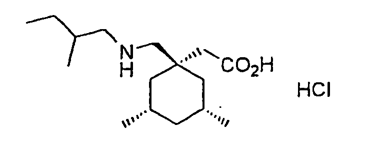

((3R, 5S)-1-Aminomethyl-3,5-dimethyl-cyclohexyl)-acetic acid; hydrochoride salt (1.0 g, 4.4 mmol) was dissolved in ethanol (47 mL) and treated with 1N NaOH (4.4 mL). Isobutyraldehyde (1 mL, 3 eq) was added followed by PtO₂ (0.17 g). Reaction was shaken under an atmosphere of H₂ (50 psi i.e. 344.737 Kilopascals) for 3 h. The reaction was then filtered, 1N HCl (10 mL) in ether was added, and the reaction was concentrated in vacuo. The residue was dissolved in hot isopropanol (100 mL) and filtered. Then it was concentrated in vacuo and recrystallized from acetone and hexane. The crystals were collected and washed with hexane to yield the desired product (0.89 g, 75%).
MS: 270.1 (M+1 for C₁₆H₃₁NO₂); mp 171-173 °C; Analysis (C₁₆H₃₁NO₂.HCl) (Cal.) C: 62.82, H: 10.54, N: 4:58; (found) C: 62.59, H: 10.43, N: 4.52. IR (KBr, cm⁻¹): 2964 (br), 1721, 1485, 1186, 840. ¹H NMR (CD₃OD) 5 0.48-0.57 (m, 1H), 0.81-1.07 (m, 12H), 1.23-1.27 (m, 1H), 1.45 -1.72 (m, 6H), 1.83-1.91 (m, 1H), 2.50 (s, 2H), 2.83 (dd, 1H, J = 8.3, 12.5 Hz), 3.00 (dd, 1H, J = 6.1, 12.5 Hz), 3.13-3.29 (m, 4H).

### EXAMPLE 43: {(3R, 5S)-1-[(2-Hydroxy-1-methyl-ethylamino)-methyl]-3,5-dimethyl-cyclohexyl}-acetic acid; hydrochloride salt

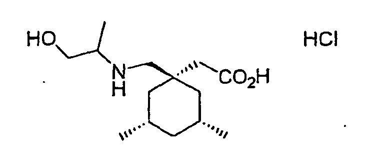

((3R, 5S)-1-Aminomethyl-3,5-dimethyl-cyclohexyl)-acetic acid; hydrochoride salt (1.0 g, 4.3 mmol) was dissolved in ethanol (45 mL) and treated with 1N NaOH (4.3 mL). 1-Hydroxy-propan-2-one (1 mL. 3 eq) was added followed by PtO₂ (0.15 g). Reaction was shaken under an atmosphere of H₂ (50 psi i.e. 344.737 Kilopascals) for 82 h. PtO₂ (0.1 g) and 1-Hydroxy-propan-2-one was added (1 mL), and the reaction was shaken under an H₂ atmosphere for another 24h. The reaction was then filtered, 1N HCl in ether (35 mL) was added, and the reaction was concentrated in vacuo. The residue was dissolved in hot isopropanol (150 mL) and filtered. Then it was concentrated in vacuo and recrystallized from hot isopropanol, acetone, and ether. The crystals were collected, washed with ether as well as acetone, and dried. (0.30 g, 1.04 mmol, 24%)
MS: 258.1 (M+1 for C₁₄H₂₇NO₃); mp 178-179 °C; Analysis (C₁₄H₂₇NO₃.HCl) (Cal.) C: 57.23, H: 9.60, N: 4.77; (found) C: 56.77, H: 9.39, N: 4.45. IR (KBr, cm⁻¹): 3349, 2897, 1704, 1419, 1194. ¹H NMR (CD₃OD) δ 0.48-0.57 (m, 1H), 0.82-0.90 (m, 8H), 1.25-1.32 (m, 4H), 1.60-1.75 (m, 5H), 2.47 (s, 2H), 3.23 (d, 2H, J= 5.1 Hz), 3.38 (m, 1H), 3.58 (dd, 1H, J = 12.0, 7.1 Hz), 3.79 (dd, 1H, J = 12.0, 4.2 Hz)

### EXAMPLE 44: {(3R, 5S)-1-[(2,2-Dimethoxy-ethylamino)-methyl]-3,5-dimethylcyclohexyl}-acetic acid; hydrochloride salt

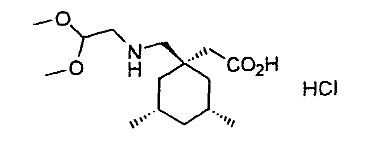

((3R, 5S)-1-Aminomethyl-3,5-dimethyl-cyclohexyl)-acetic acid; hydrochloride salt (1.0 g, 4.3 mmol) was dissolved in ethanol (47 mL). Glyoxal 1,1-dimethyl acetal (3.7 mL, 3.0 eq) was added and the reaction was treated with PtO₂ (0.17 g). The reaction was then shaken under an atmosphere of H₂ (50 psi i.e. 344.737 Kilopascals) for 3 h. The reaction was filtered, 1N HCl in ether (10 mL) was added, and the reaction was concentrated in vacuo. The residue was dissolved in hot isopropanol (100 mL) and filtered. Then, the filtrate was concentrated, and the residue was recrystallized from acetone and hexanes to give the desired product (0.92 g, 75 %).
MS: 288.1 (M+1 for C₁₅H₂₉NO₄); mp 125 °C (dec); Analysis (C₁₅H₂₉NO₄.HCl) (Cal.) C: 55.63, H: 9.34, N: 4.32; (found) C: 55.29, H: 9.48, N: 4.18. IR (KBr, cm⁻¹): 2949, 1715, 1457, 1191, 1134, 1077. ¹H NMR (CD₃OD) δ 0.47-0.56 (m, 1H), 0.81-0.90 (m, 8H), 1.59-1.71 (m, 5H), 2.46 (s, 2H), 3.18 (d, 2H, J = 5.1 Hz), 3.26-3.29 (m, 2H), 3.45-3.47 (m, 6H), 4.73 (t, 1H, J = 5.1 Hz).

### EXAMPLE 45: {(3R, 5S)-1-[(Cyclopentylmethyl-amino)-methyl]-3,5-dimethylcyclohexyl}-acetic acid; hydrochloride salt

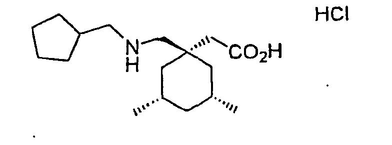

((3R, 5S)-1-Aminomethyl-3,5-dimethyl-cyclohexyl)-acetic acid; hydrochloride salt (1.0 g, 4.1 mmol) was dissolved in ethanol (45 mL) and treated with 1N NaOH (4.2 mL). Cyclopentanecarbaldehyde (1.5 mL, 3 eq) were added followed by PtO₂ (0.2 g). Reaction was shaken under an atmosphere of H₂ (50 psi i.e. 344.737 Kilopascals) for 12 h. The reaction was then filtered, 1N HCl in ether (35 mL) were added, and the reaction was concentrated in vacuo. The residue was dissolved in hot isopropanol (150 mL) and filtered. Then the solution was concentrated in vacuo and recrystallized from hot isopropanol, acetone, and ether. The crystals were collected and washed with ether and acetone. (0.40 g, 27%)
MS: 282.1 (M+1 for C₁₇H₃₁NO₂); mp 187-188 °C; Analysis (C₁₇H₃₁NO₂.HCl) (Cal.) C: 64.23, H: 10.15, N: 4.41; (found) C: 64.11, H: 10.04, N: 4.34. IR (KBr, cm⁻¹): 2952, 1720, 1452, 1185. ¹H NMR (CD₃OD) δ 0.48-0.57 (m, 1H), 0.81-0.90 (m, 8H), 1.25-1.32 (m, 2H) 1.60-1.71 (m, 9H), 1.89-1.93 (m, 2H), 2.25-2.29 (m, 113), 2.49 (s, 2H), 3.01 (d, 2H, J = 7.6 Hz), 3.20 (s, 2H).

### EXAMPLE 46: {(3R, 5S)-1-[(Cyclohexylmethyl-amino)-methyl]-3,5-dimethylcyclohexyl}-acetic acid; hydrochloride salt

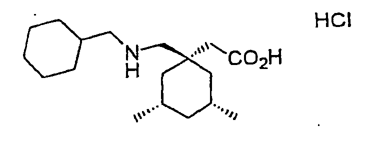

((3R, 5S)-1-Aminomethyl-3,5-dimethyl-cyclohexyl)-acetic acid; hydrochloride salt (1.0 g, 4.2 mmol) was dissolved in ethanol (45 mL) and treated with 1N NaOH (4.3 mL). Cyclohexanecarbaldehyde (1.5 mL, 3 eq) were added followed by PtO₂ (0.15 g). Reaction was shaken under an atmosphere of H₂ (50 psi i.e. 344.737 Kilopascals) for 17 h. The reaction was then filtered, 1N HCl in ether (35 mL) was added, and the reaction was concentrated in vacuo. The residue was dissolved in hot isopropanol (150 mL) and filtered. Then the solution was concentrated in vacuo and recrystallized from hot isopropanol, acetone, and ether. The crystals were collected and washed with ether and acetone. (0.46 g, 34%)
MS: 296.2 (M+1 for C₁₈H₃₃NO₂); mp 176-177 °C; Analysis (C₁₈H₃₃NO₂.HCl) (Cal.) C: 65.13, H: 10.32, N: 4.22; (found) C: 64.89, H: 10.36, N: 4.09. IR (KBr, cm⁻¹): 2919, 2851 1721, 1453, 1192. ¹H NMR (CD₃OD) δ 0.48-0.57 (m, 1H), 0.81-0.90 (m, 8H), 1.00-1.36 (m, 5H), 1.60-1.71 (m, 11H), 2.49 (s, 2H), 2.88 (d, 2H, J = 6.8Hz), 3.18 (s, 2H).

### EXAMPLE 47: ((3R, 5S)-1-Cyclohexylaminomethyl-3,5-dimethyl-cyclohexyl)-acetic acid; hydrochloride salt

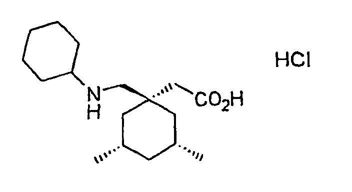

((3R, 5S)-1-Aminomethyl-3,5-dimethyl-cyclohexyl)-acetic acid; hydrochloride salt (0.99 g, 4.2 mmol) was dissolved in ethanol (45 mL) and treated with 1N NaOH (4.2 mL). Cyclohexanone (1.5 mL, 3 eq) was added followed by PtO₂ (0.2 g). Reaction was shaken under an atmosphere of H₂ (50 psi i.e. 344.737 Kilopascals) for 17 h. The reaction was then filtered, 1N HCl in ether (35 mL) was added, and the reaction was concentrated in vacuo. The residue was dissolved in hot isopropanol (150 mL) and filtered. Then the solution was concentrated in vacuo and recrystallized from hot isopropanol, acetone, and ether. The crystals were collected and washed with ether and acetone. (0.33 g, 25%)
MS: 282.1 (M+1 for C₁₇H₃₁NO₂); mp 210 °C; Analysis (C₁₇H₃₁NO₂.HCl) (Cal.) C: 64.23, H: 10.15, N: 4.41; (found) C: 63.89, H: 9.98, N: 4.33. IR (KBr, cm⁻¹):2921, 1691, 1.452, 1216. ¹H NMR (CD₃OD) δ 0.47-0.57 (m, 1H), 0.82-0.90 (m, 8H), 1.30-1.46 (m, 5H), 1.57-1.63 (m, 2H), 1.69 (d, 4H, J = 12.9 Hz), 1.86 (d, 2H, J = 12.5 Hz) 2.13 (d, 2H, J = 10.3 Hz), 2.46 (s, 2H), 3.04-3.10 (m 1H), 3.21 (s, 2H).

### EXAMPLE 48: ((3R,5S)-1-Carboxymethyl-3,5-dimethyl-cyclohexyl)-acetic acid

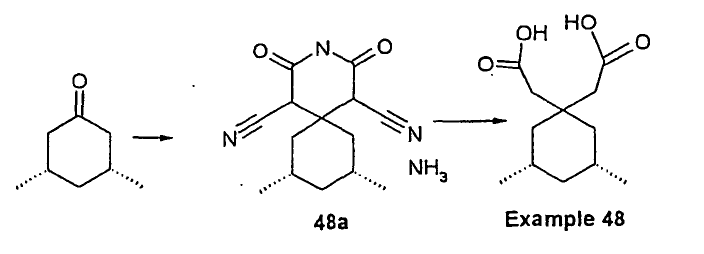

**Step i: Synthesis of (8R,10S)-8,10-Dimethyl-2,4-dioxo-3-aza-spiro[5.5]undecane-1,5-dicarbonitrile; ammonium salt (48a):** A solution of absolute ethanol (40 mL) was cooled to 0 °C and saturated with NH₃ gas, then treated with cis-3,5-dimethyl-cyclohexanone (5.0 g, 39.62 mmol) and ethyl cyanoacetate (8.43 mL, 79.24 mmol). The flask was stoppered, allowed to warm to room temperature, and stirred overnight. The precipitate was filtered, rinsed with Et₂O and dried under vacuum to give 6.04 g (59%) of the desired product as a pale solid.
MS: 260.1 (M+1 for C₁₄H₁₇N₃O₂); Analysis (C₁₄H₁₇N₃O₂.NH₃.0.88 H₂O) (Cal.) C: 57.55, H: 7.51, N: 19.17 (found) C: 57.67, H: 7.45, N: 19.06; IR (KBr, cm⁻¹) 3298, 2954, 2172, 1700, 1575. ¹H NMR (CDCl₃) δ 0.46-0.50 (m, 1 H), 0.82-1.17 (m, 11 H), 1.65-1.98 (m, 3 H), 3.58 (dd, 2 H, J = 13.9, 6.8 Hz).

**Step ii: Synthesis of Example 48:** (8S, 10R)-8,10-Dimethyl-2,4-dioxo-3-aza-spiro[5.5]undecane-1,5-dicarbonitrile (**48a**, 25.36 g, 97.8 mmol) was treated with concentrated H₂SO₄ (102 mL) and water (30 mL). The reaction was heated to 150 °C for 5 h; then allowed to cool to room temperature and stir overnight. The reaction was filtered and the solid was collected and dissolved in 1 N NaOH. This solution was filtered, then the filtrate was acidified with concentrated HCl (40 mL). The precipitate was collected and dried overnight under high vacuum. The compound was then dissolved in hot isopropanol (100 mL) and recrystallized from water yielding the desired product (13.21 g, 59%).
MS: 229.1 (M+1 for C₁₂H₂₀O₄); Analysis (C₁₂H₂₀O₄) (Cal.) C: 63.14, H: 8.83; (found) C: 63.36, H: 8.92. IR (KBr, cm⁻¹): 2950, 2913, 1698. ¹H NMR (CD₃OD) δ 0.40-0.48 (m, 1H), 0.77-0.85 (m, 8H) 1.57-1.77 (m, 5H) 2.41 (s, 2H), 2.56 (s, 2H).

### EXAMPLE 49: trans-((3R, 5S)-1-Hydroxymethyl-3,5-dimethyl-cyclohexyl-acetic acid; hydrochloride salt

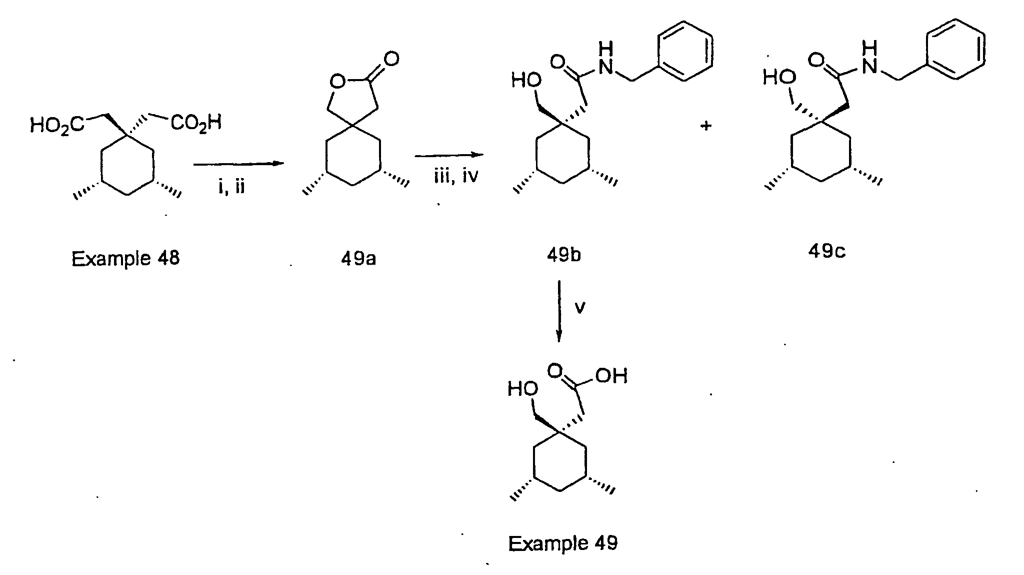

### Step i & ii: Synthesis of (cis/trans)-(7S,9R)-7,9-Dimethyl-2-oxa-spiro[4.5]decan-3-one (49a):

(i). The di-acid (**48**, 6.36 g, 27.9 mmol) was suspended in H₂O (93 mL), treated with ammonium hydroxide (3.5 mL) and silver nitrate (4.73 g, 27.9 mmol), and stirred for 20 minutes at room temperature. The reaction was filtered and the solid re-slurried three times with H₂O, then dried under vacuum in the dark for two days at 50 °C to give 6.41 g of the silver di-salt, which was carried on immediately (**ii**). The silver di-salt (1.24 g, 2.81 mmol) was placed in an amber flask, treated with CCl₄ (50 mL) and iodine (0.72 g, 2.81 mmol), and heated to 75 °C overnight. The reaction was filtered, concentrated in vacuo, and chromatographed on silica gel eluting with 4:1 hexanes/EtOAc to give 0.37 g (72%) of the desired product as a pale oil (**49a**).
MS: 183.0 (M+1 for C₁₁H₁₈O₂); Analysis (C₁₁H₁₈O₂.0.05H₂O) (Cal.) C: 72.13, H: 9.96 (found) C: 71.77, H: 9.69; IR (KBr, cm⁻¹) 3298, 2954, 2172, 1700, 1575, 1450, 1337. ¹H NMR (CDCl₃, ~1:1 mixture of cis/trans diastereomers) δ 0.49-0.59 (m, 1 H), 0.82-1.02 (m, 10 H), 1.42-1.53 (m, 2 H), 1.58-1.72 (m, 3 H), 2.30 (s, 1 H, for one isomer), 2.39 (s, 1 H, for the other isomer 2), 3.96 (s, 1 H, for one isomer), 4.10 (s, 1 H, for the other isomer).

**Step iii: Synthesis of (cis/trans)-*****N*****-Benzyl-2-((3R,5S)-1-hydroxymethyl-3,5-dimethylcyclohexyl)-acetamide (49b and 49c):** The lactone (49a, 0.85 g, 4.66 mmol) was dissolved in THF (18 mL), treated with benzylamine (0.61 mL, 5.60 mmol), and heated to reflux for two days. The reaction was cooled to room temperature, diluted with EtOAc, washed with saturated sodium bicarbonate solution and brine, dried over Na₂SO₄, and concentrated in vacuo. The residue was chromatographed on silica gel eluting with 1:1 hexane/EtOAc to give 0.79 g (59%) of the desired product as a clear oil (mixture of cis and trans isomers, **49b** and **49c**).
MS: 290.1 (M+1 for C₁₈H₂₇N₁O₂); an oil; TLC: SiO₂, R_{f} 0.16 (2:1 hexane/EtOAc); Analysis (C₁₈H₂₇N₁O₂) (Cal.) C: 74.70, H: 9.40, N: 4.84 (found) C: 74.41, H: 9.35, N: 4.57.

**Step iv: Separation of 49b and 49c:** The mixture of cis- and trans-isomers (**49b** and **49c**) was separated by preparative HPLC (C18 column eluting with a gradient of 60% CH₃CN / 40% H₂O for 5 minutes, then 45% CH₃CN / 55% H₂O for 35 minutes, all mobile phases with 0.05% TFA).

The trans-isomer (**49b**): RT = 23.600 min, 54 mg yield. ¹H NMR (CDCl₃) δ 0.36-0.62 (m, 3 H), 0.82 (d, 6 H, J = 6.3 Hz), 1.39-1.70 (m, 5 H), 2.14 (s, 2.H), 2.58 (br, 1 H), 3.60 (s, 2 H), 4.43 (d, 2 H, J = 5.9 Hz), 5.97 (br, 1 H), 7.26-7.37 (m, 5 H).

The cis-isomer (49c): RT = 25.828 min, 87 mg yield. ¹H NMR (CDCl₃) δ 0.47-0.56 (m, 1 H), 0.65 (t, 2 H, J = 13.2 Hz), 0.85 (d, 6 H, J = 6.3 Hz), 1.47-1.68 (m, 5 H), 2.38 (s, 2 H), 2.58 (br, 1 H), 3.40 (s, 2 H), 4.45 (d, 2 H, J = 5.9 Hz), 6.03 (br, 1 H), 7.28-7.36 (m, 5 H).

**Step v: The synthesis of Example 49:** The trans isomer of *N*-Benzyl-2-(1-hydroxymethyl-cyclohexyl)-acetamide (**49b,** 87 mg, 0.303 mmol) was taken from HPLC, dissolved in 5 mL of water, and treated with 24 mg of NaOH. The reaction was heated to reflux for 3 d. Then it was cooled to room temperature, diluted with 1 N NaOH, and extracted with EtOAc. The aqueous layer was acidified with conc. HCl (5 mL), and then diluted with 1 N HCl, and the product was extracted with EtOAc. The organic layer was washed with brine and dried over Na₂SO₄. The solution was concentrated to yield 23 mg (61%) of the desired product.
MS: 201 (M+1 for C₁₁H₂₀O₃); ¹H NMR (CD₃OD) δ 0.48-0.57 (m, 1 H), 0.88-1.00 (m, 8H), 1.40-1.49 (m, 2H), 1.68-1.70 (m, 3H), 2.28 (s, 2H), 4.11 (s, 2H).

### EXAMPLE 50: cis-((3R, 5S)-1-Hydroxymethyl-3,5-dimethyl-cyclohexyl-acetic acid; hydrochloride salt

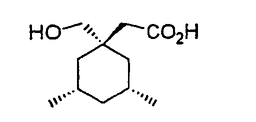

The cis isomer of *N*-Benzyl-2-(1-hydroxymethyl-cyclohexyl)-acetamide (**49c**, 87 mg, 0.303 mmol) was taken from HPLC, dissolved in 5 mL of water, and treated with 24 mg of NaOH. The reaction was heated to reflux for 3 d, then cooled to room temperature, diluted with 1 N NaOH, and extracted with EtOAc. The aqueous layer was acidified with concentrated HCl (5 mL), and then diluted with 1 N HCl, and the product was extracted with EtOAc. The organic layer was washed with brine and dried over Na₂SO₄. The solution was concentrated to yield 43 mg (71%) of the desired product.
MS: 201.1 (M+1 for C₁₁H₂₀O₃); ¹H NMR (CD₃OD) δ 0.48-0.57 (m, 1H), 0.88-0.96 (m, 8H), 1.42-1.50 (m, 2H), 1.59-1.69 (m, 3H), 2.37 (s, 2H), 3.94 (s, 2H).

### EXAMPLE 51: (1-Dimethylaminomethyl-cyclohexyl)-acetic acid; hydrochloride salt

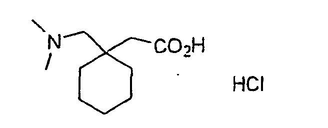

(1-Aminomethyl-cyclohexyl)-acetic acid (0.51 g, 3.0 mmol) was dissolved in ethanol, and formaldehyde (3 mL, 2 eq) was added. 10% Pd/C (1.6 g) was added and the reaction was shaken at room temperature under a hydrogen atmosphere of 760 Torr i.e. 101.325 Kilopascals for 12 h. Then the reaction was treated with HCl until the pH was about 2, and it was recrystallized from acetone and ether to give 1.62 g (87%) of the desired product.
MS: 200.1 (M+1 for C₁₁H₂₁NO₂); mp: 140-142°C; Analysis (C₁₁H₂₁NO₂.HCl) (Cal.) C: 54.99, H: 9.44 N: 5.83; (found) C: 54.90, H: 9.36, N: 6.22. ¹H NMR (CD₃OD) δ 1.42-1.59 (m, 10H), 2.63 (s, 2H), 2.96 (s, 6H), 3.26-3.29 (m, 2H).

### EXAMPLE 52: (1-Butylaminomethyl-cyclohexyl)-acetic acid; hydrochloride salt

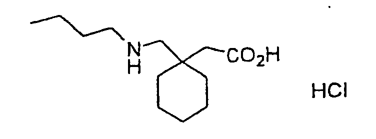

(1-Aminomethyl-cyclohexyl)-acetic acid (0.86 g, 5.0 mmol) was dissolved in ethanol (100 mL), then Pd/C (10%, 0.86 g) and butyraldehyde (1.44 g, 3 eq) were added. The reaction was placed under 760 Torr i.e. 101.325 Kilopascals of hydrogen at room temperature until the reaction was complete. It was then filtered, acidified, and concentrated. The residue was dissolved in isopropanol, filtered, and recrystallized from acetone and ether to yield the final product.
MS: 228.1 (M+1 for C₁₃H₂₅NO₂); mp: 143-154°C; Analysis (C₁₃H₂₅NO₂.HCl) (Cal.) C: 59.19, H: 9.93, N: 5.31; (found) C: 58.85, H: 9.86, N: 5.05. ¹H NMR (CD₃OD) δ 0.98 (t, 3H, J = 7.3 Hz), 1.39-1.54 (m, 12H), 1.66-1.72 (m, 2H), 2.57 (s, 2H), 3.01 (t, 2H, J = 9.3 Hz), 3.09 (s, 2H).

### EXAMPLE 53: {1-[2,2-Dimethoxy-ethylamino)-methyl]-cyclohexyl}-acetic acid; hydrochloride salt

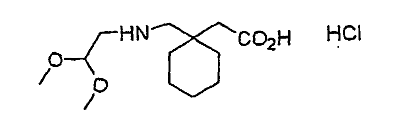

(1-Aminomethyl-cyclohexyl)-acetic acid (2.0 g, 11.7 mmol), was dissolved in ethanol (100 mL). Glyoxal 1,1-dimethyl acetal (3.7 mL, 3 eq) were added and the reaction was treated with PtO₂ (0.5 g). The reaction was then shaken under au atmosphere of H₂ (50 psi i.e. 344.737 Kilopascals) for 4 h. The reaction was filtered, 1N HCl in ether (15 mL) was added, and the reaction was concentrated in vacuo. The residue was recrystallized from hot isopropanol (25 mL) and acetone (700 mL) to give 0.44 g (52%) of the desired product.
MS: 260.1 (M+1 for C₁₃H₂₅NO₄); mp 128-129 °C; Analysis (C₁₃H₂₅NO₂.HCl) (Cal.) C: 52.79, H: 8.86, N: 4.74; (found) C: 52.84, H: 8.94, N: 4.67. IR (KBr, cm⁻¹): 2926, 1693,1457, 1208, 1087. ¹H NMR (CD₃OD) δ 1.40-1.55 (m, 10H), 2.60 (s, 2H), 3.13 (s, 2H), 3.17 (d, 2H, J = 4.9 Hz), 3.45 (s, 6H), 4.72 (t, 1H, J = 5.1 Hz)

### EXAMPLE 54: (1-methylaminomethyl-cyclohexyl)-acetic acid; hydrochloride salt

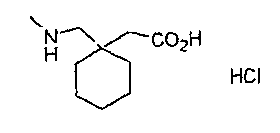

{1-[(Benzyl-methyl-amino)-methyl]-cyclohexyl}-acetic acid; hydrochloride salt (Example 55, 4.1 g, 13.2 mmol) was dissolved in 95% ethanol (250 mL). 10% Pd/C (1 g) was added and the reaction was shaken at room temperature under a hydrogen atmosphere of 760 Torr i.e. 101.325 Kilopascals for 1 h. The reaction was filtered and concentrated, then recrystallized from acetone and ether to yield 2.69 g (92%) of desired product.
MS: 186.0 (M+1 for C₁₀H₁₉NO₂); mp: 158-162°C; Analysis (C₁₀H₁₉NO₂.HCl) (Cal.) C: 53.09, H: 9.13, N: 6.19; (found) C: 53.14, H: 8.96, N: 6.34; ¹H NMR (CD₃OD) δ 1.42-1.54 (m, 10H), 2.50-2.53 (m, 2H), 2.71-2.74 (m, 3H), 3.09-3.11 (m, 2H).

### EXAMPLE 55: {1-[(Benzyl-methyl-amino)-methyl]-cycloheayl}-acetic acid; hydrochloride salt

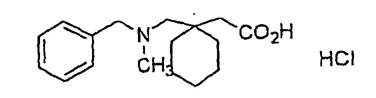

[1-(Benzylamino-methyl)-cyclohexyl]-acetic acid; hydrochloride salt (0.49 g, 1.7 mmol) was dissolved in ethanol (45 mL) and treated with 1N NaOH (1.8 mL). Formaldehyde (1 mL, 5 eq) was added and the reaction was treated with PtO₂ (0.2 g). The reaction was then shaken under an atmosphere of H₂ (50 psi i.e. 344.737 Kilopascals) for 30 h. The reaction was filtered, 1N HCl in ether (10 mL) was added, and the reaction was concentrated in vacuo. To give 0.31 g (1.14 mmol, 67%) of the desired product.
MS: 274.1 (M-1 for C₁₇H₂₅NO₂); an oil; IR (KBr, cm⁻¹): 3205, 2930, 2261, 1684, 1451, 1198. HRMS: (calc) 276.1963 (found) 276.1973 (M+1 for C₁₇H₂₅NO₂); ¹H NMR (CD₃OD) δ 1.33-1.63 (m, 13H), 2.28 (s, 2H), 3.06 (s, 2H), 4.40 (s, 2H), 7.21-7.34 (m, 5H).

### EXAMPLE 56: [1-(Phenethylamino-methyl)-cyclohexyl]-acetic acid; hydrochloride salt

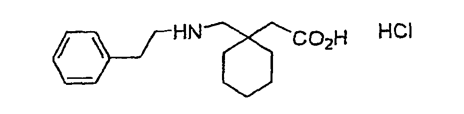

(1-Aminomethyl-cyclohexyl)-acetic acid (2.0 g, 11.7 mmol) was dissolved in ethanol (100 mL). Phenyl-acetaldehyde (4 mL, 3 eq) was added and the reaction was treated with 10% Pd/C (0.5 g). The reaction was then shaken under an atmosphere of H₂ (50 psi i.e. 344.737 Kilopascals) for 3 h. The reaction was filtered, 1N HCl in ether (20 mL) was added, and the reaction was concentrated in vacuo. The residue was recrystallized from hot isopropanol and ether to give 0.15 g (4.1 %) of the desired product.
MS: 276.1 (M+1 for C₁₇H₂₅NO₂); mp 182-183 °C; Analysis (C₁₇H₂₅NO₂.HCl) (Cal.) C: 64.54, H: 8.46, N: 4.43; (found) C: 64.54, H: 8.35, N: 4.34. IR (KBr, cm⁻¹):2929, 2855, 2813, 1693, 1455, 1220. ¹H NMR (CD₃OD) δ 1.38-1.58 (m, 10H), 2.56 (s, 2H), 3.01-3.06 (m, 2H), 3.14 (s, 2H), 3.23-3.29 (m, 2H), 7.21-7.34 (m, 5H)

### EXAMPLE 57: {1-[(3-Phenyl-propylamino)-methyl]-cyclohexyl}-acetic acid; hydrochloride salt

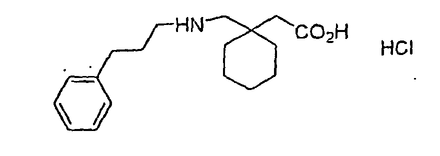

(1-Aminomethyl-cyclohexyl)-acetic acid (2.00 g, 11.7 mmol) was dissolved in ethanol (100 mL). 3-Phenyl-propionaldehyde (4.6 mL, 3 eq) were added and the reaction was treated with 10% Pd/C (0.5 g). The reaction was then shaken under an atmosphere of H₂ (50 psi i.e. 344.737 Kilopascals) for 3 h. The reaction was filtered, 1N HCl (20 mL) in ether was added, and the reaction was concentrated in vacuo. The residue was recrystallized from hot isopropanol and ether to give 0.46 g (12%) of the desired product.
MS: 290.1 (M+1 for C₁₈H₂₇NO₂); mp 168-169 °C; Analysis (C₁₈H₂₇NO₂.HCl) (Cal.) C: 66.34, H: 8.66, N: 4.40; (found) C: 66.48, H: 8.65, N: 4.17. IR (KBr, cm⁻¹): 2932, 2854, 1686, 1454, 1217. ¹H NMR (CD₃OD) δ 1.38-1.58 (m, 10H), 2.00-2.04 (m, 2H), 2.56 (s, 2H), 2.71 (t, 2H, J = 7.6 Hz) 3.01-3.08 (m, 4H), 7.21-7.34 (m, 5H).

### EXAMPLE 58: ((3R, 5S)-1-Hydroxymethyl-3,5-dimethyl-cyclohexyl)-acetic acid; sodium salt

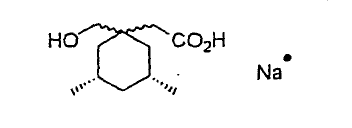

(7S, 9R)-7,9-Dimethyl-2-oxa-spiro[4.5]decan-3-one (**49a**, 1.04 g, 5.71 mmol) was dissolved in water (25 mL) and treated with NaOH (2.5 g). The reaction was heated to reflux (115°C) for 4h. The solution was then cooled to room temperature, washed with Et₂O, and the aqueous layer was concentrated in vacuo to give a solid (8 g). The residue was washed with 1N acetic acid, and it was extracted with EtOAc, which was then concentrated. The residue was then recrystallized from EtOH, MeOH, and hexanes to yield 0.75 g (3.75 mmol, 66%).
MS: 199.0 (M-1 for C₁₁H₂₀O₃); HRMS: (calc) 199.1334 (found) 199.1333 (M-1 for C₁₁H₂₀O₃); mp >250 °C; IR (KBr, cm⁻¹): 2953, 2910, 1777, 1706, 1458, 1168, 1012.

### EXAMPLE 59: ((3R, 5S)-1-Ethylaminomethyl-3,5-dimethyl-cyclohexyl)-acetic acid; hydrochloride salt

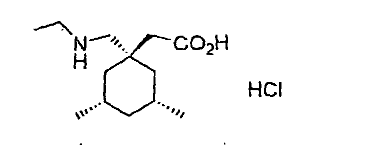

((3R, 5S)-1-Aminomethyl-3,5-dimethyl-cyclohexyl)-acetic acid; hydrochloride salt (59a, 0.418 g, 1.8 mmol, Compound 59a was prepared according to the literature procedure: Bryans, J. Bioorganic & Medicinal Chemistry Letters, 1997, 7(19), 2481.) was dissolved in ethanol (45 mL) and treated with 1N NaOH (1.8 mL). Acetaldehyde (0.5 mL, 3 eq) was added and the reaction was treated with PtO₂ (0.2 g). The reaction was then shaken under an atmosphere of H₂ (50 psi i.e. 344.737 Kilopascals) for 3 b. The reaction was filtered, 1N HCl (2 mL) in ether was added, and the reaction was concentrated in vacuo. The residue was dissolved in hot isopropanol (100 mL) and filtered. Hexanes (100 mL) was added to the filtrate at 0 °C, and the precipitate was collected to give 31 mg (0.14 mmol, 8%) of the desired product
MS: 228.1 (M+1 for C₁₃H₂₅NO₂); mp 167-168 °C; IR (KBr, cm⁻¹): 2954 (br), 2502, 1685, 1459, 1223. Analysis (C₁₃H₂₅NO₂.HCl) (Cal.) C: 56.60, H: 9.98, N: 5.11; (found) C: 56.60, H: 9.85, N: 4.77. ¹H NMR (CD₃OD) δ 0.47-0.56 (m, 1H), 0.78-0.89 (m, 8H), 1.30-1.36 (m, 3H), 1.61-1.79 (m, 5H), 2.58 (s, 2H), 2.99-3.10 (m, 4H).

### EXAMPLE 60: (1-Aminomethyl-4-ethyl-cyclohexyl)-acetic acid; hydrochloric salt

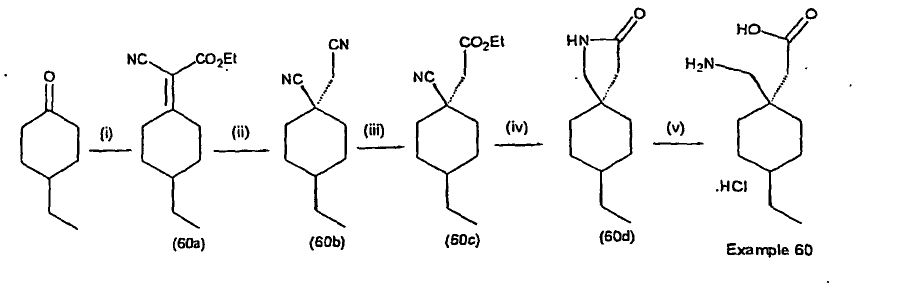

**Step i: Synthesis of 2-Cyano-(4-ethyl-cyclohexylidene)-acetic acid ethyl ester: (60a):** 4-Ethylcyclohexanone (13.0 g, 103.0 mmol) was dissolved in toluene (150 mL), and treated with ammonium acetate (0.79 g, 10.3 mmol), glacial acetic acid (1.2 mL), and ethyl cyanoacetate (11.65 g, 103.0 mmol). The reaction was heated to reflux for 56 h with a Dean-Stark trap; then it was cooled room temperature and washed with H₂O (2x100mL). The aqueous layer was backwashed with toluene (2x150mL). The organic layers were combined and dried over Na₂SO₄ and concentrated in vacuo. The residue was chromatographed on silica gel eluting with 5:1 hexanes/ethyl acetate to give 19.81 g (87%) of the desired product as a clear yellow oil (**60a**).
MS: 222.1 (M+1 for C₁₃H₁₉N₁O₂); an oil; TLC: SiO₂, R_{f} 0.93 (1:1 hexane/ EtOAc); IR (KBr, cm⁻¹): 2931, 2225, 1728, 1602, 1233. ¹H NMR (CDCl₃) δ 0.82 (t, 3H, J = 7.4 Hz), 1.11-1.38 (m, 7H), 1.39-1.45 (m, 1H), 1.90-2.09 (m, 3H), 2.20-2.28 (m, 1H), 2.91-2.98 (m, 1H), 3.75-3.79 (m, 1 H)) 4.15-4.20 (m, 2H).

**Step ii: Synthesis of** ***cis*****-1-Cyanomethyl-4-ethyl-cyclohexanecarbonitrile: (60b):** 2-Cyano-(4-ethyl-cyclohexylidene)-acetic acid ethyl ester (60a, 16.57 g, 74.86 mmol) was dissolved in EtOH (100 mL) and added to a suspension of NaCN (3.67 g, 74.86 mmol) in EtOH / H₂O (300 mL / 16 mL). The reaction was heated to reflux overnight, then cooled to room temperature and filtered to remove salt that had precipitated (4.58 g). The filtrate was concentrated in vacuo to yield a clear, highly viscous, yellow oil. The residue was flash chromatographed on silica gel eluting with 16:1 hexanes/EtOAc to give 11.82 g (77%) (a mixture of 81% of cis- and 19% of trans-isomers) of product as a yellow oil (**60c**).
MS: no M+1 ion observed (C₁₁H₁₆N₂); an oil; TLC: SiO₂, R_{f} 0.55 (2:1 hetane/EtOAc); Analysis (C₁₁H₁₆N₂) (Cal.) C: 74.96, H: 9.15, N: 15.89 (found) C: 74.84, H: 8.97, N: 15.74. IR (KBr, cm⁻¹): 2934, 2240,1453. ¹H NMR (CD₃OD) δ 0.90 (t, 3H, J = 7.6 Hz), 1.18-1.47 (m, 7H) 1.83-1.87 (m, 2H) 2.07-2.10 (m, 2H), 2.85 (s, 2H).

**Step iii. Synthesis of** ***cis*****-(1-Cyano-4-ethyl-cyclohexyl)-acetic acid ethyl ester ((60c):** *cis-*1-Cyanomethyl-4-ethyl-cyclohexanecarbonitrile (60b, 6.16 g, 34.95 mmol) was dissolved in EtOH (92 mL) and toluene (92 mL), cooled to 0 °C, and saturated with HCl gas. The solution was stoppered and allowed to stir overnight at room temperature. It was then concentrated in vacuo to give a yellowish white solid. The residue was triturated with ethyl ether (200 mL) and dried under vacuum, then treated with H₂O (90 mL) and 1 N HCl (4 mL) was added. The reaction was allowed to stir overnight at room temperature and yellow oil droplets were observed in solution after 18 h. The compound was extracted with EtOAc (3 x 100 mL); the organic layers were combined, washed with brine, dried over Na₂SO₄, and concentrated. The residue was flash chromatographed with 16:1 hexanes/EtOAc to give 4.40 g (56%) of the desired product (60c).
MS: 224.1 (M+1 for C₁₃H₂₁NO₂); oil; TLC: SiO₂, R_{f} 0.38 (CH₂Cl₂); Analysis (C₁₃H₂₁NO₂) (Cal.) C: 69.92, H: 9.48, N: 6.27 (found) C: 69.97, H: 9.49, N: 6.17. IR (KBr, cm⁻¹): 2930, 2236, 1736, 1187. ¹H NMR (CD₃OD) δ 0.87-0.90 (m, 3H), 1.18-1.46, (m, 10H), 1.77-1.80 (m, 2H), 2.05-2.09 (m, 2H), 2.59 (s, 2H), 4.13-4.15 (m, 2H).

**Step iv: Synthesis of 8-Ethyl-2-aza-spiro[4,5]decan-3-one: (60d):** *cis*-(1-Cyano-4-ethyl-cyclohexyl)-acetic acid ethyl ester (60c.2.7 g, 12.1 mmol) was dissolved in MeOH (250 mL) and treated with 10% Rh/2% Pd/C (0.5 g) and shaken under an atmosphere.of H₂ (50 psi i.e. 344.737 Kilopascals) for 3 h. The reaction was then filtered, concentrated in vacuo, and
MS: 181.0 (M+1 for C₁₁H₁₉N₁O₁); mp 131-1 33 °C; TLC: SiO₂, R_{f} 0.22 (4% MeOH/CH₂Cl₂); Analysis (C₁₁H₁₈N₁O₁) (Cal.) C: 72.28, H: 10.08, N: 7.66 (found) C: 72.66, H: 10.45, N: 7.26. IR (KBr, cm⁻¹): 3197, 2918, 1680. ¹H NMR (CDCl₃) δ 0.83-0.97 (m, 5 H), 1.06-1.23 (m, 3 H), 1.31-1.39 (m, 2H), 1.63-1.67 (m, 4 H), 2.09-2.11 (m, 2 H), 3.15-3.17 (m, 2 H), 5.73 (br, 1 H).

**Step v: Synthesis of Example 60:** 8-Ethyl-2-aza-spiro[4.5]decan-3-one (60d, 3.8 g, 21.1 mmol) was treated with concentrated HCl (141 mL) and heated to 110 °C for 24 h. The reaction was then concentrated, and recrystallized from hot isopropanol (20 mL) and acetone to yield 3.3 g (16.6 mmol, 79%) of the product.
MS: 200.1 (M+1 for C₁₁H₂₁NO₂); Analysis (C₁₁H₂₁NO₂.HCl) (Cal.) C: 56.04, H: 9.41, N: 5.94; (found) C: 56.06, H: 9.46, N: 5.88. IR (KBr, cm⁻¹): 2916 (br), 1698, 1526, 1281. ¹H NMR (CD₃OD) δ 0.89 (t, 3H, J = 7.3 Hz), 1.02-1.40 (m, 8H), 1.68 (t, 4H, J = 16.1 Hz), 2.38 (s, 2H), 3.13 (s, 2H).

### EXAMPLE 61: (1-Aminomethyl-4-propyl-cyclohexyl)-acetic acid; hydrochloric salt

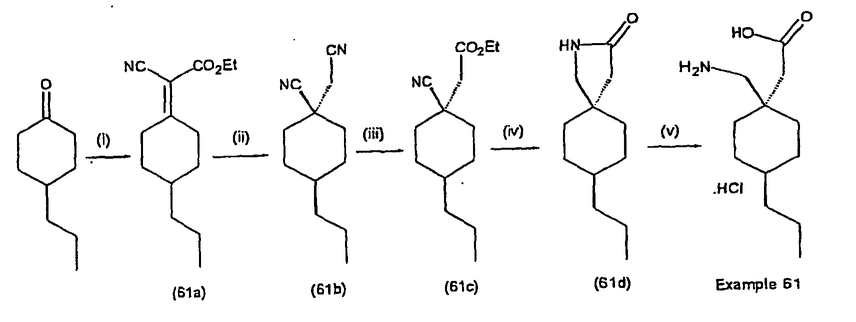

**Step i. Synthesis of 2-Cyano-(4-propyl-cyclohexylidene)-acetic acid ethyl ester:** (**61a**): 4-*n*-Propyl-cyclohexanone (14.0 g, 99.8 mmol) was dissolved in toluene (145 mL), and treated with ammonium acetate (0.77 g, 9.98 mmol), glacial acetic acid (1.1 mL), and ethyl cyanoacetate (11.3 g, 99.8 mmol). The reaction was heated to reflux overnight with a Dean-Stark trap; then it was cooled room temperature and washed with H₂O (2 x 200 mL). The aqueous layer was backwashed with toluene (3 x 200 mL). The organic layers were combined and dried over Na₂SO₄, and concentrated in vacuo. The residue was chromatographed on silica gel eluting with 8:1 hexane/ethyl acetate to give 21.58 g (92%) of the desired product as a clear yellow oil (**61a**).
MS: 236.1 (M+1 for C₁₄H₂₁NO₂); an oil; TLC: SiO₂, R_{f} 0.57 (8:1 hexane/ EtOAc); Analysis (C₁₄H₂₁NO₂) (Cal.) C: 71.46, H: 8.99, N: 5.95 (found) C: 71.38, H: 8.92, N: 6.10 IR (KBr, cm⁻¹): 2929, 2225, 1728, 1603, 1227. ¹H NMR (CDCl₃) δ 0.83 (t, 3H, J = 7.3 Hz), 1.07-1.30 (m, 9H), 1.51-1.56 (m, 1H), 1.91-2.00 (m, 2H), 2.04-2.12 (m, 1H), 2.23-2.30 (m, 1H), 2.95-3.01 (m, 1H), 3.77-3.83 (m, 1H), 4.20 (q, 2H, J = 7.1 Hz).

**Step ii: Synthesis of** ***cis*****-1-Cyanomethyl-4-propyl-cyclohexanecarbonitrile: (61b):** 2-Cyano-(4-*n*-propyl-cyclohexylidene)-acetic acid ethyl ester (61a, 19.46 g, 82.70 mmol) was dissolved in EtOH (100 mL) and added to a suspension of NaCN (4.053 g, 82.70 mmol) in EtOH / H₂O (340 mL / 20 mL). The reaction was heated to reflux overnight, then cooled to room temperature and filtered to remove salt that had precipitated. The filtrate was concentrated in vacuo to yield a clear, highly viscous, yellow oil. This oil was redissolved in EtOAc and washed with brine (2 x 150 mL), dried over Na₂SO₄, and concentrated. The residue was flash chromatographed on silica gel eluting with 19:1 hexane/EtOAc to give 4.69 g of 61b. (74% yield).
MS: no M+1 ion observed (C₁₂H₁₈N₂); an oil; TLC: SiO₂, R_{f} 0.38 (3:1 hexane/EtOAc); Analysis (C₁₂H₁₈N₂) (Cal.) C: 75.74, H: 9.53, N: 14.72 (found) C: 76.05, H: 9.58, N: 14.90. IR (KBr, cm⁻¹): 2931, 2239, 1453, 758. ¹H NMR (CDCl₃) 5 0.56 (t, 3H, J = 7.1 Hz), 1.18-1.34 (m, 7H), 1.42-1.61 (m, 2H), 1.81-1.83 (m, 2H), 2.06-2.09 (m, 2H), 2.65 (s, 2H).

**Step iii. Synthesis of** ***cis*****-(1-Cyano-4-propyl-cyclohexyl)-acetic acid ethyl (61c):** *cis*-1-Cyanomethyl-4-*n*-propyl-cyclohexanecarbonitrile (61b, 3.87 g, 20.34 mmol) was dissolved in EtOH (50 mL) and toluene (50 mL), cooled to 0 °C, and saturated with HCl gas over 20 minutes. The solution was stoppered and allowed to stir overnight at room temperature. It was then concentrated in vacuo to give a yellowish white solid. The residue was triturated with ethyl ether (200 mL) and dried under vacuum, then dissolved in H₂O (80 mL). 1 N HCl (4 mL) was added. The reaction became clear after 4 hours of stirring and it was allowed to continue to stir overnight at room temperature. Yellow oil droplets were observed in solution after 18 h. The compound was extracted with EtOAc (4 x 200 mL); the organic layers were combined, washed with brine (2 x 200 mL), dried over Na₂SO₄, and concentrated. The residue was flash chromatographed with 19:1 hexanes/EtOAc and 3.27 g of product (61c, 68%) was obtained.
MS: 238.1 (M+1 for C₁₃H₂₁NO₂); oil; TLC: SiO₂, R_{f} 0.38 (CH₂Cl₂); Analysis (C₁₃H₂₁NO₂) (Cal.) C: 70.85, H: 9.77, N: 5.90 (found) C: 70.65, H: 9.72, N: 5.84. IR (KBr, cm⁻¹): 2929, 2236, 1736, 1185. ¹H NMR (CDCl₃) δ 0.85 (t, 3H, J = 7.1 Hz), 1.19-1.55 (m, 10H), 1.71-1.74 (m, 2H), 2.08-2.10 (m, 2H), 2.59 (s, 2H), 4.16 (q, 2H, J = 7.1 Hz).

**Step iv. Synthesis of** ***cis*****-8-propyl-2-aza-spiro[4.5]decan-3-one (61d):** *cis*-(1-Cyano-4-propyl-cyclohexyl)-acetic acid ethyl ester (61c, 8.27 g, 34.8 mmol) was dissolved in methanol (90 mL), treated with triethylamine and 10% Rh / 2% Pd/C, and shaken under an atmosphere of H₂ (50 psi i.e. 344.737 Kilopascals) for 55 h. The reaction was filtered and concentrated in vacuo. The residue was chromatographed on silica gel eluting with 5:1 hexanes/EtOAc to give 6.41 g (61d, 94%) of the desired product.
MS: 196.1 (M+1 for C₁₂H₂₁NO); an oil; TLC: SiO₂, R_{f} 0.38 (hexane/EtOAc); Analysis (C₁₂H₂₁NO) (Cal.) C: 73.80, H: 10.84, N: 7.17; (found) C: 73.86, H: 10.76, N: 7.11. IR (KBr, cm⁻¹): 3192, 2924, 1704, 1678. ¹H NMR (CDCl₃) δ 0.83-1.17 (m, 5H), 1.18-1.39 (m, 7H), 1.61-1.65 (m, 2H), 1.71-1.74 (m, 2H) 2.11 (s, 2H), 3.16 (s, 2H), 5.85 (br, 1H).

**Step v: Synthesis of Example 61: (1-Aminomethyl-4-propyl-cyclohexyl)-acetic acid; hydrochloric salt:** 8-Propyl-2-aza-spiro[4.5]decan-3-one (**61d**, 5.8 g, 29.75 mmol) was treated with concentrated HCl (150 mL) and heated to 115°C overnight. The reaction was then allowed to cool to room temperature and precipitate was observed. This was filtered to obtain 3.47 g of a white solid, and the filtrate was cooled and re-filtered to give a total of 6.24 g (99%) of the desired product.
MS: 214.1 (M+1 for C₁₂H₂₃NO₂); mp 142-144°C; Analysis (C₁₂H₂₃NO₂.HCl.0.86H₂O) (Cal.) C: 58.91, H: 9.83, N: 5.72; (found) C: 59.27, H: 9.60, N: 5.71. ¹H NMR (CD₃OD) δ 0.86-0.90 (m, 3H), 0.97-1.41 (m, 10H), 1.61-1.75 (m, 4H), 2.37 (s, 2H), 3.13 (s, 2H) IR (KBr, cm⁻¹) 2919, 1702, 1525, 1461, 1198.

### EXAMPLE 62: ((3R, 5S)-3,5-Dimethyl-1-propylaminomethyl-cyclohexyl)-acetic acid; hydrochloride salt

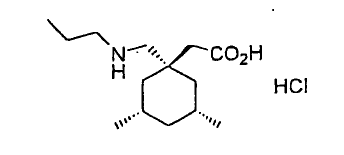

((3R, 5S)-1-Aminomethyl-3,5-dimethyl-cyclohexyl)-acetic acid; hydrochloride salt (59a, 0.50 g, 2.12 mmol) was dissolved in ethanol (45 mL) and treated with 1.8 mL of 1N NaOH. Propionaldehyde (0.5 mL, 3 eq) was added and the reaction was treated with PtO₂ (0.2 g). The reaction was then shaken under an atmosphere of H₂ (50 psi i.e. 344.737 Kilopascals) for 3 h.
The reaction was filtered, 1N HCl in ether (5 mL) was added, and the reaction was concentrated in vacuo. The residue was dissolved in hot isopropanol (100 mL) and filtered. The filtrate was concentrated and recrystallized from hot isopropanol (4 mL), Et₂O (225 mL), and hexanes (150 mL) to give 200 mg (0.83 mmol, 39%) of product.
MS: 242.1 (M+1 for C₁₄H₂₇NO₂); IR (KBr, cm⁻¹): 2952 (br), 1686, 1457, 1218. Analysis (C₁₄H₂₇NO₂·HCl·0.24H₂O) (Cal.) C: 59.60, H: 10.17, N: 4.96; (found) C: 59.68, H: 10.09, N: 4.64. ¹H NMR (CD₃OD) δ 0.48-0.57 (m, 1H), 0.78-1.05 (m, 11H), 1.61-1.78 (m, 7H), 2.60 (s, 2H), 2.93-3.00 (m, 4H).

### EXAMPLE 63: [(1R, 3R)-1-(Benzylamino-methyl)-3-methyl-cyclohexyl]-acetic acid; hydrochloride salt

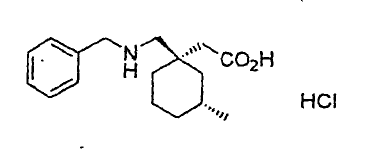

((1R, 3R)-1-Aminomethyl-3-methyl-cyclohexyl)-acetic acid; hydrochloride salt (Example 1, 3.0 g, 13.5 mmol) was dissolved in ethanol (80 mL) and treated with 1 N NaOH (14 mL, 1+ equivalents). Benzaldehyde (4.5 mL) was added followed by 10% Pd/C (1.0 g), and the reaction was shaken under an atmosphere of H₂ (50 psi i.e. 344.737 Kilopascals) for 2.75 h. The reaction was filtered, 1 M HCl in ether (20 mL) was added, and the solution was concentrated in vacuo. The residue was then dissolved in hot isopropanol (100 mL), filtered, and concentrated in vacuo. The residue was then dissolved in the minimum amount of hot isopropanol necessary and recrystallized from ether. The solid was filtered and the filtrate was cooled overnight to yield a second crop of crystals. The solids were combined to give 1.35 g (36%) of the desired product.
MS: 276.1 (M+1 for C₁₇H₂₅NO₂); HRMS: (calc) 276.1963 (found) 276.1962 (M+1 for C₁₇H₂₅NO₂); mp: 157-158 °C; IR (KBr, cm⁻¹): 2932 (br), 1691, 1450, 1201; Analysis (C₁₇H₂₅NO₂·HCl·0.24H₂O) (Cal.) C: 64.58, H: 8.44, N: 4.43; (found) C: 64.57, H: 8.44, N: 4.28. ¹H NMR (CD₃OD) δ 0.77-1.69 (m, 12H), 2.42 (s, 2H), 3.21 (s, 2H), 4.25 (s, 2H), 7.43-7.52 (m, 5H).

### EXAMPLE 64: {(1R, 3R)-1-[(Benzyl-methyl-amino)-methyl]-3-methyl-cyclohexyl}-acetic acid; hydrochloride salt

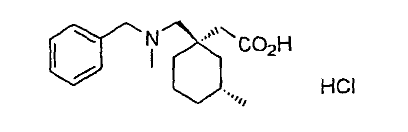

[(1R, 3R)-1-(Benzylamino-methyl)-3-methyl-cyclohexyl]-acetic acid; hydrochloride salt (Example 63, 1.02 g, 3.27 mmol) was dissolved in ethanol (40 mL) and treated with 1 N NaOH (3.3 mL, 1+ eq). Formaldehyde (2.5 mL) was added followed by PtO₂ (0.1 g), and the reaction was shaken under an atmosphere of H₂ (50 psi i.e. 344.737 Kilopascals) for 8 h. The reaction was filtered, 1 M HCl in ether (10 mL) was added, and the solution was concentrated in vacuo. The residue was then dissolved in hot isopropanol (100 mL), filtered, and concentrated in vacuo. The residue was then dissolved in the minimum amount of hot isopropanol necessary and recrystallized from ether and hexane. The solid was collected to give 0.704 g (2.16 mmol, 66%) of the desired product.
MS: 290.1 (C₁₈H₂₇NO₂); HRMS: 290.2128 (C₁₈H₂₇NO₂); mp: 204-205 °C; IR (KBr, cm⁻¹): 2926 (br), 1720, 1457, 1387, 1203; ¹H NMR (CD₃OD) δ 0.82-0.98 (m, 5H), 1.24-1.91 (m, 7H), 2.38-2.58 (m, 2H), 2.91 (d, 3H, J = 11.2 Hz), 3.43-3.54 (m, 2H), 4.33-4.49 (m, 2H), 7.50-7.56 (m, 5H).

### EXAMPLE 65: ((1R, 3R)-3-Methyl-1-methylaminomethyl-cyclohexyl)-acetic acid; hydrochloride salt

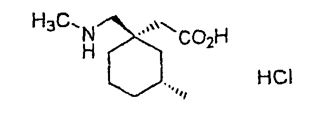

{(1R, 3R)-1-[(Benzyl-methyl-amino)-methyl]-3-methyl-cyclohexyl}-acetic acid; hydrochloride salt (Example 64, 0.60 g, 1.84 mmol) was dissolved in ethanol (50 mL). 20% Pd/C (0.1 g), and the reaction was shaken under an atmosphere of H₂ (50 psi i.e. 344.737 Kilopascals) for 7 min.. The reaction was filtered, 1 M HCl in ether (5 mL) was added, and the solution was concentrated in vacuo. The residue was then dissolved in hot isopropanol (100 mL), filtered, and concentrated in vacuo. The residue was then dissolved in the minimum amount of hot isopropanol necessary and recrystallized from ether and hexane. The crystals were to give 0.15 g (35%) of the desired product.
MS: 200.1 (M+1 for C₁₁H₂₁NO₂); HRMS: (calc) 200.1650, (found) 200.1646 (M+1 for C₁₁H₂₁NO₂); mp: 160-161 °C; IR (KBr, cm⁻¹): 2925 (br), 1719, 1457; Analysis (C₁₁H₂₁NO₂·HCl·0.40 H₂O) (Cal.) C: 54.38, H: 9.46, N: 5.77; (found) C: 54.27, H: 9.36, N: 5.65. ¹H NMR (CD₃OD) δ 0.83-0.97 (m, 5H), 1.20-1.28 (m, 1H) 1.42-1.74 (m, 6H) 2.38 (s, 2H), 2.73 (s, 3H), 3.23 (s, 2H)

### EXAMPLE 66: Summary

Using an *ex vivo* model of freshly dissected rat retinas, the mechanisms by which brain glutamate levels are maintained within normal limits in neural tissue was investigated. Anaplerotic glutamate synthesis may be important to replenish glutamate oxidized in the glia following neurotransmission. Points of rate control through this *de novo* pathway were identified as pyruvate carboxylation and branched chain amino transamination. To test the relationship between glutamate synthesis and pyruvate carboxylase activity, synthesis of ¹⁴C-glutamate plus ¹⁴C-glutamine from H¹⁴CO₃ was measured as a function of medium pyruvate. Increasing pyruvate from 0.2 to 5 mM increased the ¹⁴C-amino acid synthesis by 66 ± 15%. The specific radioactivity of glutamine relative to that of H¹⁴CO₃ after 20 minutes of incubation indicated that □30% of glutamine in the retina is derived from bicarbonate fixation. To test the dependence of glutamate synthesis on availability of branched chain amino acids (BCAA) as a source of nitrogen, the effect of inhibiting the neuronal branched chain aminotransferase (BCATc) was measured. Gabapentin (GBP) (1 mM), a selective inhibitor of BCATc in neurons, inhibited synthesis of ¹⁴C-glutamate plus ¹⁴C-glutamine from H¹⁴CO₃ by 31% without slowing H¹⁴CO₃ incorporation into lactate and citric acid cycle intermediates. Inhibition of *de novo* glutamate synthesis was reversed by addition of 0.6 mM BCAA. The data indicated that pyruvate carboxylase and branched chain aminotransferase share rate control of *de novo* glutamate synthesis in the retina, and that *de novo* glutamate synthesis is quantitatively important for maintenance of retinal glutamate levels.

### Methods

### Experimental animals

Sprague Dawley rats (200-400g) were used for all experiments. Rats were housed under a 12 hour light/dark cycle, and fed and watered *ad libidum*. After anaesthetizing the rats with-nembutal (IP), eyes were enucleated and retinas dissected in ice-cold buffer.

### CO₂ fixation

Freshly isolated half-retinas were preincubated at 37°C for 3 minutes in buffer A (pH 7.4) containing 118 mM NaCl, 4.7 mM KCl, 2.5 mM CaCl, 1.2 mM KH₂PO₄, 1.17 mM MgSO₄, 20 mM HEPES, 5 mM glucose, 0.05 mM NH₄Cl and 25 mM NaH¹⁴CO₃. Pyruvate (either 0.2 or 5mM) was also included in the buffer. 1 ml buffer as well as the gas phase within 20 ml vials were equilibrated with 95% O₂, 5% CO₂. Incubations were initiated by addition of 10 µCi/ml H¹⁴CO₃. The vessels were then immediately closed to the atmosphere. Most incubations continued for 20 minutes, although in some preliminary studies, times were varied from 10-60 minutes. Reactions were stopped by removing the half retinas from the buffer and placing them in 2% perchloric acid. The buffer was separately acidified with perchloric acid (final concentration 2%) and unreacted ¹⁴CO₂ was allowed to diffuse out of the acidified samples.

The total amount of product (non-volatile carbon 14 metabolites) was determined by measuring the radioactivity in aliquots of the medium and tissue samples. Metabolites in the extracts were separated by Dowex-1 acetate chromatography as previously described: Williamson, J.R., and Corkey, B. (1969) "Methods in Enzymology", Vol, 13, ed. Lowenstein, J.M. *(Academic Press)* pp 434-513, and quantified by scintillation counting. In most instances, individual metabolite carbon 14 data is reported as the sum of the radioactivity in the medium plus tissue extracts. Complete quantification of ¹⁴C-metabolite amounts in both the retina and in the medium can be found below in Tables I and II.

Data not shown in the results and discussion sections where collected in the course of carrying out the evaluation of the influences of pyruvate on H¹⁴CO₃ fixation in the retina. Figure 2 shows only the effect of pyruvate on the sum glutamate plus glutamine and the sum pyruvate plus lactate. Table I provides values for all metabolites that became labeled with H¹⁴CO₃ and their compartmentation.

Likewise additional data, not shown in the results and discussion sections, were collected in evaluating the influence of gabapentin. Thus Table II is an expansion of the data shown in Figure 4, and provides values for all metabolites labeled with H¹⁴CO₃ and their compartmentation.

**Table I**

| Condition | Compartment | Gln | Glu | Asp | Lact | TCA cycle | Total |
|---|---|---|---|---|---|---|---|
| Control (0.2 Pyr) | in | 0.59 ± 0.05 | 1.47 ± 0.29 | 0.85 ± 0.06 | 0.16 ± 0.06 | 1.02 ± 0.26 | 4.08 ± 0.41 |
| | out | 0.61 ± 0.08 | 0.04 ± 0.01 | 0.08 ± 0.01 | 0.55 ± 0.03 | 0 | 1.19 ± 0.13 |
| | sum | 1.20 ± 0.09 | 1.51 ± 0.29 | 0.93 ± 0.08 | 0.71 ± 0.06 | 1.02 ± 0.26 | 5.27 ± 0.45 |
| +5.0 Pyr | in | 0.91 ± 0.13 | 2.67 ± 0.29 | 1.08 ± 0.12 | 0.29 ± 0.04 | 2.31 ± 0.13 | 7.26 ± 0.61 |
| | out | 0.96 ± 014 | 0.06 ± 0.01 | 0.08 ± 0.01 | 1.18 ± 0.11 | 0 | 2.24 ± 021 |
| | sum | 1.87 ± 023 | 2.73 ± 028 | 1.16 ± 0.12 | 1.47 ± 0.13 | 2.31 ±0.13 | 9.50 ± 0.74 |

**Table II**

| ondition | Compartment | Gln | Glu | Asp | Lact | TCA cycle | Total |
|---|---|---|---|---|---|---|---|
| ontrol | in | 0.440 ± 0.032 | 1.477 ± 0.097 | 0.634 ± 0.038 | 0.080 ± 0.008 | 1.096 ± 0.105 | 3.715 ± 0.266 |
| | out | 0.551 ± 0.036 | 0.008 ± 0.001 | 0.070 ± 0.003 | 0.478 ± 0.030 | 0 | 1.117 ± 0.075 |
| | sum | 0.991 ± 0.054 | 1.485 ± 0.096 | 0.704 ± 0.040 | 0.558 ± 0.031 | 1.096 ± 0.105 | 4.832 ± 0.303 |
| +GBP | in | 0.389 ± 0.026 | 1.324 ± 0.025 | 0.549 ± 0.019 | 0.089 ± 0.006 | 1.514 ± 0.103 | 3.902 ± 0.122 |
| | out | 0.545 ± 0.024 | 0.008 ± 0.002 | 0.035 ± 0.003 | 0.516 ± 0.030 | 0 | 1.066 ± 0.028 |
| | sum | 0.934 ± 0.036 | 1.332 ± 0.025 | 0.584 ± 0.022 | 0.605 ± 0.028 | 1.514 ± 0.103 | 4.968 ± 0.028 |
| +GBP +BCAA | in | 0.480 ± 0.011 | 1.549 ± 0.070 | 0.609 ± 0.021 | 0.086 ± 0.008 | 1.220 ± 0.116 | 3.989 ± 0.043 |
| | out | 0.806 ± 0.086 | 0.009 ± 0.001 | 0.093 ± 0.008 | 0.458 ± 0.048 | 0 | 1.322 ± 0.076 |
| | sum | 1.286 ± 0.082 | 1.558 ± 0.070 | 0.702 ± 0.016 | 0.5445 ± 0.040 | 1.220 ± 0.116 | 5.311 ± 0.119 |

The chromatographic procedure employed in this example separates glutamine, glutamate, aspartate, and lactic acid. The citric acid cycle intermediates remain on the Dowex columns and are estimated as the difference between the total counts in the sample and the counts that are eluted. Evidence of the validity of this procedure has been reported previously. Before subjecting samples to chromatography, ¹⁴C-pyruvic acid in the samples was converted stoichiometrically to lactate by treating the samples with excess NADH and lactate dehydrogenase. Therefore the single lactate peak reported contains all carbon 14 in both lactate and pyruvate. To determine specific activity of glutamate and glutamine in the retinal samples, the mass amount of the glutamate was measured fluorometrically using a standard enzymatic procedure: Patel, M.S. (1989) in Neuromethods: Carbohydrate and Energy Metabolism, Vol II, eds. Boulton, A.A and Baker G.D. *(The Humana Press, Inc.)* pp 309-340. The mass amount of glutamine was measured after chromatographic separation from glutamate followed by a luminometric determination previously described. ATP and creatine phosphate were measured fluorometrically using an enzymatic assay.

Retinal protein was determined using the DC protein assay (Bio-Rad, Richmond, CA, USA). Each half retina contained about 0.4 mg protein.

### Pyruvate Carboxylase Assay

Pyruvate carboxylase activity was assayed in fresh samples of whole brain and excised retinas. The method described by Patel Patel, M.S. (1989) in *Neuromethods: Carbohydrate and Energy Metabolism*, Vol II, eds. Boulton, A.A and Baker G.D. (The Humana Press, Inc.) pp 309-340, was used and involves measuring ¹⁴CO₂ fixation under optimal conditions in sonicated tissue.

### Western Blot

SDS-PAGE was performed as described Patel, M.S. (1989) in *Neuromethods: Carbohydrate and Energy Metabolism,* Vol II, eds. Boulton, A.A and Baker G.D. (The Humana Press, Inc.) pp 309-340, using 10% gels. For immunoblotting, protein were transferred to Immobilon P membranes. Membranes were blocked with 5% BSA and incubated with immunoaffinity purified rabbit anti-rat BCATc peptide antibody (1:1000 dilution) or immunoaffinity purified rabbit anti-human BCATm peptide antibody (1:1000 dilution). The immunoreactive protein bands were visualized using the ECL system according to manufacturer's instructions (Amersham, Arlington Heights, IL, USA).

### Calculations and Statistics

Data are reported in text and figures by determining the dpm/mg in a particular metabolite and dividing by the specific activity of medium HCO₃. Therefore all data are reported as nmoles of H¹⁴CO₃/mg protein for any given metabolite.

Results are reported as the mean ± standard error of the mean. Statistical significance was judged by paired t-test and a p<0.05. Numbers of independent determinations (n) are provided in figure and table legends.

### RESULTS

### Pyruvate carboxylase levels are similar in retina and whole brain

Patel demonstrated that pyruvate carboxylase is the major CO₂ fixing enzyme in the adult rodent brain. To judge whether this might also be true in the retina, the activity of pyruvate carboxylase at 37°C in sonicates from neocortex was compared with those from the retina. The neocortex levels (2.9 ± 0.1 nmoles H¹⁴CO₃ fixed/min/mg) were similar to those found in sonicated retinal tissues (3.4 ± 0.4 nmoles H¹⁴CO₃ fixed/min/mg), indicating that pyruvate carboxylase was just as active in retina as whole brain.

### Rates of both ¹⁴CO₂ fixation and ¹⁴C-glutamate appearance are a function of pyruvate levels in the retina

In preliminary experiments retinas were incubated with 0.2 mM pyruvate as described in methods for 10, 20, 40 and 60 minute periods. Total ¹⁴CO₂ was monitored as well as ATP and creatine phosphate levels. ATP and creatine phosphate levels remained constant as a function of time, demonstrating that oxygenation and pH control were adequate throughout. Total CO₂ fixation (5.50 ± 0.14 nmoles/mg protein) peaked between 20 and 40 minutes, but was already 76% of maximal (4,2 ± 0.1 nmoles/mg) at 10 minutes. Since ATP (20 ± 0.2 nmoles/mg) and creatine phosphate (16 ± 1.1 nmoles/mg) levels were high and did not change with time, this peak indicated that the rate of CO₂ fixation was constant, but that continuous breakdown of non-volatile carbon 14 products to CO₂ + H₂O produced a near steady state level of glutamate and glutamine specific radioactivity by 20 minutes.

To assess the control of pyruvate carboxylation over glutamate synthesis, retinas were incubated with either 0.2 or 5 mM pyruvate in the medium and intracellular and extracellular metabolites analyzed. The total ¹⁴CO₂ fixed in 20 minutes in the presence of 0.2 mM pyruvate was 5.27 ± 0.45 nmoles H¹⁴CO₃/mg, whereas that fixed in the presence of 5 mM pyruvate was 9.50 ± 0.74 nmoles H¹⁴CO₃/mg. Figure 2 shows that increasing unlabelled pyruvate from 0.2 to 5 mM increases the sum ¹⁴C-glutamate plus ¹⁴C-glutamine (from 1.74 ± 0.19 to 2.90 ± 0.31 nmoles/mg, p<0.005). Figure 2 also shows that the higher pyruvate incubation increases the ¹⁴C-lactate plus ¹⁴C-pyruvate values. Thus, at steady state, an increase in pyruvate concentration from 0.2 to 5 mM increased incorporation of H¹⁴CO₃ into glutamate and glutamine by 66% whereas the increase in pyruvate increased H¹⁴CO₃ incorporation into pyruvate and lactate by 111%. The carbon 14 cycled into pyruvate and lactate represents glial recycling through pyruvate carboxylase, malic enzyme and phosphoenolpyruvate carboxykinase and for the first time demonstrates active citric acid cycle decarboxylation in intact neural tissue.

These data show that an increase in flux through pyruvate carboxylase induces an increase in *de novo* synthesis of retinal glutamate. A somewhat larger increase in pyruvate recycling occurs simultaneously.

### A large fraction of total glutamine synthesis is derived from pyruvate carboxylation

The importance of pyruvate carboxylation as a source for replenishing the glutamate released from retinal nerve endings during neuronal transmission was assessed by determining the specific radioactivity of glutamine in retinal tissue incubated with medium containing H¹⁴CO₃. A fixed proportion of glutamine synthesis in retina derives from unlabelled glutamate released by neurons and taken up by Müller glia. The remainder comes from glial α-ketoglutarate synthesized anaplerotically from pyruvate and ¹⁴CO₂. Therefore, the specific radioactivity of glutamine relative to the specific activity of H¹⁴CO₃ provides a minimal value for the proportion of glutamine derived from anaplerosis. The product of pyruvate carboxylase in the glia is [1,4-¹⁴C]-oxaloacetate. Both the 1 and 4 positions are labelled because the carbon 14 initially incorporated into the 4-C position of oxaloacetate is randomized between the 1 and 4 positions by equilibration of oxalacetate with symmetrical fumarate. The labelled oxaloacetate condenses with pyruvate to form citrate, which is then isomerized to isocitrate. [1,4-¹⁴C]-isocitrate is oxidized and decarboxylated to [5-¹⁴C] α-ketoglutarate by isocitrate dehydrogenase. Therefore the α-ketoglutarate synthesized anaplerotically has approximately half of the specific radioactivity of the original substrate H¹⁴CO₃, depending on the degree of randomization of the label in oxaloacetate. This is also true of the glutamine synthesized from the [5-¹⁴C] α-ketoglutarate.

The specific activity of glutamine was measured in the retinal tissue extracts incubated with 0.2 mM pyruvate and H¹⁴CO₃ for 10, 20, 40 and 60 minutes. Only the tissue glutamine (not medium) is reported. Specific radioactivity of glutamine in the tissue was constant between 10 and 60 minutes. After 20 minutes of incubation the mass amount of glutamine in the tissue was 4.50 ± 0.44 nmoles/mg, and its specific radioactivity was 1970 ± 115 dpm/nmol or 0.158 ± 0.009 dpm/nmol of the bicarbonate specific activity. This indicates that about 32% of the glutamine was produced by anaplerosis and pyruvate carboxylase.

The specific radioactivity of retinal glutamate (269 ± 8 dpm/nmol) was lower than that of glutamine and also constant as a function of time from 10-60 min. It was only 14% of the specific activity of glutamine. This suggests that glutamate metabolic turnover in the neurons is rapid with respect to anaplerotic synthesis. However, since neuronal glutamate turnover cannot replenish the glutamate carbon skeleton lost during neurotransmission, the anaplerotic pathway is of prime importance in maintaining neuronal glutamate levels. The present data show unequivocally that the anaplerotic pathway provides a large (□32%) proportion of the glutamine required for glutamate replenishment.

### Synthesis of retinal glutamate and glutamine is partially dependent on branched chain amino acid transamination

Recent studies of glutamate synthesis in cultured astrocytes suggested that the conversion of α-ketoglutarate to glutamate could act as a rate-controlling step in anaplerotic synthesis of glutamate. Several groups have indicated that the α-amino group of glutamate cannot be supplied as ammonia and must therefore be supplied by transamination of α-ketoglutarate with another amino acid: Kanamori, K., Ross, B.D., and Kondrat, R.W. (1998) *J. Neurochem.* 70, 1304-1315, Laemmli, U.K (1970) *Nature* 227, 680-885, including our own Haberg, A., Qu, H., Bakken, I.J., Sande, L.M., White, L.R, Haraldseth, O., Unsgard, G., Assley, J., Sonnewald, U. (1998) *Dev. Neurosci.* 20, 389-398. Branched chain amino acids are likely nitrogen contributors since they can cross the blood-brain and blood-retinal barriers efficiently. Hutson and coworkers have proposed that a nitrogen shuttle between neurons and glia which involves branched chain amino acids and two BCAT isoenzymes, one in neuronal cytosol (BCATc) and one in glial mitochondria (BCATm) can also provide the needed nitrogen: Hutson, S.M., Berkich, D., Down, P., Xu, B., Aschner, M., and LaNoue, K.F. (1998) *J. Neurochem.* 71, 863-874. According to this hypothetical shuttle, neuronal BCATc facilitates glial BCATm conversion of α-ketoglutarate to glutamate by providing BCAA regenerated in the neurons (cf Figure 1).

As a first step toward investigating the role of the BCAT isoenzymes in *de novo* glutamate synthesis in the retina, immunoblotting with BCATm and BCATc specific antibodies was used to determine whether the BCAT isoenzymes are expressed in rat retina. As shown in Figure 3, both BCATc and BCATm are expressed in the retina at levels that are comparable to those found in whole brain homogenates. Therefore, freshly dissected rat retina is an appropriate intact neural system in which to test the concept of nitrogen shuttling between neurons and glia.

The leucine analog gabapentin (GBP) is a neuroactive drug which inhibits BCATc, but not BCATm: Su, T.-Z., Lunney, E., Campbell, G., Oxender, D.L. (1995) *J. Neurochem.* 64, 2125-2131. It was confirmed that GBP inhibits the synthesis of leucine in freshly dissected rat retinas (data not shown). The effect of GBP on the synthesis of ¹⁴C-glutamate plus ¹⁴C-glutamine from H¹⁴CO₃ in *ex vivo* rat retinas was then measured. As shown in Figure 4, 1 mM GBP reduced formation of ¹⁴C-glutamate plus ¹⁴C-glutamine to 71% of control values (2.26 ± 0.05 versus 3.18 ± 0.12 nmol/mg, p<0.001). Addition of 200 µM each of leucine, isoleucine and valine reversed the inhibition to 89% of control (2.84 ± 1.15 nmol/mg, p<0.01 versus GBP alone). There was no effect of GBP on either ATP or creatine phosphate indicating that the GBP did not impair retinal bioenergetic processes. The small increase in incorporation of carbon 14 into lactate and citric acid cycle intermediates (from 1.93 ± .06 to 2.12 ± 0.13) due to the presence of 1 mM GBP was not significant. Incorporation of the label into all of the metabolites is shown in Table II.

Further experiments were carried out to investigate dose/response properties of GBP. Freshly dissected retinas were incubated for 20 minutes in buffer A containing either 0, 0.2, 1, or 5 mM GBP. As shown in Figure 5, increasing the concentration of GBP over 1 mM did not significantly increase the extent of its inhibition of the synthesis of ¹⁴C-glutamate plus ¹⁴C-glutamine from H¹⁴CO₃. Again GBP has no influence on formation of citric acid cycle intermediates, lactate and pyruvate (Figure 5). This supports the proposal that the site of GBP inhibition of glutamate synthesis is the BCAT mediated conversion of α-ketoglutarate to glutamate.

### Discussion

The studies described evaluated mechanisms for maintenance of CNS glutamate levels. Previous studies showed that glutamine synthetase is required in retinas and hippocampal slices to maintain levels of neuronal glutamate: Su, T.-Z., Lunney, E., Campbell, G., Oxender, D.L. (1995) *J. Neurochem.* 64, 2125-2131. Immunocytochemical measurements indicate that 90% or more of retinal and whole brain glutamate is neuronal and that inhibition of glutamine synthetase for 90 minutes depletes the neuronal glutamate pool to negligible levels: Ottersen, O.P., Zhang, N., and Walberg, F. (1992) *Neurosci.* 46, 519-534. This demonstrated that glutamine synthetase is required for maintenance of neuronal glutamate. Although glutamate can be synthesized in neurons from α-ketoglutarate, lack of pyruvate carboxylase or another anaplerotic enzyme prevents replenishment of α-ketoglutarate, and thus, precludes net glutamate synthesis within the neurons except from glutamine via glutaminase.

In this example the sources and control of glutamine synthesis was investigated by incubating excised retinas with H¹⁴CO₃ and measuring the appearance and specific radioactivity of ¹⁴C-glutamine and ¹⁴C-glutamate. More ¹⁴C-glutamine is synthesized in the presence of 5 mM pyruvate than in the presence of 0.2 mM. This indicates that pyruvate carboxylase activity influences the rate of glutamine synthesis. However, lack of sensitivity of this control is demonstrated by the observation that when pyruvate is increased from 0.2 mM to 5 mM the magnitude of the increase in pyruvate carboxylase flux (80.3%) is not directly translated into an equivalent increase in ¹⁴C-glutamate plus ¹⁴C-glutamine (69.0%). Flexibility of the control is provided by the potential for citric acid cycle decarboxylation and pyruvate recycling. The pyruvate dependence of total H¹⁴CO₃ fixation in retinal tissue was similar to that seen in cultured isolated astrocytes: Gamberino, W.C., Berkich, D.A., Lynch, C.J., Xu, B., and LaNoue, K.F (1997) *J. Neurochem. 69*, 2312-2325. However, in astrocytes, which have no way to regenerate endogenous BCAA used to transaminate α-ketoglutarate, the pyruvate-induced increases in CO₂ fixation-were not translated into increases in ¹⁴C-glutamate and ¹⁴C-glutamine levels. Instead, the carbon 14 accumulated solely in citric acid cycle intermediates and into "recycled" pyruvate.

In the retinal studies reported here, the specific radioactivity of glutamine at steady state was 0.158 of the bicarbonate specific activity. Since, approximately half of the ³⁴C incorporated into oxalacetate is retained in α-ketoglutarate, the results show that ≈ 32% of the glutamine is derived from H¹⁴CO₃. This is the first direct estimate of the contribution of anaplerosis to glutamine synthesis in a neuronal tissue. Previous estimates have been indirect, and involved isotopomer analysis of NMR spectra following infusion of ¹³C glucose (3-5, 11-13) into intact brains. Estimates of the contribution of pyruvate carboxylation to glutamine synthesis using these NMR methods have been lower than the present one, and range from less than 1% (3) to 5-10% (4,5) to 20-25%: Shank, R.P., Bennet, G.S., Freytag, S.O., and Campbell, G.L. (1985) *Brain Res*. 329, 3 64-367, Griffen, J.L., Rae, C., Radda, G.K., and Matthews, P.M. (1999) *Biochim. Biophys. Acta* 1450, 297-307, depending largely on initial assumptions of the mathematical models employed. The present results suggest that glial pyruvate carboxylation and the conversion of citric acid cycle intermediates to glutamate and glutamine are likely to be quantitiatively important for retinal function and possibly for whole brain function.

The observation that GBP slows the conversion of citric acid cycle intermediates to glutamine and glutamate strongly suggests that neuronal synthesis of BCAA by BCATc is necessary for optimal rates of amination of α-ketoglutarate to glutamate. A reasonable scheme for their involvement in this process is shown in Figure 1.

The results, which demonstrate inhibition of ¹⁴C-glutamate and ¹⁴C-glutamine synthesis from H¹⁴CO₃ by GBP and reversal of the inhibition by BCAA, are consistent with the described shuttle. The hypothesis also provides a possible explanation for the anti-epileptic action of GBP: Taylor, C.P., Gee, N.S., Su, T.Z., Kocsis, J.D., Welty, D.F., Brown, J.P., Dooley, D.J., Boden, P., Singh, L. (1998) *Epilepsy Res.* 29, 233-249. Indeed, decreased glutamate synthesis is a common action of GBP and the anti-epileptic carbonic anhydrase inhibitors: Hazen, S.A., Waheed, A., Sly, W.S., LaNoue, K.F., Lynch, C.J. (1997) *Dev. Neurosci*. 19, 162-171. Also, Maple Syrup Urine Disease, the hereditary ailment caused by a mutation of the gene for branched chain keto acid dehydrogenase, if untreated induces high serum BCAA in patients and subsequent neurological abnormalities: Chuang, D.I. and Shih, V.E. (1995) *in The Metabolic Basis of Inherited Disease*, eds. Schriver, C.R., Beaudet, A.L., Sly, S., and Vaile, D. (McGraw-Hill) pp. 1239-1277, which may be related to abnormal regulation of glutamate synthesis. Finally, the implications of nitrogen shuttling during glutamate synthesis must be studied further in light of the efficacy of GBP in the treatment of neuropathic pain: Ottersen, O.P., Zhang, N., and Walberg, F. (1992) *Neurosci.* 46, 519-534 and neurodegeneration, Chuang, D.I. and Shih, V.E. (1995) in *The Metabolic Basis of Inherited Disease,* eds. Schriver, C.R., Beaudet, A.L., Sly, S., and Vaile, D. (McGraw-Hill) pp. 1239-1277, Rothsetin, J.D. and Kuncl, R.W. (1995) *J. Neurochem*. 65, 643-651.

### EXAMPLE 67

**Enzyme preparation**: Frozen rat brains were thawed on ice and minced into fine pieces. For each brain 5 ml of isotonic buffer [225 mM mannitol, 75 mM sucrose, 5 mM MOPS (pH 7.1), 1 mM EDTA, 1 mM EGTA, 1 mM DTT containing leupeptin, pepstatin and PMSF] was added. The samples were homogenized in Potters homogenizer (B. Bran Biotech International) at 500 rpm for ten strokes, followed by additional ten strokes at 800 rpm. The homogenates were centrifuged in Beckman J2-M1 centrifuge with rotor JA-20 at 19, 000 rpm for 1 hr. The supernatant was dialyzed with at least three changes of dialysis buffer (225 mM mannitol, 75 mM sucrose, 5 mM MOPS pH 7.1, 1mM EDTA, 1 mM EGTA, and 1 mM DTT) for 24 hrs. The resulting enzyme was stored at -80 °C for further assays.

**Assay of BCAT**: The reaction was done in 1.5 ml Eppendorf tubes in a volume of 100 µl containing 50 mM Tris-HCL (pH 8.3), 25 µM [¹⁴C] L-leucine, 10 mM 2-oxoglutarate, 15 µM pyridoxal phosphate, 1 mM DTT, and enzyme. The solutions for the reaction mixture were made in 10-fold concentrations. After 10 min incubation at room temperature (22 °C), the reaction was stopped by adding 100 µl of 15 mM p-nitrophenyl hydrazine in 2 M HCL. After a few minutes, 1 mL of a toluene-based scintillation liquid (4.5 g of PPO and 0.5 g ofbis-MSB in 1 liter of toluene) was added, the tube was capped and mixed thoroughly with a Vortex mixer. Then the tubes were centrifuged at 13, 500 rpm (Eppendorf centrifuge 5415C) for 10 min. Six hundreds µl of organic layer from each tube was placed into scintillation vials, followed by adding 10 ml of scintillation cocktail for counting.

| Example # | rBCATc IC50 (uM) |
|---|---|
| 1 | 146 |
| 2 | 1136 |
| 3 | 1660 |
| 4 | 2515 |
| 5 | 2934 |
| 6 | 2620 |
| 7 | 2165 |
| 8 | 74 |
| 9 | 120 |
| 10 | 121 |
| 11 | 1387 |
| 12 | 1490 |
| 13 | >10,000 |
| 14 | 8592 |
| 15 | 82 |
| 16 | 1319 |
| 17 | >10,000 |
| 18 | --- |
| 19 | --- |
| 20 | 469 |
| 21 | 169 |
| 22 | 389 |
| 23 | 2121 |
| 24 | 942 |
| 25 | 170 |
| 26 | 8592 |
| 27 | 1553 |
| 28 | 181 |
| 29 | 391 |
| 30 | 2250 |
| 31 | 908 |
| 32 | 243 |
| 33 | 1018 |
| 34 | 3218 |
| 35 | 1768 |
| 36 | --- |
| 37 | 1193 |
| 38 | 2618 |
| 39 | 933 |
| 40 | 3800 |
| 41 | 363 |
| 42 | 669 |
| 43 | 2079 |
| 44 | 2728 |
| 45 | 1331 |
| 46 | 1129 |
| 47 | 15580 |
| 48 | 8530 |
| 49 | 84 |
| 50 | 842 |
| 51 | >10,000 |
| 52 | 2516 |
| 53 | 3340 |
| 54 | 217 |
| 55 | 9242 |
| 56 | --- |
| 57 | --- |
| 58 | 179 |
| 59 | 3370 |
| 60 | 11530 |
| 61 | 114 |
| 62 | 4216 |
| 63 | --- |
| 64 | --- |
| 65 | --- |

## Claims

1. A compound of Formula IV 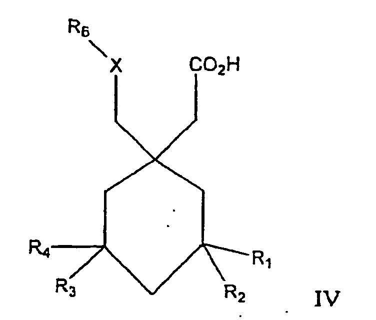 R1-R4 are hydrogen or alkyl;
X is NR5 or O;
R5 is hydrogen or alkyl,
R6 is alkyl, benzyl, alkoxyalkanoyl, arylalkyl, alkoxy, cycloalkyl, allyl, alkylcycloalkyl, trisubstituted halogenalkyl, and wherein R₁-R₄ are each hydrogen the R₆ is not hydrogen or methyl; or a pharmaceutically acceptable salt, ester, prodrug, or amide thereof.

2. A compound according to Claim 1 wherein
R₂ and R₄ are hydrogen and R₁ and R₃ are alkyl;
R₂ and R₄ are hydrogen and R₁ and R₃ are methyl;
R₁ - R₄ are hydrogen;
R₁ is alkyl and R₂ - R₄ are hydrogen;
R₁ is methyl and R₂- R₄ are hydrogen;
R₅ is hydrogen;
X is O;
R₆ is alkyl;
R₆ is benzyl;
R₆ is phenylalkyl;
R₆ is cycloalkyl;
R₆ is trifluoroalkyl;
R₆ is alkylcycloalkyl;
R₆ is alkoxy;
R₆ is allyl.

3. A compound according to Claim 1 wherein
R₂ and R₄ are hydrogen and R₁ and R₃ are methyl;
R₁-₄ are hydrogen;
R₁ is methyl and R₂-R4 are hydrogen;
R₅ is hydrogen;
R₆ is alkyl;
R₆ is benzyl;
R₆ is phenylalkyl;
R₆ is cycloalkyl;
R₆ is trifluoroalkyl;
R₆ is alkylcycloalkyl;
R₆ is alkoxy;
R₆ is allyl.

4. A compound according to Claim 1 wherein
R₂ and R₄ are hydrogen and R₁ and R₃ are methyl;
R₁-R₄ are hydrogen.

5. A compound according to Claim 1 and selected from the group consisting of:
(1-Allylaminomethyl-cyclohexyl)-acetic acid;
(1-Prop-2-ynylaminomethyl-cyclohexyl)-acetic acid;
{1-[(2,2,2-Trifluoro-ethylamino)-methyl]-cyclohexyl}-acetic acid;
{1-[(3,3,3-Trifluoro-propylamino)-methyl]-cyclohexyl}-acetic acid;
1α,3β,5β-(1-Allylaminomethyl-3,5-dimethyl-cyclohexyl)-acetic acid;
1α,3β,5β-(3,5-Dimethyl-1-prop-2-ynylaminomethyl-cyclohexyl)-acetic acid;
1α,3β,5β-{3,5-Dimethyl-1-[(2,2,2-trifluoro-ethylamino)-methyl]-cyclohexyl}-acetic acid;
1α,3β,5β-{3,5-Dimethyl-1-[(3,3,3-trifluoro-propylamino)-methyl]-cyclohexyl}-acetic acid;
trans-((1R,3R)-1-Allylaminomethyl-3-methyl-cyclohexyl)-acetic acid;
trans-((1R,3R)-3-Methyl-1-prop-2-ynylaminomethyl-cyclohexyl)-acetic acid;
trans-{(1R,3R)-3-Methyl-1-[(2,2,2-trifluoro-ethylamino)-methyl]-cyclohexyl}-acetic acid;
trans-{(1R,3R)-3-Methyl-1-[(3,3,3-trifluoro-propylamino)-methyl]-cyclohexyl}-acetic acid;
trans-{(1R,3R)-3-Methyl-1-[(4,4,4-trifluoro-butylamino)-methyl]-cyclohexyl}-acetic acid;
1α,3β,5β-{3,5-Dimethyl-1-[(4,4,4-trifluoro-butylamino)-methyl]-cyclohexyl}-acetic acid;
1α,3β,5β-{1-[(Cyclopropylmethyl-amino)-methyl]-3,5-dimethyl-cyclohexyl}-acetic acid;
trans-{(1R,3R)-1-[(Cyclopropylmethyl-amino)-methyl]-3-methyl-cyclohexyl}-acetic acid;
trans-((1R,3R)-3-Methyl-1-methylaminomethyl-cyclohexyl)-acetic acid;
trans-((1R,3R)-1-Ethylaminomethyl-3-methyl-cyclohexyl)-acetic acid;
trans-((1R,3R)-3-Methyl-1-propylaminomethyl-cyclohexyl)-acetic acid;
trans-((1R,3R)-1-Butylaminomethyl-3-methyl-cyclohexyl)-acetic acid;
1α,3β,5β-(3,5-Dimethyl-1-propylaminomethyl-cyclohexyl)-acetic acid, hydrochloride salt;
1α,3β,5β-(1-Ethylaminomethyl-3,5-dimethyl-cyclohexyl)-acetic acid, hydrochloride salt;
1α,3β,5β-(1-Benzylamino-methyl)-3,5-dimethyl-cyclohexyl]-acetic acid, hydrochloride salt;
1α,3β,5β-(1-Dimethylaminomethyl-3,5-dimethyl-cyclohexyl)-acetic acid, hydrochloride salt;
1α,3β,5β-(1-Butylaminomethyl-3,5-dimethyl-cyclohexyl)-acetic acid, hydrochloride salt;
1α,3β,5β-{1-[(Benzyl-methyl-amino)-methyl]-3,5-dimethyl-cyclohexyl}-acetic acid, bydrochloride salt;
1α,3β,5β-(3,5-Dimethyl-1-methylaminomethyl-cyclohexyl)-acetic acid, hydrochloride salt;
1α,3β,5β-[1-(Isobutylamino-methyl)-3,5-dimethyl-cyclohexyl]-acetic acid, hydrochloride salt;
1α,3β,5β-[3,5-Dimethyl-1-(phenethylamino-methyl)-cyclohexyl]-acetic acid, hydrochloride salt;
1α,3β,5β-{3,5-Dimethyl-1-[(3-phenyl-propylamino)-methyl]-cyclohexyl}-acetic acid, hydrochloride salt;
{1-[(Cyclobutylmethyl-amino)-methyl]-cyclohexyl}-acetic acid, hydrochloride salt;
1α,3β,5β-[1-(Isopropylamino-methyl)-3,5-dimethyl-cyclohexyl]-acetic acid, hydrochloride salt;
{1-[(2-Methyl-butylamino)-methyl]-cyclohexyl}-acetic acid, hydrochloride salt;
{1-[(4,4,4-Trifluoro-butylamino)-methyl]-cyclohexyl}-acetic acid, hydrochloride salt;
(1-Ethylaminomethyl-cyclohexyl)-acetic acid, hydrochloride salt;
{1-[(Cyclopropylmethyl-amino)-methyl]-cyclohexyl}-acetic acid, hydrochloride salt;
[1-(Isobutylamino-methyl)-cyclohexyl]-acetic acid, hydrochloride salt;
(1-Propylaminomethyl-cyclohexyl)-acetic acid, hydrochloride salt;
[1-(Isopropylamino-methyl)-cyclohexyl]-acetic acid, hydrochloride salt;
(1-Cyclohexylaminomethyl-cyclohexyl)-acetic acid, hydrochloride salt;
[1-(Benzylamino-methyl)-cyclohexyl]-acetic acid, hydrochloride salt;
((1R,3R)-3-Methyl-1-propylaminomethyl-cyclohexyl)-acetic acid, hydrochloride salt;
{1-[Cyclopentylmethyl-amino)-methyl]-cyclohexyl}-acetic acid, hydrochloric salt;
{1-[(Cyclohexylmethyl-amino)-methyl]-cyclohexyl}-acetic acid, hydrochloric salt;
[1-(*tert*-Butoxycarhonylamino-methyl)-cyclohexyl]-acetic acid;
((3R, 5S)-1-Cyclobutylaminomethyl-3,5-dimethyl-cyclohexyl)-acetic acid, hydrochloride salt;
{(3R, 5S)-3,5-Dimethyl-1-[(2-methyl-butylamino)-methyl]-cyclohexyl}-acetic acid, hydrochloride salt;
{(3R, 5S)-1-[(2,2-Dimethoxy-ethylamino)-methyl]-3,5-dimethyl-cyclohexyl}-acetic acid, hydrochloride salt;
{(3R, 5S)-1-[(Cyclopentylmethyl-amino)-methyl]-3,5-dimethyl-cyclohexyl}-acetic acid, hydrochloride salt;
{(3R,5S)-1-[(Cyclohexylmethyl-amino)-methyl]-3,5-dimethyl-cyclohexyl}-acetic acid, hydrochloride salt;
((3R, 5S)-1-Cyclohexylaminomethyl-3,5-dimethyl-cyclohexyl)-acetic acid, hydrochloride salt;
(1-Dimethylaminomethyl-cyclohexyl)-acetic acid, hydrochloride salt;
(1-Butylaminomethyl-cyclohexyl)-acetic acid, hydrochloride salt;
{1-[2,2-Dimethoxy-ethylamino)-methyl]-cyclohexyl}-acetic acid, hydrochloride salt;
(1-methylaminomethyl-cyclohexyl)-acetic acid, hydrochloride salt;
{1-[(Benzyl-methyl-amino)-methyl]-cyclohexyl}-acetic acid, hydrochloride salt;
[1-(Phenethylamino-methyl)-cyclohexyl]-acetic acid, hydrochloride salt;
{1-[(3-Phenyl-propylamino)-methyl]-cyclohexyl}-acetic acid, hydrochloride salt;
((3R, 5S)-1-Hydroxymethyl-3,5-dimethyl-cyclohexyl)-acetic acid, sodium salt;
((3R, 5S)-1-Ethylaminomethyl-3,5-dimethyl-cyclohexyl)-acetic acid, hydrochloride salt;
((3R, 5S)-3,5-Dimethyl-1-propylaminomethyl-cyclohexyl)-acetic acid, hydrochloride salt;
[(1R, 3R)-1-(Benzylamino-methyl)-3-methyl-cyclohexyl]-acetic acid, hydrochloride salt;
{(1R, 3R)-1-[(Benzyl-methyl-amino)-methyl]-3-methyl-cyclohexyl}-acetic acid, hydrochloride salt;
or
((1R, 3R)-3-Methyl-1-methylaminomethyl-cyclohexyl)-acetic acid, hydrochloride salt;

6. Use of a compound of formula I, II and/or III: 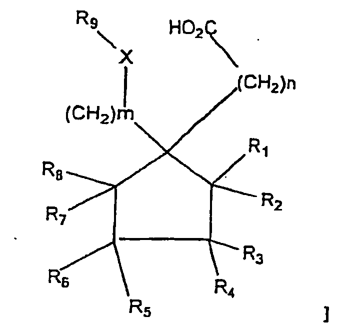 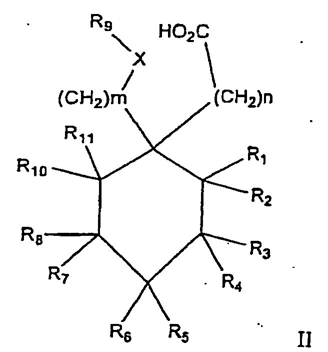 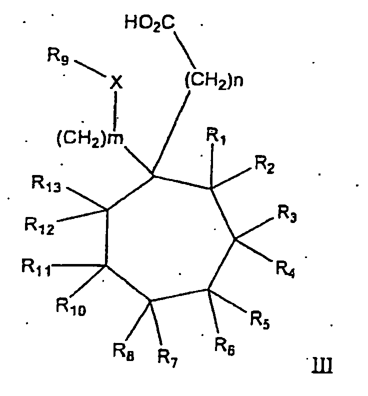 wherein:
R₉ is H; alkyl; cycloalkyl; substituted alkyl containing halogen, amine, alkoxy, cycloalkyl, or hydroxy; allyl; alkynyl; alkanoyl; alkoxyalkanoyl; sulfonyl; phenyl; benzyl; or arylalkyl;
m and n are independently an integer of 1-3;
R₁ - R₈ and R₁₀ - R₁₄ are independently H, alkyl, or substituted alkyl; and
X = NR₁₄, O, or S
where there is more than one stereoisomer, each chiral center may be independently R or S; or a pharmaceutically acceptable salt, ester, prodrug, or amide thereof
for the manufacture of a pharmaceutical for the treatment of diabetic retinopathy.

7. Use according to Claim 6 wherein
m and n are 1;
X is NR₁₄;
R₉ is H;
R₄ is methyl;
R₄ and R₅ are methyl;
R₈ is methyl;
R₁₀ is methyl;
R₇ and R₈ are methyl;
R₄ and R₈ are methyl;
R₁ - R₈ and R₁₀- R₁₃ are H;
R₉ is alkyl,
Rg is benzyl;
R₉ is arylalkyl;
R₉ is cycloalkyl;
R₁₄ is alkyl;
R₁-R₈ are H;
R₁ - R₈ and R₁₀ - R₁₁ are H;
R₁ - R₂ and R₇ - R₈ are H;
or R₂ is methyl.

8. Use according to Claim 6 wherein
R₃ is alkyl, R₁ - R₂ and R₄ - R₁₁ and R₁₄ are hydrogen and m and n are 1, and X is NR₁₄;
R₃ and R₁₁ are alkyl, R₁ - R₂ and R₄ - R₁₀ and R₁₄ are hydrogen, m and n are 1, and X is NR₁₄;
R₃ and R₁₁ are alkyl, R₁ - R₂ and R₄ - R₁₀ and R₁₄ are hydrogen, m and n are 1, R₉ is alkyl, and X is NR₁₄;
R₁ - R₁₁ and R₁₄ are hydrogen, m and n are 1, and X is O.

9. Use according to Claim 6 wherein the compound is selected from the group consisting of:
(1-Allylaminomethyl-cyclohexyl)-acetic acid;
(1-Prop-2-ynylaminomethyl-cyclohexyl)-acetic acid;
{1-[(2,2,2-Trifluoro-ethylamino)-methyl]-cyclohexyl}-acetic acid;
{1-[(3,3,3-Trifluoro-propylamino)-methyl]-cyclohexyl}-acetic acid;
1α,3β,5β-(1-Allylaminomethyl-3,5-dimethyl-cyclohexyl)-acetic acid;
1α,3β,5β-(3,5-Dimethyl-1-prop-2-ynylaminomethyl-cyclohexyl)-acetic acid;
1α,3β,5β-{3,5-Dimethyl-1-[(2,2,2-trifluoro-ethylamino)-methyl]-cyclohexyl}-acetic acid;
1α,3β,5β-{3,5-Dimethyl-1-[(3,3,3-trifluoro-propylamino)-methyl]-cyclohexyl}-acetic acid;
trans-((1R,3R)-1-Allylaminomethyl-3-methyl-cyclohexyl)-acetic acid;
trans-((1R,3R)-3-Methyl-1-prop-2-ynylaminomethyl-cyclohexyl)-acetic acid;
trans-{(1R,3R)-3-Methyl-1-[(2,2,2-trifluoro-ethylamino)-methyl]-cyclohexyl}-acetic acid;
trans-{(1R,3R)-3-Methyl-1-[(3,3,3-trifluoro-propylamino)-methyl]-cyclohexyl}-acetic acid;
trans-{(1R,3R)-3-Methyl-1-[(4,4,4-trifluoro-butylamino)-methyl]-cyclohexyl}-acetic acid;
1α,3β,5β-{3,5-Dimethyl-1-[(4,4,4-trifluoro-butylamino)-methyl]-cyclohexyl}-acetic acid;
1α,3β,5β-{1-[(Cyclopropylmethyl-amino)-methyl]-3,5-dimethyl-cyclohexyl}-acetic acid;
trans-{(1R,3R)-1-[(Cyclopropylmethyl-amino)-methyl]-3-methyl-cyclohexyl}-acetic acid;
trans-((1R,3R)-3-Methyl-1-methylaminomethyl-cyclohexyl)-acetic acid;
trans-((1R,3R)-1-Ethylaminomethyl-3-methyl-cyclohexyl)-acetic acid;
trans-((1R,3R)-3-Methyl-1-propylaminomethyl-cyclohexyl)-acetic acid;
trans-((1R,3R)-1-Butylaminomethyl-3-methyl-cyclohexyl)-acetic acid;
trans-((1R,3R)-1-Hydroxymethyl-3-methyl-cyclohexyl)-acetic acid;
1α,3β,5β-{1-[Hydroxymethyl-amino)-methyl]-3,5-dimethyl-cyclohexyl}-acetic acid;
1α,3β,5β-(1-Aminomethyl-3,5-diethyl-cyclohexyl)-acetic acid, hydrochloride;
trans-(1R,3R)(1-Aminomethyl-3-methyl-cyclohexyl)-acetic acid, hydrochloride;
(1-Aminomethyl-2-methyl-cyclohexyl)-acetic acid, hydrochloride;
(1-Aminomethyl-3,3-dimethyl-cyclohexyl)-acetic acid, hydrochloride;
(±)-(trans)-(1-Aminomethyl-3,4-dimethyl-cyclopentyl)-acetic acid, hydrochloride;
(cis/trans)-(3R)-(1-Aminomethyl-3-methyl-cyclopentyl)-acetic acid, hydrochloride;
(+)-(trans)-(1-Aminomethyl-3,4-dimethyl-cyclopentyl)-acetic acid, hydrochloride;
(+)-(trans)-(1-Aminomethyl-3,4-dimethyl-cyclopentyl)-acetic acid, hydrochloride;
1α,3β,5β-(1-Aminomethyl-3,5-dimethyl-cyclohexyl)-acetic acid, hydrochloride;
1α,3β,5β-(3,5-Dimethyl-1-propylaminomethyl-cyclohexyl)-acetic acid, hydrochloride salt;
1α,3β,5β-(1-Ethylaminomethyl-3,5-dimethyl-cyclohexyl)-acetic acid, hydrochloride salt;
1α,3β,5β-(1-Benzylamino-methyl)-3,5-dimethyl-cyclohexyl]-acetic acid, hydrochloride salt;
1α,3β,5β-(1-Dimethylaminomethyl-3,5-dimethyl-cyclohexyl)-acetic acid, hydrochloride salt
1α,3β,5β-(1-Butylaminomethyl-3,5-dimethyl-cyclohexyl)-acetic acid, hydrochloride salt;
1α,3β,5β-{1-[(Benzyl-methyl-amino)-methyl]-3.5-dimethyl-cyclohexyl}-acetic acid, hydrochloride salt;
1α,3β,5β-(3,5-Dimethyl-1-methylaminomethyl-cyclohexyl)-acetic acid, hydrochloride salt;
1α,3β,5β-[1-(Acetylamino-methyl)-3,5-dimethyl-cyclohexyl]-acetic acid;
1α,3β,5β-(1-(Isobutylamino-methyl)-3,5-dimethyl-cyclohexyl]-acetic acid, hydrochloride salt;
1α,3β,5β-[3,5-Dimethyl-1-(phenethylamino-methyl)-cyclohexyl]-acetic acid, hydrochloride salt;
1α,3β,5β-{3,5-Dimethyl-1-[(3-phenyl-propylamino)-methyl]-cyclohexyl}-acetic acid, hydrochloride salt;
{1-[(Cyclobutylmethyl-amino)-methyl]-cyclohexyl}-acetic acid, hydrochloride salt;
1α,3β,5β-(1-(Isopropylamino-methyl)-3,5-dimethyl-cyclohexyl]-acetic acid, hydrochloride salt;
1-Aminomethyl-1-cyclohexane-acetic acid;
1-Aminomethyl-1-cyclopentane-acetic acid;
1-Aminomethyl -1-cyclopentane-acetic acid, sodium salt;
1-(hydroxymethyl)cyclohexane-acetic acid, sodium salt;
{1-[(2-Methyl-butylamino)-methyl]-cyclohexyl}-acetic acid, hydrochloride salt;
{1-[(4,4,4-Trifluoro-butylamino)-methyl]-cyclohexyl}-acetic acid, hydrochloride salt;
(1-Ethylaminomethyl-cyclohexyl)-acetic acid, hydrochloride salt;
{1-[(Cyclopropylmethyl-amino)-methyl]-cyclohexyl}-acetic acid, hydrochloride salt;
{1-[(2-Hydroxy-1-methyl-ethylamino)-methyl]-cyclohexyl}-acetic acid, hydrochloride salt;
[1-(Isobutylamino-methyl)-cyclohexyl]-acetic acid, hydrochloride salt;
(1-Propylaminomethyl-cyclohexyl)-acetic acid, hydrochloride salt;
[1-(Isopropylamino-methyl)-cyclohexyl]-acetic acid, hydrochloride salt;
(1-Cyclohexylaminomethyl-cyclohexyl)-acetic acid, hydrochloride salt;
[1-(Benzylamino-methyl)-cyclohexyl]-acetic acid, hydrochloride salt;
((1R,3R)-3-Methyl-1-propylaminomethyl-cyclohexyl)-acetic acid, hydrochloride salt;
{1-[Cyclopentylmethyl-amino)-methyl]-cyclohexyl}-acetic acid, hydrochloric salt;
{1-[(Cyclohexylmethyl-amino)-methyl]-cyclohexyl}-acetic acid, hydrochloric salt;
[1-(*tert*-Butoxycarbonylamino-methyl)-cyclohexyl]-acetic acid;
[1-(Acetylamino-methyl)-cyclohexyl]-acetic acid;
((3R, 5S)-1-Cyclobutylaminomethyl-3,5-dimethyl-cyclohexyl)-acetic acid, hydrochloride salt;
{(3R, 5S)-3,5-Dimethyl-1-[(2-methyl-butylamino)-methyl]-cyclohexyl}-acetic acid, hydrochloride salt;
{(3R, 5S)-1-[(2-Hydroxy-1-methyl-ethylamino)-methyl]-3,5-dimethylcyclohexyl}-acetic acid, hydrochloride salt;
{(3R, 5S)-1-[(2,2-Dimethoxy-ethylamino)-methyl)-3,5-dimethyl-cyclohexyl}-acetic acid, hydrochloride salt;
{(3R, 5S)-1-[(Cyclopentylmethyl-amino)-methyl]-3,5-dimethyl-cyclohexyl}-acetic acid, hydrochloride salt;
{(3R,5S)-1-[(Cyclohexylmethyl-amino)-methyl]-3,5-dimethyl-cyclohexyl}-acetic acid, hydrochloride salt;
((3R, 5S)-1-Cyclohexylaminomethyl-3,5-dimethyl-cyclohexyl)-acetic acid, hydrochloride salt;
((3R,5S)-1-Carboxymethyl-3,5-dimethyl-cyclohexyl)-acetic acid;
*trans*-((3R, 5S)-1-Hydroxymethyl-3,5-dimethyl-cyclohexyl-acetic acid, hydrochloride salt;
*cis*-((3R, 5S)-1-Hydroxymethyl-3,5-dimethyl-cyclohexyl)-acetic acid, hydrochloride salt;
(1-Dimethylaminomethyl-cyclohexyl)-acetic acid, hydrochloride salt;
(1-Butylaminomethyl-cyclohexyl)-acetic acid, hydrochloride salt;
{1-[2,2-Dimethoxy-ethylamino)-metnyl]-cyclohexyl}-acetic acid, hydrochloride salt;
(1-methylaminomethyl-cyclohexyl)-acetic acid, hydrochloride salt;
{1-[(Benzyl-methyl-amino)-methyl]-cyclohexyl}-acetic acid, hydrochloride salt;
[1-(Phenethylamino-methyl)-cyclohexyl]-acetic acid, hydrochloride salt;
{1-[(3-Phenyl-propylamino)-methyl]-cyclohexyl}-acetic acid, hydrochloride salt;
((3R, 5S)-1-Hydroxymethyl-3,5-dimethyl-cyclohexyl)-acetic acid, sodium salt;
((3R, 5S)-1-Ethylaminomethyl-3,5-dimethyl-cyclohexyl)-acetic acid, hydrochloride salt;
(1-Aminomethyl-4-ethyl-cyclohexyl)-acetic acid, hydrochloric salt;
(1-Aminomethyl-4-propyl-cyclohexyl)-acetic acid, hydrochloric salt;
((3R, 5S)-3,5-Dimethyl-1-propylaminomethyl-cyclohexyl)-acetic acid, hydrochloride salt;
[(1R,3R)-1-(Benzylamino-methyl)-3-methyl-cyclohexyl]-acetic acid, hydrochloride salt;
{(1R, 3R)-1-[(Benzyl-methyl-amino)-methyl]-3-methyl-cyclohexyl}-acetic acid, hydrochloride salt;
or
((1R, 3R)-3-Methyl-1-methylaminomethyl-cyclohexyl)-acetic acid, hydrochloride salt;

10. Use of a compound of formula I, II and/or III: 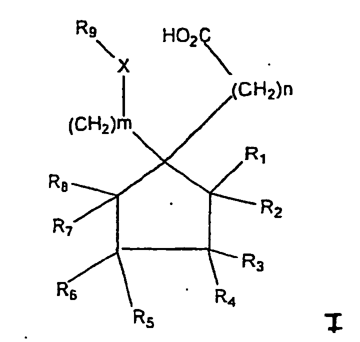 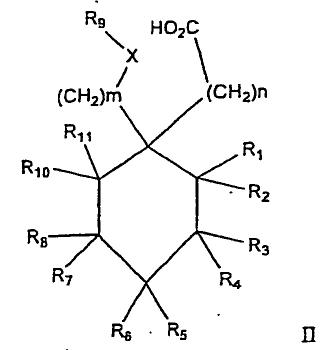 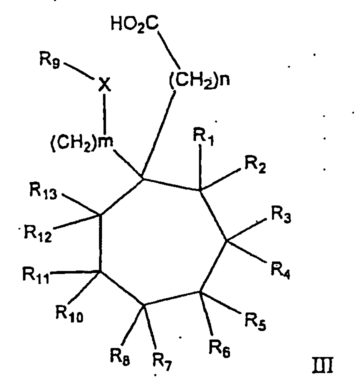 wherein:
R₉ is H; alkyl; cycloalkyl; substituted alkyl containing halogen, amine, alkoxy, cycloalkyl, or hydroxy; allyl; alkynyl; alkanoyl; alkoxyalkanoyl; sulfonyl; phenyl; benzyl; or arylalkyl;
and the proviso that R₉ cannot be hydrogen or alkanoyl for compounds of formula II; m and n are independently an integer of 1-3;
R₁ - R₈ and R₁₀ - R₁₄ are independently H, alkyl, or substituted alkyl; and
X = NR₁₄, O, or S
where there is more than one stereoisomer, each chiral center may be independently R or S; or a pharmaceutically acceptable salt, ester, prodrug, or amide thereof
for the manufacture of a pharmaceutical for inhibiting the branch chain amino acid-dependent aminotransferase.

11. Use according to Claim 10 wherein
m and n are 1;
X is NR_{14;}
R₉ is H;
R₄ is methyl;
R₄ and R₅ are methyl;
R₈ is methyl;
R₁₀ is methyl;
R₇ and R₈ are methyl;
R₄ and R₈ are methyl;
R₁ - R₈ and R₁₀ - R₁₃ are H;
R₉ is alkyl;
R₉ is benzyl;
R₉ is arylalkyl;
R₉ is cycloalkyl;
R₁₄ is alkyl;
R₁ - R₈ are H;
R₁ - R₈ and R₁₀ - R₁₁ are H;
R₁ - R₂ and R₇ - R₈ are H;
or R₂ is methyl.

12. Use according to Claim 10 wherein
R₃ is alkyl, R₁ - R₂ and R₄ - R₁₁ and R₁₄ are hydrogen and m and n are 1, and X is NR₁₄;
R₃ and R₁₁ are alkyl, R₁ - R₂ and R₄ - R₁₀ and R₁₄ are hydrogen, m and n are 1, and X is NR₁₄;
R₃ and R₁₁ are alkyl, R₁ - R₂ and R₄ - R₁₀ and R₁₄ are hydrogen, m and n are 1, R₉ is alkyl, and X is NR₁₄;
R₁- R₁₁ and R₁₄ are hydrogen, m and n are 1, and X is O.

13. Use according to Claim 10 wherein the compound is selected from:
(1-Allylaminomethyl-cyclohexyl)-acetic acid;
(1-Prop-2-ynylaminomethyl-cyclohexyl)-acetic acid;
{1-[(2,2,2-Trifluoro-ethylamino)-methyl]-cyclohexyl}-acetic acid;
{1-[(3,3,3-Trifluoro-propylamino)-methyl]-cyclohexyl}-acetic acid;
1α,3β,5β-(1-Allylaminomethyl-3.5-dimethyl-cyclohexyl)-acetic acid;
1α,3β,5β-(3,5-Dimethyl-1-prop-2-ynylaminomethyl-cyclohexyl)-acetic acid;
1α,3β,5β-{3,5-Dimethyl-1-[(2,2,2-trifluoro-ethylamino)-methyl]-cyclohexyl}-acetic acid;
1α,3β,5β-{3,5-Dimethyl-1-[(3,3,3-trifluoro-propylamino)-methyl]-cyclohexyl}-acetic acid;
trans-((1R,3R)-1-Allylaminomethyl-3-methyl-cyclohexyl)-acetic acid;
trans-((1R,3R)-3-Methyl-1-prop-2-ynylaminomethyl-cyclohexyl)-acetic acid;
trans-{(1R,3R)-3-Methyl-1-[(2,2,2-trifluoro-ethylamino)-methyl]-cyclohexyl}-acetic acid;
trans-{(1R,3R)-3-Methyl-1-[(3,3,3-trifluoro-propylamino)-methyl]-cyclohexyl}-acetic acid;
trans-{(1R,3R)-3-Methyl-1-[(4,4,4-trifluoro-butylamino)-methyl]-cyclohexyl}-acetic acid;
1α,3β,5β-{3,5-Dimethyl-1-[(4,4,4-trifluoro-butylamino)-methyl]-cyclohexyl}-acetic acid;
1α,3β,5β-{1-[(Cyclopropylmethyl-amino)-methyl]-3,5-dimethyl-cyclohexyl}-acetic acid;
trans-{(1R,3R)-1-[(Cyclopropylmethyl-amino)-methyl]-3-methyl-cyclohexyl}-acetic acid;
trans-((1R,3R)-3-Methyl-1-methylaminomethyl-cyclohexyl)-acetic acid;
trans-((1R,3R)-1-Ethylaminomethyl-3-methyl-cyclohexyl)-acetic acid;
trans-((1R,3R)-3-Methyl-1-propylaminomethyl-cyclohexyl)-acetic acid;
trans-((1R,3R)-1-Butylaminomethyl-3-methyl-cyclohexyl)-acetic acid;
trans-((1R,3R)-1-Hydroxymethyl-3-methyl-cyclohexyl)-acetic acid;
1α,3β,5β-{1-[(Hydroxymethyl-amino)-methyl]-3,5-dimethyl-cyclohexyl}-acetic acid;
1α,3β,5β-(1-Aminomethyl-3,5-diethyl-cyclohexyl)-acetic acid, hydrochloride;
trans-(1R,3R)(1-Aminomethyl-3-methyl-cyclohexyl)-acetic acid, hydrochloride;
(1-Aminomethyl-2-methyl-cyclohexyl)-acetic acid, hydrochloride;
(1-Aminomethyl-3,3-dimethyl-cyclohexyl)-acetic acid, hydrochloride;
(±)-(trans)-(1-Aminomethyl-3,4-dimethyl-cyclopentyl)-acetic acid, hydrochloride;
(cis/trans)-(3R)-(1-Aminomethyl-3-methyl-cyclopentyl)-acetic acid, hydrochloride;
(+)-(trans)-(1-Aminomethyl-3,4-dimethyl-cyclopentyl)-acetic acid, hydrochloride;
(+)-(trans)-(1-Aminomethyl-3,4-dimethyl-cyclopentyl)-acetic acid, hydrochloride;
1α,3β,5β-(1-Aminomethyl-3,5-dimethyl-cyclohexyl)-acetic acid, hydrochloride;
1α,3β,5β-(3,5-Dimethyl-1-propylaminomethyl-cyclohexyl)-acetic acid, hydrochloride salt;
1α,3β,5β-(1-Ethylaminomethyl-3,5-dimethyl-cyclohexyl)-acetic acid, hydrochloride salt;
1α,3β,5β [-(1-Benzylamino-methyl)-3,5-dimethyl-cyclohexyl]-acetic acid, hydrochloride salt;
1α,3β,5β-(1-Dimethylaminomethyl-3,5-dimethyl-cyclohexyl)-acetic acid, hydrochloride salt;
1α,3β,5β-(1-Butylaminomethyl-3,5-dimethyl-cyclohexyl)-acetic acid, hydrochloride salt;
1α,3β,5β-{1-[(Benzyl-methyl-amino)-methyl]-3,5-dimethyl-cyclohexyl}-acetic acid, hydrochloride salt;
1α,3β,5β-(3,5-Dimethyl-1-methylaminomethyl-cyclohexyl)-acetic acid, hydrochloride salt;
1α,3β,5β-[1-(Acetylamino-methyl)-3,5-dimethyl-cyclohexyl]-acetic acid;
1α,3β,5β-[1-(Isobutylamino-methyl)-3,5-dimethyl-cyclohexyl]-acetic acid, hydrochloride salt;
1α,3β,5β-[3,5-Dimethyl-1-(phenethylamino-methyl)-cyclohexyl]-acetic acid, hydrochloride salt;
1α,3β,5β-{3,5-Dimethyl-1-[(3-phenyl-propylamino)-methyl]-cyclohexyl}-acetic acid, hydrochloride salt;
{1-[(Cyclobutylmethyl-amino)-methyl]-cyclohexyl}-acetic acid, hydrochloride salt;
1α,3β,5β-[1-(Isopropylamino-methyl)-3,5-dimethyl-cyclohexyl]-acetic acid, hydrochloride salt;
1-Aminomethyl-1-cyclohexane-acetic acid;
1-Aminomethyl-1-cyclopentane-acetic acid;
1-Aminomethyl-1'-cyclopentane-acetic acid, sodium salt;
1-(hydroxymethyl)cyclohexane-acetic acid, sodium salt;
{1-[(2-Methyl-butylamino)-methyl]-cyclohexyl}-acetic acid, hydrochloride salt;
{1-[(4,4,4-Trifluoro-butylamino)-methyl]-cyclohexyl}-acetic acid, hydrochloride salt;
(1-Ethylaminomethyl-cyclohexyl)-acetic acid, hydrochloride salt;
{1-[(Cyclopropylmethyl-amino)-methyl]-cyclohexyl}-acetic acid, hydrochloride salt;
{1-[(2-Hydroxy-1-methyl-ethylamino)-methyl]-cyclohexyl}-acetic acid, hydrochloride salt;
[1-(Isobutylamino-methyl)-cyclohexyl]-acetic acid, hydrochloride salt;
(1-Propylaminomethyl-cyclohexyl)-acetic acid, hydrochloride salt;
[1-(Isopropylamino-methyl)-cyclohexyl]-acetic acid, hydrochloride salt;
(1-Cyclohexylaminomethyl-cyclohexyl)-acetic acid, hydrochloride salt;
[1-(Benzylamino-methyl)-cyclohexyl]-acetic acid, hydrochloride salt;
((1R,3R)-3-Methyl-1-propylaminomethyl-cyclohexyl)-acetic acid, hydrochloride salt;.
{1-[Cyclopentylmethyl-amino)-methyl]-cyclohexyl}-acetic acid, hydrochloric salt;
{1-[(Cyclohexylmethyl-amino)-methyl]-cyclohexyl}-acetic acid, hydrochloric salt;
[1-(*tert*-Butoxycarbonylamino-methyl)-cyclohexyl]-acetic acid;
[1-(Acetylamino-methyl)-cyclohexyl]-acetic acid;
((3R, 5S)-1-Cyclobutylaminomethyl-3,5-dimethyl-cyclohexyl)-acetic acid, hydrochloride salt;
{(3R, 5S)-3,5-Dimethyl-1-[(2-methyl-butylamino)-methyl]-cyclohexyl}-acetic acid, hydrochloride salt;
{(3R, 5S)-1-[(2-Hydroxy-1-methyl-ethylamino)-methyl]-3,5-dimethylcyclohexyl}-acetic acid, hydrochloride salt;
{(3R, 5S)-1-[(2,2-Dimethoxy-ethylamino)-methyl]-3,5-dimethyl-cyclohexyl}-acetic acid, hydrochloride salt;
{(3R, 5S)-1-[(Cyclopentylmethyl-amino)-methyl]-3,5-dimethyl-cyclohexyl}-acetic acid, hydrochloride salt;
{(3R,5S)-1-[(Cyclohexylmethyl-amino)-methyl]-3,5-dimethyl-cyclohexyl}-acetic acid, hydrochloride salt;
((3R, 5S)-1-Cyclohexylaminomethyl-3,5-dimethyl-cyclohexyl)-acetic acid, hydrochloride salt;
((3R,5S)-1-Carboxymethyl-3,5-dimethyl-cyclohexyl)-acetic acid;
*trans*-((3R, 5S)-1-Hydroxymethyl-3,5-dimethyl-cyclohexyl)-acetic acid, hydrochloride salt;
*cis*-((3R, 5S)-1-Hydroxymethyl-3,5-dimethyl-cyclohexyl)-acetic acid, hydrochloride salt;
(1-Dimethylaminomethyl-cyclohexyl)-acetic acid, hydrochloride salt;
(1-Butylaminomethyl-cyclohexyl)-acetic acid, hydrochloride salt;
{1-[2,2-Dimethoxy-ethylamino)-methyl]-cyclohexyl}-acetic acid, hydrochloride salt;
(1-methylaminomethyl-cyclohexyl)-acetic acid, hydrochloride salt;
{1-[(Benzyl-methyl-amino)-methyl]-cyclohexyl}-acetic acid, hydrochloride salt;
[1-(Phenethylamino-methyl)-cyclohexyl]-acetic acid, hydrochloride salt;
{1-[(3-Phenyl-propylamino)-methyl]-cyclohexyl}-acetic acid, hydrochloride salt;
((3R, 5S)-1-Hydroxymethyl-3,5-dimethyl-cyclohexyl)-acetic acid, sodium salt;
((3R, 5S)-1-Ethylaminomethyl-3,5-dimethyl-cyclohexyl)-acetic acid, hydrochloride salt;
(1-Aminomethyl-4-ethyl-cyclohexyl)-acetic acid, hydrochloric salt;
(1-Aminomethyl-4-propyl-cyclohexyl)-acetic acid, hydrochloric salt;
((3R, 5S)-3,5-Dimethyl-1-propylaminomethyl-cyclohexyl)-acetic acid, hydrochloride salt;
[(1R, 3R)-1-(Benzylamino-methyl)-3-methyl-cyclohexyl]-acetic acid, hydrochloride salt;
{(1R, 3R)-1-[(Benzyl-methyl-amino)-methyl]-3-methyl-cyclohexyl}-acetic acid, hydrochloride salt;
or
((1R, 3R)-3-Methyl-1-methylaminomethyl-cyclohexyl)-acetic acid, hydrochloride salt;

14. Use of a compound of formula IV according to claim 1 for the manufacture of a pharmaceutical for the treatment of neurological disorders, depression, anxiety panic, mania, bipolar disorders, antiflammatory diseases, glaucoma, pain or gastrointestinal damage.

## Patentansprüche

1. Verbindung der Formel IV 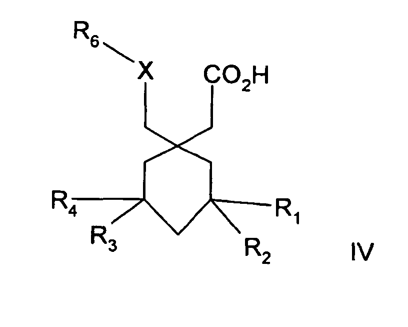 worin
R₁ bis R₄ für Wasserstoff oder Alkyl stehen;
X für NR₅ oder O steht;
R₅ für Wasserstoff oder Alkyl steht;
R₆ für Alkyl, Benzyl, Alkoxyalkanoyl, Arylalkyl, Alkoxy, Cycloalkyl, Allyl, Alkylcycloalkyl oder trisubstituiertes Halogenalkyl steht,
wobei, wenn R₁ - R₄ für Wasserstoff stehen, R₆ nicht für Wasserstoff oder Methyl steht,
oder deren pharmazeutisch akzeptable Salze, Ester, Prodrugs oder Amide.

2. Verbindung nach Anspruch 1, worin
R₂ und R₄ für Wasserstoff und R₁ und R₃ für Alkyl stehen;
R₂ und R₄ für Wasserstoff und R₁ und R₃ für Methyl stehen;
R₁ - R₄ für Wasserstoff stehen;
R₁ für Alkyl und R₂ - R₄ für Wasserstoff stehen;
R₁ für Methyl und R₂ - R₄ für Wasserstoff stehen; .
R₅ für Wasserstoff steht;
X für Sauerstoff steht;
R₆ für Alkyl steht;
R₆ für Benzyl steht;
R₆ für Phenylalkyl steht;
R₆ für Cycloalkyl steht;
R₆ für Trifluoralkyl steht;
R₆ für Alkylcycloalkyl steht;
R₆ für Alkoxy steht;
R₆ für Allyl steht.

3. Verbindung nach Anspruch 1, worin
R₂ und R₄ für Wasserstoff und R₁ und R₃ für Methyl stehen;
R₁ - R₄ für Wasserstoff stehen;
R₁ für Methyl und R₂ - R₄ für Wasserstoff stehen;
R₅ für Wasserstoff steht;
R₆ für Alkyl steht;
R₆ für Benzyl steht;
R₆ für Phenylalkyl steht;
R₆ für Cycloalkyl steht;
R₆ für Trifluoralkyl steht;
R₆ für Alkylcycloalkyl steht;
R₆ für Alkoxy steht;
R₆ für Allyl steht.

4. Verbindung nach Anspruch 1, worin
R₂ und R₄ für Wasserstoff und R₁ und R₃ für Methyl stehen;
R₁ - R₄ für Wasserstoff stehen.

5. Verbindung nach Anspruch 1, ausgewählt aus:
(1-Allylaminomethyl-cyclohexyl)-essigsäure;
(1-Prop-2-inylaminomethyl-cyclohexyl)-essigsäure;
{1-[(2,2,2-Trifluor-ethylamino)-methyl]-cyclohexyl}-essigsäure;
{1-[(3,3,3-Trifluor-propylamino)-methyl]-cyclohexyl}-essigsäure;
1α,3β,5β-(1-Allylaminomethyl-3,5-dimethyl-cyclohexyl)-essigsäure;
1α,3β,5β-(3,5-Dimethyl-1-prop-2-inylaminomethyl-cyclohexyl)-essigsäure;
1α,3β,5β-{3,5-Dimethyl-1-[(2,2,2-trifluor-ethylamino)-methyl]-cyclohexyl}-essigsäure;
1α,3β,5β-{3,5-Dimethyl-1-[(3,3,3-trifluor-propylamino)-methyl]-cyclohexyl}-essigsäure;
trans-((1R,3R)-1-Allylaminomethyl-3-methyl-cyclohexyl)-essigsäure;
trans-((1R,3R)-3-Methyl-1-prop-2-inylaminomethyl-cyclohexyl)-essigsäure;
trans-{(1R,3R)-3-Methyl-1-[(2,2,2-trifluor-ethylamino)-methyl]-cyclohexyl}-essigsäure;
trans-{(1R,3R)-3-Methyl-1-[(3,3,3-trifluor-propylamino)-methyl]-cyclohexyl}-essigsäure;
trans-{(1R,3R)-3-Methyl-1-((4,4,4-trifluor-butylamino)-methyl]-cyclohexyl}-essigsäure
1α,3β,5β-{3,5-Dimethyl-1-[(4,4,4-trifluor-butylamino)-methyl]-cyclohexyl}-essigsäure;
1α,3β,5β-(1-[(Cyclopropylmethyl-amino)-methyl]-3,5-dimethyl-cyclohexyl}-essigsäure;
trans-{(1R,3R)-1-[(Cyclopropylmethyl-amino)-methyl]-3-methyl-cyclohexyl}-essigsäure;
trans-((1R,3R)-3-Methyl-1-methylaminomethyl-cyclohexyl)-essigsäure;
trans-((1R,3R)-1-Ethylaminomethyl-3-methyl-cyclohexyl)-essigsäure;
trans-((1R,3R)-3-Methyl-1-propylaminomethyl-cyclohexyl)-essigsäure;
trans-((1R,3R)-1-Butylaminomethyl-3-methyl-cyclohexyl)-essigsäure;
1α,3β,5β-(3,5-Dimethyl-1-propylaminomethyl-cyclohexyl)}-essigsäure-Hydrochlorid;
1α,3β,5β-(1-Ethylaminomethyl-3,5-dimethyl-cyclohexyl)-essigsäure-Hydrochlorid;
1α,3β,5β-[(1-Benzylamino-methyl)-3,5-dimethyl-cyclohexyl]-essigsäure-Hydrochlorid;
1α,3β,5β-(1-Dimethylaminomethyl-3,5-dimethyl-cyclohexyl)-essigsäure-Hydrochlorid;
1α,3β,5β-(1-Butylaminomethyl-3,5-dimethyl-cyclohexyl)-essigsäure-Hydrochlorid;
1α,3β,5β-{1-[(Benzyl-methyl-amino)-methyl]-3,5-dimethyl-cyclohexyl}-essigsäure-Hydrochlorid;
1α,3β,5β-(3,5-Dimethyl-1-methylaminomethyl-cyclohexyl)-essigsäure-Hydrochlorid;
1α,3β,5β-[1-(Isobutylamino-methyl)-3,5-dimethyl-cyclohexyl]-essigsäure-Hydrochlorid;
1α,3β,5β-[3,5-Dimethyl-1-(phenethylamino-methyl)-cyclohexyl]-essigsäure-Hydrochlorid;
1α,3β,5β-{3,5-Dimethyl-1-[(3-phenyl-propylamino)-methyl]-cyclohexyl}-essigsäure-Hydrochlorid;
{1-[(Cyclobutylmethyl-amino)-methyl]-cyclohexyl}-essigsäure-Hydrochlorid;
1α,3β,5β-[1-(Isopropylamino-methyl)-3,5-dimethyl-cyclohexyl]-essigsäure-Hydrochlorid;
{1-[(2-Methyl-butylamino)-methyl]-cyclohexyl}-essigsäure-Hydrochlorid;
{1-[(4,4,4-Trifluor-butylamino)-methyl]-cyclohexyl}-essigsäure-Hydrochlorid;
(1-Ethylaminomethyl-cyclohexyl)-essigsäure-Hydrochlorid;
{1-[(Cyclopropylmethyl-amino)-methyl]-cyclohexyl}-essigsäure-Hydrochlorid;
[1-(Isobutylamino-methyl)-cyclohexyl]-essigsäure-Hydrochlorid;
(1-Propylaminomethyl-cyclohexyl)-essigsäure-Hydrochlorid;
[1-(Isopropylamino-methyl)-cyclohexyl]-essigsäure-Hydrochlorid;
(1-Cyclohexylaminomethyl-cyclohexyl)-essigsäure-Hydrochlorid;
[1-(Benzylamino-methyl)-cyclohexyl]-essigsäure-Hydrochlorid;
((1R,3R)-3-Methyl-1-propylaminomethyl-cyclohexyl)-essigsäure-Hydrochlorid;
{1-[(Cyclopentylmethyl-amino)-methyl]-cyclohexyl}-essigsäure-Hydrochlorid;
{1-[(Cyclohexylmethyl-amino)-methyl]-cyclohexyl}-essigsäure-Hydrochlorid;
[1-(*tert*.-Butoxycarbonylamino-methyl)-cyclohexyl]-essigsäure;
((3R,5S)-1-Cyclobutylaminomethyl-3,5-dimethyl-cyclohexyl)-essigsäure-Hydrochlorid;
{(3R,5S)-3,5-Dimethyl-1-[(2-methyl-butylamino)-methyl]-cyclohexyl}-essigsäure-Hydrochlorid;
{(3R,5S)-1-[(2,2-Dimethoxy-ethylamino)-methyl]-3,5-dimethyl-cyclohexyl}-essigsäure-Hydrochlorid;
{(3R,5S)-1-[(Cyclopentylmethyl-amino)-methyl]-3,5-dimethyl-cyclohexyl}-essigsäure-Hydrochlorid;
{(3R,5S)-1-[(Cyclohexylmethyl-amino)-methyl]-3,5-dimethyl-cyclohexyl}-essigsäure-Hydrochlorid;
((3R,5S)-1-Cyclohexylaminomethyl-3,5-dimethyl-cyclohexyl)-essigsäure-Hydrochlorid;
(1-Dimethylaminomethyl-cyclohexyl)-essigsäure-Hydrochlorid;
(1-Butylaminomethyl-cyclohexyl)-essigsäure-Hydrochlorid;
{1-[2,2-Dimethoxy-ethylamino)-methyl]-cyclohexyl}-essigsäure-Hydrochlorid;
(1-Methylaminomethyl-cyclohexyl)-essigsäure-Hydrochlorid;
{1-[(Benzyl-methyl-amino)-methyl]-cyclohexyl}-essigsäure-Hydrochlorid;
[1-(Phenethylamino-methyl)-cyclohexyl]-essigsäure-Hydrochlorid;
{1-[(3-Phenyl-propylamino)-methyl]-cyclohexyl}-essigsäure-Hydrochlorid;
((3R,5S)-1-Hydroxymethyl-3,5-dimethyl-cyclohexyl)-essigsäure-natriumsalz;
((3R,5S)-1-Ethylaminomethyl-3,5-dimethyl-cyclohexyl)-essigsäure-Hydrochlorid;
((3R,5S)-3,5-Dimethyl-1-propylaminomethyl-cyclohexyl)-essigsäure-Hydrochlorid;
[(1R,3R)-1-(Benzylamino-methyl)-3-methyl-cyclohexyl]-essigsäure-Hydrochlorid;
{(1R,3R)-1-[(Benzyl-methyl-amino)-methyl]-3-methyl-cyclohexyl}-essigsäure-Hydrochlorid oder
((1R,3R)-3-Methyl-1-methylaminomethyl-cyclohexyl)-essigsäure-Hydrochlorid.

6. Verwendung einer Verbindung der Formel I, II und/oder III: 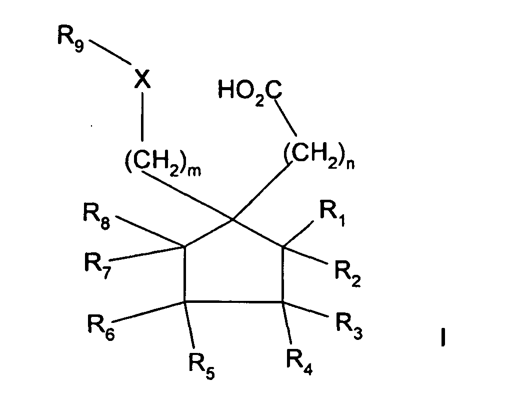 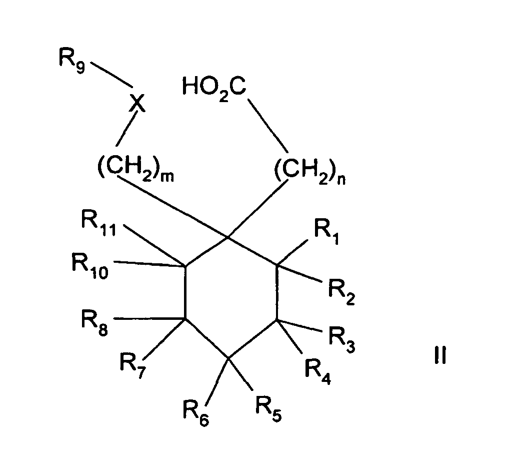 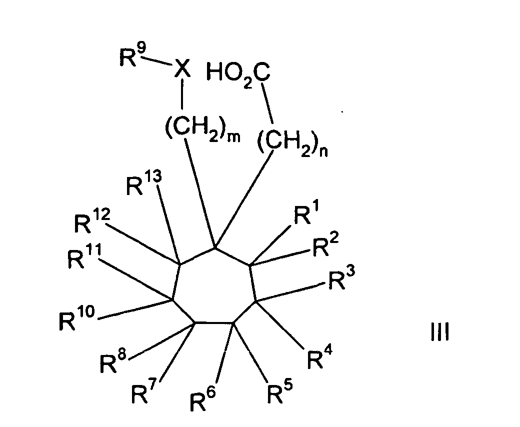 worin
R₉ für Wasserstoff; Alkyl; Cycloalkyl; substituiertes, Halogen, Amin, Alkoxy, Cycloalkyl oder Hydroxy enthaltendes Alkyl; Allyl; Alkinyl; Alkanoyl; Alkoxyalkanoyl; Sulfonyl; Phenyl; Benzyl oder Aralkyl steht;
m und n unabhängig eine ganze Zahl von 1 bis 3 bedeuten;
R₁ - R₈ und R₁₀ - R₁₄ unabhängig ausgewählt sind aus Wasserstoff, Alkyl und substituiertem Alkyl und
X für NR₁₄, Sauerstoff oder Schwefel steht,
wobei im Falle, dass mehr als ein Stereoisomer vorhanden ist, jedes chirale Zentrum unabhängig R oder S sein kann;
oder deren pharmazeutisch akzeptabler Salze, Ester, Prodrugs oder Amide,
zur Herstellung eines Arzneimittels zur Behandlung von diabetischer Retinopathie.

7. Verwendung nach Anspruch 6, worin
m und n 1 bedeuten;
X für NR₁₄ steht;
R₉ für Wasserstoff steht;
R₄ für Methyl steht;
R₄ und R₅ für Methyl stehen;
R₈ für Methyl steht;
R₁₀ für Methyl steht;
R₇ und R₈ für Methyl stehen;
R₄ und R₈ für Methyl stehen;
R₁ - R₈ und R₁₀ - R₁₃ für Wasserstoff stehen;
R₉ für Alkyl steht;
R₉ für Benzyl steht;
R₉ für Arylalkyl steht;
R₉ für Cycloalkyl steht;
R₁₄ für Alkyl steht;
R₁ - R₈ für Wasserstoff stehen;
R₁ - R₈ und R₁₀ - R₁₁ für Wasserstoff stehen;
R₁ - R₂ und R₇ - R₈ für Wasserstoff stehen;
oder R₂ für Methyl steht.

8. Verwendung nach Anspruch 6, worin
R₃ für Alkyl steht, R₁ - R₂, R₄- R₁₁ und R₁₄ für Wasserstoff stehen, m und n jeweils 1 bedeuten und X für NR₁₄ steht;
R₃ und R₁₁ für Alkyl stehen, R₁ - R₂, R₄ - R₁₀ und R₁₄ für Wasserstoff stehen, m und n jeweils 1 bedeuten und X für NR₁₄ steht;
R₃ und R₁₁ für Alkyl stehen, R₁ - R₂, R₁₄ - R₁₀ und R₁₄ für Wasserstoff stehen, m und n jeweils 1 bedeuten, R₉ für Alkyl steht und X für NR₁₄ steht;
R₁ - R₁₁ und R₁₄ für Wasserstoff stehen, m und n jeweils 1 bedeuten und X für Sauerstoff steht.

9. Verwendung nach Anspruch 6, wobei die Verbindung ausgewählt ist aus:
(1-Allylaminomethyl-cyclohexyl)-essigsäure;
(1-Prop-2-inylaminomethyl-cyclohexyl)-essigsäure;
{1-[(2,2,2-Trifluor-ethylamino)-methyl]-cyclohexyl}-essigsäure;
{1-[(3,3,3-Trifluor-propylamino)-methyl]-cyclohexyl}-essigsäure;
1α,3β,5β-(1-Allylaminomethyl-3,5-dimethyl-cyclohexyl)-essigsäure;
1α,3β,5β-(3,5-Dimethyl-1-prop-2-inylaminomethyl-cyclohexyl)-essigsäure;
1α,3β,5β-{3,5-Dimethyl-1-[(2,2,2-trifluor-ethylamino)-methyl]-cyclohexyl}-essigsäure;
1α,3β,5β-{3,5-Dimethyl-1-[(3,3,3-trifluor-propylamino)-methyl]-cyclohexyl}-essigsäure;
trans-((1R,3R)-1-Allylaminomethyl-3-methyl-cyclohexyl)-essigsäure;
trans-((1R,3R)-3-Methyl-1-prop-2-inylaminomethyl-cyclohexyl)-essigsäure;
trans-{(1R,3R)-3-Methyl-1-[(2,2,2-trifluor-ethylamino)-methyl]-cyclohexyl}-essigsäure;
trans-{(1R,3R)-3-Methyl-1-((3,3,3-trifluor-propylamino)-methyl]-cyclohexyl}-essigsäure;
trans-{(1R,3R)-3-Methyl-1-[(4,4,4-trifluor-butylamino)-methyl]-cyclohexyl}-essigsäure
1α,3β,5β-{3,5-Dimethyl-1-[(4,4,4-trifluor-butylamino)-methyl]-cyclohexyl}-essigsäure;
1α,3β,5β-{1-[(Cyclopropylmethyl-amino)-methyl]-3,5-dimethyl-cyclohexyl}-essigsäure;
trans-{(1R,3R)-1-[(Cyclopropylmethyl-amino)-methyl]-3-methyl-cyclohexyl}-essigsäure;
trans-((1R,3R)-3-Methyl-1-methylaminomethyl-cyclohexyl)-essigsäure;
trans-((1R,3R)-1-Ethylaminomethyl-3-methyl-cyclohexyl)-essigsäure;
trans-((1R,3R)-3-Methyl-1-propylaminomethyl-cyclohexyl)-essigsäure;
trans-((1R,3R)-1-Butylaminomethyl-3-methyl-cyclohexyl)-essigsäure;
trans-((1R,3R)-1-Hydroxymethyl-3-methyl-cyclohexyl)-essigsäure;
1α,3β,5β-{1-[(Hydroxymethyl-amino)-methyl]-3,5-dimethyl-cyclohexyl}-essigsäure;
1α,3β,5β-(1-Aminomethyl-3,5-diethyl-cyclohexyl)-essigsäure-Hydrochlorid;
trans-(1R,3R)(1-Aminomethyl-3-methyl-cyclohexyl)-essigsäure-Hydrochlorid;
(1-Aminomethyl-2-methyl-cyclohexyl)-essigsäure-Hydrochlorid;
(1-Aminomethyl-3,3-dimethyl-cyclohexyl)-essigsäure-Hydrochlorid;
(±)-(trans)-(1-Aminomethyl-3,4-dimethyl-cyclopentyl)-essigsäure-Hydrochlorid;
(cis/trans)-(3R)-(1-Aminomethyl-3-methyl-cyclopentyl)-essigsäure-Hydrochlorid;
(+)-(trans)-(1-Aminomethyl-3,4-dimethyl-cyclopentyl)-essigsäure-Hydrochlorid;
(+)-(trans)-(1-Aminomethyl-3,4-dimethyl-cyclopentyl)-essigsäure-Hydrochlorid;
1α,3β,5β-(1-Aminomethyl-3,5-dimethyl-cyclohexyl)-essigsäure-Hydrochlorid;
1α,3β,5β-(3,5-Dimethyl-1-propylaminomethyl-cyclohexyl)-essigsäure-Hydrochlorid;
1α,3β,5β-(1-Ethylaminomethyl-3,5-dimethyl-cyclohexyl)}-essigsäure-Hydrochlorid;
1α,3β,5β-[(1-Benzylamino-methyl)-3,5-dimethyl-cyclohexyl]-essigsäure-Hydrochlorid;
1α,3β,5β-(1-Dimethylaminomethyl-3,5-dimethyl-cyclohexyl)-essigsäure-Hydrochlorid;
1α,3β,5β-(1-Butylaminomethyl-3,5-dimethyl-cyclohexyl)-essigsäure-Hydrochlorid;
1α,3β,5β-{1-[(Benzyl-methyl-amino)-methyl]-3,5-dimethyl-cyclohexyl}-essigsäure-Hydrochlorid;
1α,3β,5β-(3,5-Dimethyl-1-methylaminomethyl-cyclohexyl)-essigsäure-Hydrochlorid;
1α,3β,5β-[1-(Acetylamino-methyl)-3,5-dimethyl-cyclohexyl)-essigsäure-Hydrochlorid;
1α,3β,5β-[1-(Isobutylamino-methyl)-3,5-dimethyl-cyclohexyl]-essigsäure-Hydrochlorid;
1α,3β,5β-[3,5-Dimethyl-1-(phenethylamino-methyl)-cyclohexyl]-essigsäure-Hydrochlorid;
1α,3β,5β-{3,5-Dimethyl-1-[(3-phenyl-propylamino)-methyl]-cyclohexyl}-essigsäure-Hydrochlorid;
{1-[(Cyclobutylmethyl-amino)-methyl]-cyclohexyl}-essigsäure-Hydrochlorid;
1α,3β,5β-[1-(Isopropylamino-methyl)-3,5-dimethyl-cyclohexyl]-essigsäure-Hydrochlorid;
1-Aminomethyl-1-cyclohexan-essigsäure;
1-Aminomethyl-1-cyclopentan-essigsäure;
1-Aminomethyl-1-cyclopentan-essigsäure-Natriumsalz;
1-(Hydroxymethyl)cyclohexan-essigsäure-Natriumsalz;
{1-[(2-Methyl-butylamino)-methyl]-cyclohexyl}-essigsäure-Hydrochlorid;
{1-[(4,4,4-Trifluor-butylamino)-methyl]-cyclohexyl}-essigsäure-Hydrochlorid;
(1-Ethylaminomethyl-cyclohexyl)-essigsäure-Hydrochlorid;
{1-[(Cyclopropylmethyl-amino)-methyl]-cyclohexyl}-essigsäure-Hydrochlorid;
{1-[(2-Hydroxy-1-methyl-ethylamino)-methyl]-cyclohexyl}-essigsäure-Hydrochlorid;
[1-(Isobutylamino-methyl)-cyclohexyl]-essigsäure-Hydrochlorid;
(1-Propylaminomethyl-cyclohexyl)-essigsäure-Hydrochlorid;
[1-(Isopropylamino-methyl)-cyclohexyl]-essigsäure-Hydrochlorid;
(1-Cyclohexylaminomethyl-cyclohexyl)-essigsäure-Hydrochlorid;
[1-(Benzylamino-methyl)-cyclohexyl]-essigsäure-Hydrochlorid;
((1R,3R)-3-Methyl-1-propylaminomethyl-cyclohexyl)-essigsäure-Hydrochlorid;
{1-[(Cyclopentylmethyl-amino)-methyl]-cyclohexyl}-essigsäure-Hydrochlorid;
{1-[(Cyclohexylmethyl-amino)-methyl]-cyclohexyl}-essigsäure-Hydrochlorid;
[1-(*tert*.-Butoxycarbonylamino-methyl)-cyclohexyl]-essigsäure;
[1-(Acetylamino-methyl)-cyclohexyl]-essigsäure;
((3R,5S)-1-Cyclobutylaminomethyl-3,5-dimethyl-cyclohexyl)-essigsäure-Hydrochlorid;
{(3R,5S)-3,5-Dimethyl-1-[(2-methyl-butylamino)-methyl]-cyclohexyl}-essigsäure-Hydrochlorid;
{(3R,5S)-1-[(2-Hydroxy-1-methyl-ethylamino)-methyl]-3,5-dimethyl-cyclohexyl}-essigsäure-Hydrochlorid;
{(3R,5S)-1-[(2,2-Dimethoxy-ethylamino)-methyl]-3,5-dimethyl-cyclohexyl}-essigsäure-Hydrochlorid;
{(3R,5S)-1-[(Cyclopentylmethyl-amino)-methyt]-3,5-dimethyl-cyclohexyl}-essigsäure-Hydrochlorid;
{(3R,5S)-1-[(Cyclohexylmethyl-amino)-methyl]-3,5-dimethyl-cyclohexyl}-essigsäure-Hydrochlorid;
((3R,5S)-1-Cyclohexylaminomethyl-3,5-dimethyl-cyclohexyl)-essigsäure-Hydrochlorid;
((3R,5S)-1-Carboxymethyl-3,5-dimethyl-cyclohexyl)-essigsäure;
*trans*-((3R,5S)-1-Hydroxymethyl-3,5-dimethyl-cyclohexyl)-essigsäure-Hydrochlorid;
*cis*-((3R,5S)-1-Hydroxymethyl-3,5-dimetyl-cyclohexyl)-essigsäure-Hydrochlorid;
(1-Dimethylaminomethyl-cyclohexyl)-essigsäure-Hydrochlorid;
(1-Butylaminomethyl-cyclohexyl)-essigsäure-Hydrochlorid;
{1-[2,2-Dimethoxy-ethylamino)-methyl]-cyclohexyl}-essigsäure-Hydrochlorid;
(1-Methylaminomethyl-cyclohexyl)-essigsäure-Hydrochlorid;
{1-[(Benzyl-methyl-amino)-methyl]-cyclohexyl}-essigsäure-Hydrochlorid;
[1-(Phenethylamino-methyl)-cyclohexyl]-essigsäure-Hydrochlorid;
{1-[(3-Phenyl-propylamino)-methyl]-cyclohexyl}-essigsäure-Hydrochlorid;
((3R,5S)-1-Hydroxymethyl-3,5-dimethyl-cyclohexyl)-essigsäure-natriumsalz;
((3R,5S)-1-Ethylaminomethyl-3,5-dimethyl-cyclohexyl)-essigsäure-Hydrochlorid;
(1-Aminomethyl-4-ethyl-cyclohexyl)-essigsäure-Hydrochlorid;
(1-Aminomethyl-4-propyl-cyclohexyl)-essigsäure-Hydrochlorid;
((3R,5S)-3,5-Dimethyl-1-propylaminomethyl-cyclohexyl)-essigsäure-Hydrochlorid;
[(1R,3R)-1-(Benzylamino-methyl)-3-methyl-cyclohexyl]-essigsäure-Hydrochlorid;
{(1R,3R)-1-[(Benzyl-methyl-amino)-methyl]-3-methyl-cyclohexyl}-essigsäure-Hydrochlorid oder
((1R,3R)-3-Methyl-1-methylaminomethyl-cyclohexyl)-essigsäure-Hydrochlorid.

10. Verwendung einer Verbindung der Formel I, II und/oder III: 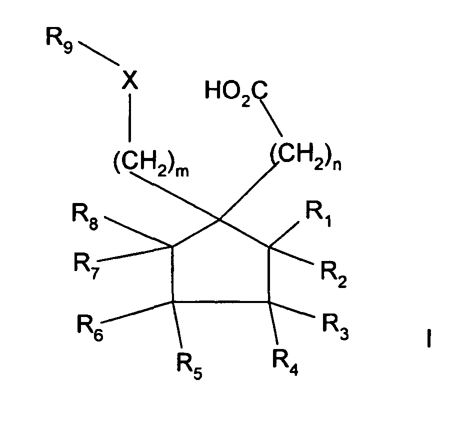 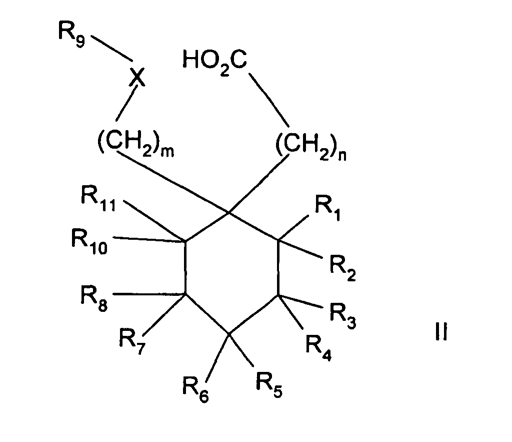 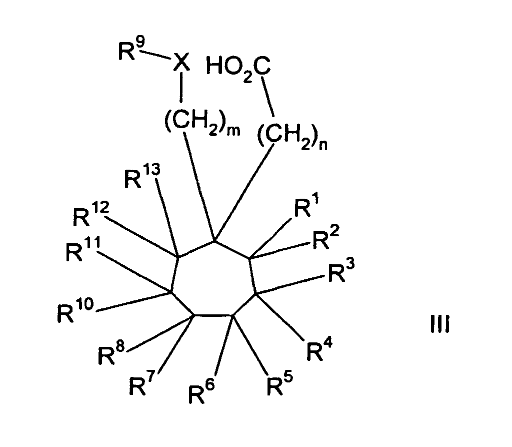 worin
R₉ für Wasserstoff; Alkyl; Cycloalkyl; substituiertes, Halogen, Amin, Alkoxy, Cycloalkyl oder Hydroxy enthaltendes Alkyl; Allyl; Alkinyl; Alkanoyl; Alkoxyalkanoyl; Sulfonyl; Phenyl; Benzyl oder Arylalkyl steht; unter der Bedingung, dass R₉ in Verbindungen der Formel II nicht Wasserstoff oder Alkanoyl bedeutet;
m und n unabhängig eine ganze Zahl von 1 bis 3 bedeuten;
R₁ - R₈ und R₁₀ - R₁₄ unabhängig ausgewählt sind aus Wasserstoff, Alkyl und substituiertem Alkyl und
X für NR₁₄, Sauerstoff oder Schwefel steht,
wobei im Falle, dass mehr als ein Stereoisomer vorhanden ist, jedes chirale Zentrum unabhängig R oder S sein kann;
oder derer pharmazeutisch akzeptablen Salze, Ester, Prodrugs oder Amide,
zur Herstellung eines Arzneimittels zur Inhibierung der verzweigte-Aminosäure-abhängigen Aminotransferase.

11. Verwendung nach Anspruch 10, worin
m und n 1 bedeuten;
X für NR₁₄ steht;
R₉ für Wasserstoff steht;
R₄ für Methyl steht;
R₄ und R₅ für Methyl stehen;
R₈ für Methyl steht;
R₁₀ für Methyl steht;
R₇ und R₈ für Methyl stehen;
R₄ und R₈ für Methyl stehen;
R₁ - R₈ und R₁₀ - R₁₃ für Wasserstoff stehen;
R₉ für Alkyl steht;
R₉ für Benzyl steht;
R₉ für Arylalkyl steht;
R₉ für Cycloalkyl steht;
R₁₄ für Alkyl steht;
R₁ - R₈ für Wasserstoff stehen;
R₁ - R₈ und R₁₀ - R₁₁ für Wasserstoff stehen;
R₁ - R₂ und R₇ - R₈ für Wasserstoff stehen;
oder R₂ für Methyl steht.

12. Verwendung nach Anspruch 10, worin
R₃ für Alkyl steht, R₁ - R₂, R₄- R₁₁ und R₁₄ für Wasserstoff stehen, m und n jeweils 1 bedeuten und X für NR₁₄ steht;
R₃ und R₁₁ für Alkyl stehen, R₁ - R₂, R₄ - R₁₀ und R₁₄ für Wasserstoff stehen, m und n jeweils 1 bedeuten und X für NR₁₄ steht;
R₃ und R₁₁ für Alkyl stehen, R₁ - R₂, R₄- R₁₀ und R₁₄ für Wasserstoff stehen, m und n jeweils 1 bedeuten, Rg für Alkyl steht und X für NR₁₄ steht;
R₁ - R₁₁ und R₁₄ für Wasserstoff stehen, m und n jeweils 1 bedeuten und X für Sauerstoff steht.

13. Verwendung nach Anspruch 10, wobei die Verbindung ausgewählt ist aus:
(1-Allylaminomethyl-cyclohexyl)-essigsäure;
(1-Prop-2-inylaminomethyl-cyclohexyl)-essigsäure;
{1-[(2,2,2-Trifluor-ethylamino)-methyl]-cyclohexyl}-essigsäure;
{1-[(3,3,3-Trifluor-propylamino)-methyl]-cyclohexyl}-essigsäure;
1α,3β,5β-(1-Allylaminomethyl-3,5-dimethyl-cyclohexyl)-essigsäure;
1α,3β,5β-(3,5-Dimethyl-1-prop-2-inylaminomethyl-cyclohexyl)-essigsäure;
1α,3β,5β-{3,5-Dimethyl-1-[(2,2,2-trifluor-ethylamino)-methyl]-cyclohexyl}-essigsäure;
1α,3β,5β-{3,5-Dimethyl-1-[(3,3,3-trifluor-propylamino)-methyl]-cyclohexyl}-essigsäure;
trans-((1R,3R)-1-Allylaminomethyl-3-methyl-cyclohexyl)-essigsäure;
trans-((1R,3R)-3-Methyl-1-prop-2-inylaminomethyl-cyclohexyl)-essigsäure;
trans-{(1R,3R)-3-Methyl-1-[(2,2,2-trifluor-ethylamino)-methyl]-cyclohexyl}-essigsäure;
trans-{(1R,3R)-3-Methyl-1-[(3,3,3-trifluor-propylamino)-methyl]-cyclohexyl}-essigsäure;
trans-{(1R,3R)-3-Methyl-1-[(4,4,4-trifluor-butylamino)-methyl]-cyclohexyl}-essigsäure;
1α,3β,5β-{3,5-Dimethyl-1-[(4,4,4-trifluor-butylamino)-methyl]-cyclohexyl}-essigsäure;
1α,3β,5β-{1-[(Cyclopropylmethyl-amino)-methyl]-3,5-dimethyl-cyclohexyl}-essigsäure;
trans-{(1R,3R)-1-[(Cyclopropylmethyl-amino)-methyl]-3-methyl-cyclohexyl}-essigsäure;
trans-((1R,3R)-3-Methyl-1-methylaminomethyl-cyclohexyl)-essigsäure;
trans-((1R,3R)-1-Ethylaminomethyl-3-methyl-cyclohexyl)-essigsäure;
trans-((1R,3R)-3-Methyl-1-propylaminomethyl-cyclohexyl)-essigsäure;
trans-((1R,3R)-1-Butylaminomethyl-3-methyl-cyclohexyl)-essigsäure;
trans-((1R,3R)-1-Hydroxymethyl-3-methyl-cyclohexyl)-essigsäure;
1α,3β,5β-{1-[(Hydroxymethyl-amino)-methyl]-3,5-dimethyl-cyclohexyl)}-essigsäure;
1α,3β,5β-(1-Aminomethyl-3,5-diethyl-cyclohexyl)}-essigsäure-Hydrochlorid;
trans-(1R,3R)(1-Aminomethyl-3-methyl-cyclohexyl)-essigsäure-Hydrochlorid;
(1-Aminomethyl-2-methyl-cyclohexyl)-essigsäure-Hydrochlorid;
(1-Aminomethyl-3,3-dimethyl-cyclohexyl)-essigsäure-Hydrochlorid;
(±)-(trans)-(1-Aminomethyl-3,4-dimethyl-cyclopentyl)-essigsäure-Hydrochlorid;
(cis/trans)-(3R)-(1-Aminomethyl-3-methyl-cyclopentyl)-essigsäure-Hydrochlorid;
(+)-(trans)-(1-Aminomethyl-3,4-dimethyl-cyclopentyl)-essigsäure-Hydrochlorid;
(+)-(trans)-(1-Aminomethyl-3,4-dimethyl-cyclopentyl)-essigsäure-Hydrochlorid;
1α,3β,5β-(1-Aminomethyl-3,5-dimethyl-cyclohexyl)}-essigsäure-Hydrochlorid;
1α,3β,5β-(3,5-Dimethyl-1-propylaminomethyl-cyclohexyl)}-essigsäure-Hydrochlorid;
1α,3β,5β-(1-Ethylaminomethyl-3,5-dimethyl-cyclohexyl)-essigsäure-Hydrochlorid;
1α,3β,5β-[(1-Benzylamino-methyl)-3,5-dimethyl-cyclohexyl]-essigsäure-Hydrochlorid;
1α,3β,5β-(1-Dimethylaminomethyl-3,5-dimethyl-cyclohexyl)-essigsäure-Hydrochlorid;
1α,3β,5β-(1-Butylaminomethyl-3,5-dimethyl-cyclohexyl)-essigsäure-Hydrochlorid;
1α,3β,5β-{1-[(Benzyl-methyl-amino)-methyl]-3,5-dimethyl-cyclohexyl)-essigsäure-Hydrochlorid;
1α,3β,5β-(3,5-Dimethyl-1-methylaminomethyl-cyclohexyl)-essigsäure-Hydrochlorid;
1α,3β,5β-[1-(Acetylamino-methyl)-3,5-dimethyl-cyclohexyl]-essigsäure;
1α,3β,5β-[1-(Isobutylamino-methyl)-3,5-dimethyl-cyclohexyl]-essigsäure-Hydrochlorid;
1α,3β,5β-[3,5-Dimethyl-1-(phenethylamino-methyl)-cyclohexyl]-essigsäure-Hydrochlorid;
1α,3β,5β-{3,5-Dimethyl-1-[(3-phenyl-propylamino)-methyl]-cyclohexyl}-essigsäure-Hydrochlorid;
{1-[(Cyclobutylmethyl-amino)-methyl]-cyclohexyl}-essigsäure-Hydrochlorid;
1α,3β,5β-[1-(Isopropylamino-methyl)-3,5-dimethyl-cyclohexyl]-essigsäure-Hydrochlorid;
1-Aminomethyl-1-cyclohexan-essigsäure;
1-Aminomethyl-1-cyclopentan-essigsäure;
1-Aminomethyl-1-cyclopentan-essigsäure-Natriumsalz;
1-(Hydroxymethyl)cyclohexan-essigsäure-Natriumsalz;
{1-[(2-Methyl-butylamino)-methyl]-cyclohexyl}-essigsäure-Hydrochlorid;
{1-[(4,4,4-Trifluor-butylamino)-methyl]-cyclohexyl}-essigsäure-Hydrochlorid;
(1-Ethylaminomethyl-cyclohexyl)-essigsäure-Hydrochlorid;
{1-[(Cyclopropylmethyl-amino)-methyl]-cyclohexyl}-essigsäure-Hydrochlorid;
{1-[(2-Hydroxy-1-methyl-ethylamino)-methyl]-cyclohexyl}-essigsäure-Hydrochlorid;
[1-(Isobutylamino-methyl)-cyclohexyl]-essigsäure-Hydrochlorid;
(1-Propylaminomethyl-cyclohexyl)-essigsäure-Hydrochlorid;
[1-(Isopropylamino-methyl)-cyclohexyl]-essigsäure-Hydrochlorid;
(1-Cyclohexylaminomethyl-cyclohexyl)-essigsäure-Hydrochlorid;
[1-(Benzylamino-methyl)-cyclohexyl]-essigsäure-Hydrochlorid;
((1R,3R)-3-Methyl-1-propylaminomethyl-cyclohexyl)-essigsäure-Hydrochlorid;
{1-[(Cyclopentylmethyl-amino)-methyl]-cyclohexyl}-essigsäure-Hydrochlorid;
{1-[(Cyclohexylmethyl-amino)-methyl]-cyclohexyl}-essigsäure-Hydrochlorid;
[1-(*tert*.-Butoxycarbonylamino-methyl)-cyclohexyl]-essigsäure;
[1-(Acetylamino-methyl)-cyclohexyl]-essigsäure;
((3R,5S)-1-Cyclobutylaminomethyl-3,5-dimethyl-cyclohexyl)-essigsäure-Hydrochlorid;
{(3R,5S)-3,5-Dimethyl-1-[(2-methyl-butylamino)-methyl]-cyclohexyl}-essigsäure-Hydrochlorid;
{(3R,5S)-1-[(2-Hydroxy-1-methyl-ethylamino)-methyl]-3,5-dimethyl-cyclohexyl}-essigsäure-Hydrochlorid;
{(3R,5S)-1-[(2,2-Dimethoxy-ethylamino)-methyl]-3,5-dimethyl-cyclohexyl}-essigsäure-Hydrochlorid;
{(3R,5S)-1-[(Cyclopentylmethyl-amino)-methyl]-3,5-dimethyl-cyclohexyl}-essigsäure-Hydrochlorid;
{(3R,5S)-1-[(Cyclohexylmethyl-amino)-methyl]-3,5-dimethyl-cyclohexyl}-essigsäure-Hydrochlorid;
((3R,5S)-1-Cyclohexylaminomethyl-3,5-dimethyl-cyclohexyl)-essigsäure-Hydrochlorid;
((3R,5S)-1-Carboxymethyl-3,5-dimethyl-cyclohexyl)-essigsäure;
*trans*-((3R,5S)-1-Hydroxymethyl-3,5-dimethyl-cyclohexyl)-essigsäure-Hydrochlorid;
*cis*-((3R,5S)-1-Hydroxymethyl-3,5-dimethyl-cyclohexyl)-essigsäure-Hydrochlorid;
(1-Dimethylaminomethyl-cyclohexyl)-essigsäure-Hydrochlorid;
(1-Butylaminomethyl-cyclohexyl)-essigsäure-Hydrochlorid;
{1-[2,2-Dimethoxy-ethylamino)-methyl]-cyclohexyl}-essigsäure-Hydrochlorid;
(1-Methylaminomethyl-cyclohexyl)-essigsäure-Hydrochlorid;
{1-[(Benzyl-methyl-amino)-methyl]-cyclohexyl}-essigsäure-Hydrochlorid;
[1-(Phenethylamino-methyl)-cyclohexyl]-essigsäure-Hydrochlorid;
{1-[(3-Phenyl-propylamino)-methyl]-cyclohexyl}-essigsäure-Hydrochlorid;
((3R,5S)-1-Hydroxymethyl-3,5-dimethyl-cyclohexyl)-essigsäure-Natriumsalz;
((3R,5S)-1-Ethylaminomethyl-3,5-dimethyl-cyclohexyl)-essigsäure-Hydrochlorid;
(1-Aminomethyl-4-ethyl-cyclohexyl)-essigsäure-Hydrochlorid;
(1-Aminomethyl-4-propyl-cyclohexyl)-essigsäure-Hydrochlorid;
((3R,5S)-3,5-Dimethyl-1-propylaminomethyl-cyclohexyl)-essigsäure-Hydrochlorid;
[(1R,3R)-1-(Benzylamino-methyl)-3-methyl-cyclohexyl]-essigsäure-Hydrochlorid;
{(1R,3R)-1-[(Benzyl-methyl-amino)-methyl]-3-methyl-cyclohexyl}-essigsäure-Hydrochlorid oder
((1R,3R)-3-Methyl-1-methylaminomethyl-cyclohexyl)-essigsäure-Hydrochlorid.

14. Verwendung einer Verbindung der Formel IV gemäß Anspruch 1 zur Herstellung eines Arzneimittels zur Behandlung von neurologischen Störungen, Depression, Angst, Panik, Manien, bipolaren Störungen, Entzündungskrankheiten, Glaucom, Schmerzen oder Magen-Darm-Störungen.

## Revendications

1. Composé répondant à la formule IV 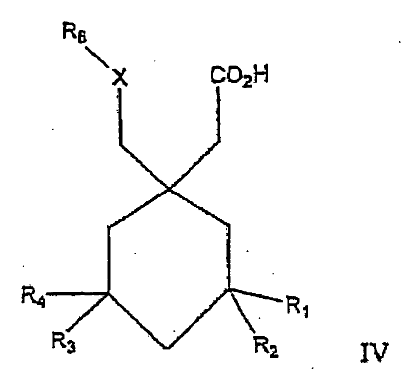 R₁ -R₄ représentent un atome d'hydrogène ou un groupe alkyle ;
X représente un groupe NR₅ ou un atome d'oxygène ;
R₅ représente un atome d'hydrogène ou un groupe alkyle ;
R₆ représente un groupe alkyle, un groupe benzyle, un groupe alcoxyalcanoyle, un groupe arylalkyle, un groupe alcoxy, un groupe cycloalkyle, un groupe allyle, un groupe alkylcycloalkyle, un groupe halogénoalkyle trisubstitué; et dans lequel lorsque R₁-R₄ représentent respectivement un atome d'hydrogène, le radical R₆ ne représente pas un atome d'hydrogène ou un groupe méthyle ; ou un de ses sels, de ses esters, de ses promédicaments ou de ses amides pharmaceutiquement acceptables.

2. Composé selon la revendication 1, dans lequel
R₂ et R₄ représentent un atome d'hydrogène et R₁ et R₃ représentent un groupe alkyle ;
R₂ et R₄ représentent un atome d'hydrogène et R₁ et R₃ représentent un groupe méthyle ;
R₁ - R₄ représentent un atome d'hydrogène ;
R₁ représente un groupe alkyle et R₂ - R₄ représentent un atome d'hydrogène ;
R₁ représente un groupe méthyle et R₂ - R₄ représentent un atome d'hydrogène ;
R₅ représente un atome d'hydrogène ;
X représente un atome d'oxygène ;
R₆ représente un groupe alkyle ;
R₆ représente un groupe benzyle ;
R₆ représente un groupe phénylalkyle ;
R₆ représente un groupe cycloalkyle ;
R₆ représente un groupe trifluoroalkyle ;
R₆ représente un groupe alkylcycloalkyle ;
R₆ représente un groupe alcoxy ;
R₆ représente un groupe allyle.

3. Composé selon la revendication 1, dans lequel
R₂ et R₄ représentent un atome d'hydrogène et R₁ et R₃ représentent un groupe méthyle ;
R₁-R₄ représentent un atome d'hydrogène ;
R₁ représente un groupe méthyle et R₂-R₄ représentent un atome d'hydrogène ;
R₅ représente un atome d'hydrogène ;
R₆ représente un groupe alkyle ;
R₆ représente un groupe benzyle ;
R₆ représente un groupe phénylalkyle ;
R₆ représente un groupe cycloalkyle ;
R₆ représente un groupe trifluoroalkyle ;
R₆ représente un groupe alkylcycloalkyle ;
R₆ représente un groupe alcoxy ;
R₆ représente un groupe allyle.

4. Composé selon la revendication 1, dans lequel
R₂ et R₄ représentent un atome d'hydrogène et R₁ et R₃ représentent un groupe méthyle ;
R₁-R₄ représentent un atome d'hydrogène.

5. Composé selon la revendication 1 et choisi parmi le groupe constitué par:
l'acide (1-allylaminométhyl-cyclohexyl)-acétique ;
l'acide (1-prop-2-ynylaminométhyl-cyclohexyl)-acétique ;
l'acide {1-[(2,2,2-trifluoro-éthylamino)-méthyl]-cyclohexyl}-acétique ;
l'acide {1-[(3,3,3-trifluoro-propylamino)-méthyl]-cyclohexyl}-acétique ;
l'acide 1α,3β,5β-(1-allylaminométhyl-3,5-diméthyl-cyclohexyl)-acétique ;
l'acide 1α,3β,5β-(3,5-diméthyl-1-prop-2-ynylaminométhyl-cyclohexyl)-acétique ;
l'acide 1α,3β,5β-{3,5-diméthyl-1-[(2,2,2-trifluoro-éthylamino)-méthyl]-cyclohexyl}-acétique ;
l'acide 1α,3β,5β-{3,5-diméthyl-1-[(3,3,3-trifluoro-propylamino)-méthyl]-cyclohexyl}-acétique ;
l'acide trans-((1R,3R)-1-allylaminométhyl-3-méthyl-cyclohexyl)-acétique ;
l'acide trans-((1R,3R)-3-méthyl-1-prop-2-ynylaminométhyl-cyclohexyl)-acétique ;
l'acide trans-{(1R,3R)-3-méthyl-1-[(2,2,2-trifluoro-éthylamino)-méthyl]-cyclohexyl}-acétique ;
l'acide trans-{(1R,3R)-3-méthyl-1-[(3,3,3-trifluoro-propylamino)-méthyl]-cyclohexyl}-acétique ;
l'acide trans-{(1R,3R)-3-méthyl-1-[(4,4,4-trifluoro-butylamino)-méthyl]-cyclohexyl}-acétique ;
l'acide 1α,3β,5β-{3,5-diméthyl-1-[(4,4,4-trifluoro-butylamino)-méthyl]-cyclohexyl}-acétique ;
l'acide 1α,3β,5β-{1-[(cyclopropylméthyl-amino)-méthyl]-3,5-diméthyl-cyclohexyl}-acétique ;
l'acide trans-{(1R,3R)-1-[(cyclopropylméthyl-amino)-méthyl]-3-méthyl-cyclohexyl}-acétique ;
l'acide trans-((1R,3R)-3-méthyl-1-méthylaminométhyl-cyclohexyl)-acétique ;
l'acide trans-((1R,3R)-1-éthylaminométhyl-3-méthyl-cyclohexyl)-acétique ;
l'acide trans-((1R,3R)-3-méthyl-1-propylaminométhyl-cyclohexyl)-acétique ;
l'acide trans-((1R,3R)-1-butylaminométhyl-3-méthyl-cyclohexyl)-acétique ;
l'acide 1α,3β,5β-(3,5-diméthyl-1-propylaminométhyl-cyclohexyl)-acétique, sel chlorhydrate ;
l'acide 1α,3β,5β-(1-éthylaminométhyl-3,5-diméthyl-cyclohexyl)-acétique, sel chlorhydrate ;
l'acide 1α,3β,5β-[(1-benzylamino-méthyl)-3,5-diméthyl-cyclohexyl]-acétique, sel chlorhydrate ;
l'acide 1α,3β,5β-(1-diméthylaminométhyl-3,5-diméthyl-cyclohexyl)-acétique, sel chlorhydrate ;
l'acide 1α,3β,5β-(1-butylaminométhyl-3,5-diméthyl-cyclohexyl)-acétique, sel chlorhydrate ;
l'acide 1α,3β,5β-{1-[(benzyl-méthyl-amino)-méthyl]-3,5-diméthyl-cyclohexyl}-acétique, sel chlorhydrate ;
l'acide 1α,3β,5β-(3,5-diméthyl-1-méthylaminométhyl-cyclohexyl)-acétique, sel chlorhydrate ;
l'acide 1α,3β,5β-[1-(isobutylamino-méthyl)-3,5-diméthyl-cyclohexyl]-acétique, sel chlorhydrate ;
l'acide 1α,3β,5β-[3,5-diméthyl-1-(phénéthylamino-méthyl)-cyclohexyl]-acétique, sel chlorhydrate ;
l'acide 1α,3β,5β-{3,5-diméthyl-1-[(3-phényl-propylamino)-méthyl]-cyclohexyl}-acétique, sel chlorhydrate ;
l'acide {1-[(cyclobutylméthyl-amino)-méthyl]-cyclohexyl]-acétique, sel chlorhydrate ;
l'acide 1α,3β,5β-1-[(isopropylamino-méthyl)-3,5-diméthyl-cyclohexyl]-acétique, sel chlorhydrate ;
l'acide {1-[(2-méthyl-butylamino)-méthyl]-cyclohexyl}-acétique, sel chlorhydrate ;
l'acide {1-[(4,4,4-trifluoro-butylamino)-méthyl]-cyclohexyl}-acétique, sel chlorhydrate ;
l'acide (1-éthylaminométhyl-cyclohexyl)-acétique, sel chlorhydrate ;
l'acide {1-[(cyclopropylméthyl-amino)-méthyl]-cyclohexyl}-acétique, sel chlorhydrate ;
l'acide [1-(isobutylamino-méthyl)-cyclohexyl]-acétique, sel chlorhydrate ;
l'acide (1-propylaminométhyl-cyclohexyl)-acétique, sel chlorhydrate ;
l'acide [1-(isopropylamino-méthyl)-cyclohexyl]-acétique, sel chlorhydrate ;
l'acide (1-cyclohexylaminométhyl-cyclohexyl)-acétique, sel chlorhydrate ;
l'acide [1-(benzylamino-méthyl)-cyclohexyl]-acétique, sel chlorhydrate ;
l'acide ((1R,3R)-3-méthyl-1-propylaminométhyl-cyclohexyl)-acétique, sel chlorhydrate ;
l'acide {1-[(cyclopentylméthyl-amino)-méthyl]-cyclohexyl}-acétique, sel chlorhydrate ;
l'acide {1-[(cyclohexylméthyl-amino)-méthyl]-cyclohexyl}-acétique, sel chlorhydrate ;
l'acide [1-(*tert*-butoxycarbonylamino-méthyl)-cyclohexyl]-acétique ;
l'acide ((3R,5S)-1-cyclobutylaminométhyl-3,5-diméthyl-cyclohexyl)-acétique, sel chlorhydrate ;
l'acide {(3R,5S)-3,5-diméthyl-1-[(2-méthyl-butylamino)-méthyl]-cyclohexyl}-acétique, sel chlorhydrate ;
l'acide {(3R,5S)-1-[(2,2-diméthoxy-éthylamino)-méthyl]-3,5-diméthyl-cyclohexyl}-acétique, sel chlorhydrate ;
l'acide {(3R,5S)-1-[(cyclopentylméthyl-amino)-méthyl]-3,5-diméthyl-cyclohexyl}-acétique, sel chlorhydrate ;
l'acide {(3R,5S)-1-[(cyclohexylméthyl-amino)-méthyl]-3,5-diméthyl-cyclohexyl}-acétique, sel chlorhydrate ;
l'acide ((3R,5S)-1-cyclohexylaminométhyl-3,5-diméthyl-cyclohexyl)-acétique, sel chlorhydrate ;
l'acide (1-diméthylaminométhyl-cyclohexyl)-acétique, sel chlorhydrate ;
l'acide (1-butylaminométhyl-cyclohexyl)-acétique, sel chlorhydrate ;
l'acide {1-[(2,2-diméthoxy-éthylamino)-méthyl]-cyclohexyl}-acétique, sel chlorhydrate ;
l'acide (1-méthylaminométhyl-cyclohexyl)-acétique, sel chlorhydrate ;
l'acide {1-[(benzyl-méthyl-amino)-méthyl]-cyclohexyl}-acétique, sel chlorhydrate ;
l'acide [1-(phénéthylamirio-méthyl)-cyclohexyl]-acétique, sel chlorhydrate ;
l'acide {1-[(3-phényl-propylamino)-méthyl]-cyclohexyl}-acétique, sel chlorhydrate ;
l'acide ((3R,5S)-1-hydroxyméthyl-3,5-diméthyl-cyclohexyl)-acétique, sel de sodium ;
l'acide ((3R,5S)-1-éthylaminométhyl-3,5-diméthyl-cyclohexyl)-acétique, sel chlorhydrate ;
l'acide ((3R,5S)-3,5-diméthyl-1-propylaminométhyl-cyclohexyl)-acétique, sel chlorhydrate ;
l'acide [(1R,3R)-1-(benzylamino-méthyl)-3-méthyl-cyclohexyl]-acétique, sel chlorhydrate ;
l'acide {(1R,3R)-1-[(benzyl-méthyl-amino)-méthyl]-3-méthyl-cyclohexyl}-acétique, sel chlorhydrate ;
ou
l'acide ((1R,3R)-3-méthyl-1-méthylaminométhyl-cyclohexyl)-acétique, sel chlorhydrate.

6. Utilisation d'un composé répondant aux formules I, II et/ou III : 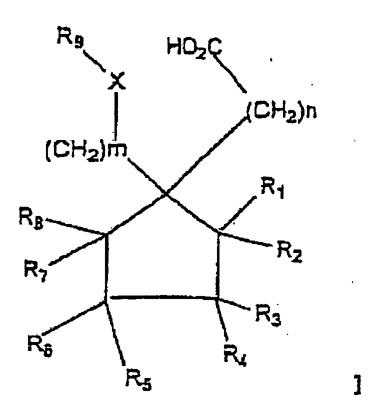 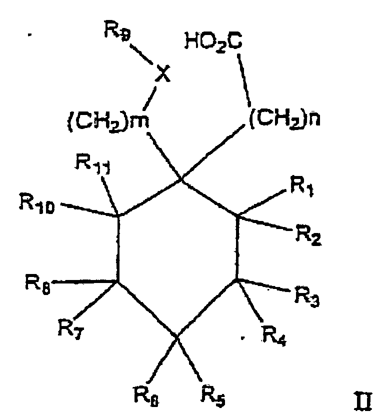 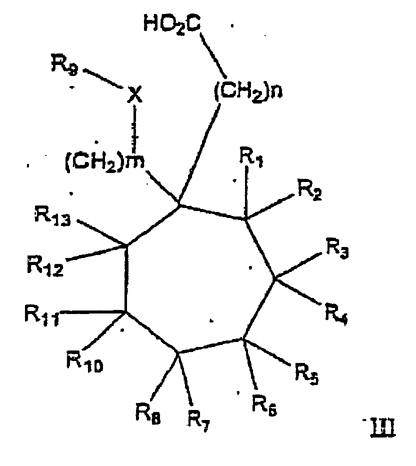 dans lesquelles
R₉ représente un atome d'hydrogène ; un groupe alkyle ; un groupe cycloalkyle ; un groupe alkyle substitué contenant un atome d'halogène, un groupe amine, un groupe alcoxy, un groupe cycloalkyle ou un groupe hydroxyle ; un groupe allyle ; un groupe alcynyle ; un groupe alcanoyle ; un groupe alcoxyalcanoyle ; un groupe sulfonyle ; un groupe phényle ; un groupe benzyle ; ou un groupe arylalkyle ;
m et n représentent, indépendamment l'un de l'autre, un entier de 1-3 ;
R₁ - R₈ et R₁₀ - R₁₄ représentent indépendamment les uns des autres un atome d'hydrogène, un groupe alkyle, ou un groupe alkyle substitué ; et
X représente un groupe NR₁₄, un atome d'oxygène ou un atome de soufre ;
lorsqu'il y a plus d'un stéréo-isomère, chaque centre chiral peut représenter, de manière indépendante, la configuration R ou la configuration S ; ou d'un de ses sels, de ses esters, de ses promédicaments ou de ses amides pharmaceutiquement acceptables
pour la préparation d'un produit pharmaceutique pour le traitement de la rétinopathie diabétique.

7. Utilisation selon la revendication 6, dans laquelle
m et n sont égaux à 1 ;
X représente un groupe NR₁₄ ;
R₉ représente un atome d'hydrogène ;
R₄ représente un groupe méthyle ;
R₄ et R₅ représentent un groupe méthyle ;
R₈ représente un groupe méthyle ;
R₁₀ représente un groupe méthyle ;
R₇ et R₈ représentent un groupe méthyle ;
R₄ et R₈ représentent un groupe méthyle ;
R₁ - R₈ et R₁₀ - R₁₃ représentent un atome d'hydrogène ;
Rg représente un groupe alkyle ;
R₉ représente un groupe benzyle ;
R₉ représente un groupe arylalkyle ;
R₉ représente un groupe cycloalkyle ;
R₁₄ représente un groupe alkyle ;
R₁-R₈ représentent un atome d'hydrogène ;
R₁ - R₈ et R₁₀ - R₁₁ représentent un atome d'hydrogène ;
R₁ - R₂ et R₇ - R₈ représentent un atome d'hydrogène ;
ou bien R₂ représente un groupe méthyle.

8. Utilisation selon la revendication 6, dans laquelle
R₃ représente un groupe alkyle, R₁ - R₂ et R₄ - R₁₁ et R₁₄ représentent un atome d'hydrogène et m et n sont égaux à 1, et X représente un groupe NR₁₄ ;
R₃ et R₁₁ représentent un groupe alkyle, R₁ - R₂ et R₄ - R₁₀ et R₁₄ représentent un atome d'hydrogène, m et n sont égaux à 1, et X représente un groupe NR₁₄ ;
R₃ et R₁₁ représentent un groupe alkyle, R₁ - R₂ et R₄ - R₁₀ et R₁₄ représentent un atome d'hydrogène, m et n sont égaux à 1, Rg représente un groupe alkyle, et X représente un groupe NR₁₄ ;
R₁ - R₁₁ et R₁₄ représentent un atome d'hydrogène, m et n sont égaux à 1, et X représente un atome d'oxygène.

9. Utilisation selon la revendication 6, dans laquelle le composé est choisi parmi le groupe constitué par :
l'acide (1-allylaminométhyl-cyclohexyl)-acétique ;
l'acide (1-prop-2-ynylaminométhyl-cyclohexyl)-acétique ;
l'acide {1-[(2,2,2-trifluoro-éthylamino)-méthyl]-cyclohexyl}-acétique ;
l'acide {1-[(3,3,3-trifluoro-propylamino)-méthyl]-cyclohexyl}-acétique ;
l'acide 1α,3β,5β-(1-allylaminométhyl-3,5-diméthyl-cyclohexyl)-acétique ;
l'acide 1α,3β,5β-(3,5-diméthyl-1-prop-2-ynylaminométhyl-cyclohexyl)-acétique ;
l'acide 1α,3β,5β-{3,5-diméthyl-1-[(2,2,2-trifluoro-éthylamino)-méthyl]-cyclohexyl}-acétique ;
l'acide 1α,3β,5β-{3,5-diméthyl-1-[(3,3,3-trifluoro-propylamino)-méthyl]-cyclohexyl}-acétique ;
l'acide trans-((1R,3R)-1-allylaminométhyl-3-méthyl-cyclohexyl)-acétique ;
l'acide trans-((1R,3R)-3-méthyl-1-prop-2-ynylaminométhyl-cyclohexyl)-acétique ;
l'acide trans-{(1R,3R)-3-méthyl-1-[(2,2,2-trifluoro-éthylamino)-méthyl]-cyclohexyl}-acétique ;
l'acide trans-{(1R,3R)-3-méthyl-1-[(3,3,3-trifluoro-propylamino)-méthyl]-cyclohexyl}-acétique ;
l'acide trans-{(1R,3R)-3-méthyl-1-[(4,4,4-trifluoro-butylamino)-méthyl]-cyclohexyl}-acétique ;
l'acide 1α,3β,5β-{3,5-diméthyl-1-[(4,4,4-trifluoro-butylamino)-méthyl]-cyclohexyl}-acétique ;
l'acide 1α,3β,5β-{1-[(cyclopropylméthyl-amino)-méthyl]-3,5-diméthyl-cyclohexyl}-acétique ;
l'acide trans-{(1R,3R)-1-[(cyclopropylméthyl-amino)-méthyl]-3-méthyl-cyclohexyl}-acétique ;
l'acide trans-((1R,3R)-3-méthyl-1-méthylaminométhyl-cyclohexyl)-acétique ;
l'acide trans-((1R,3R)-1-éthylaminométhyl-3-méthyl-cyclohexyl)-acétique ;
l'acide trans-((1R,3R)-3-méthyl-1-propylaminométhyl-cyclohexyl)-acétique ;
l'acide trans-((1R,3R)-1-butylaminométhyl-3-méthyl-cyclohexyl)-acétique ;
l'acide trans-((1R,3R)-1-hydroxyméthyl-3-méthyl-cyclohexyl)-acétique ;
l'acide 1α,3β,5β-{1-[(hydroxyméthyl-amino)-méthyl]-3,5-diméthyl-cyclohexyl}-acétique ;
l'acide 1α,3β,5β-(1-aminométhyl-3,5-diéthyl-cyclohexyl)-acétique, chlorhydrate ;
l'acide trans-(1R,3R)-(1-aminométhyl-3-méthyl-cyclohexyl)-acétique, chlorhydrate ;
l'acide (1-aminométhyl-2-méthyl-cyclohexyl)-acétique, chlorhydrate ;
l'acide (1-aminométhyl-3,3-diméthyl-cyclohexyl)-acétique, chlorhydrate ;
l'acide (±)-(trans)-(1-aminométhyl-3,4-diméthyl-cyclopentyl)-acétique, chlorhydrate ;
l'acide (cis/trans)-(3R)-(1-aminométhyl-3-méthyl-cyclopentyl)-acétique, chlorhydrate ;
l'acide (+)-(trans)-(1-aminométhyl-3,4-diméthyl-cyclopentyl)-acétique, chlorhydrate ;
l'acide (+)-(trans)-(1-aminométhyl-3,4-diméthyl-cyclopentyl)-acétique, chlorhydrate ;
l'acide 1α,3β,5β-(1-aminométhyl-3,5-diméthyl-cyclohexyl)-acétique, chlorhydrate ;
l'acide 1α,3β,5β-(3,5-diméthyl-1-propylaminométhyl-cyclohexyl)-acétique, sel chlorhydrate ;
l'acide 1α,3β,5β-(1-éthylaminométhyl-3,5-diméthyl-cyclohexyl)-acétique, sel chlorhydrate ;
l'acide 1α,3β,5β-[(1-benzylamino-méthyl)-3,5-diméthyl-cyclohexyl]-acétique, sel chlorhydrate ;
l'acide 1α,3β,5β-(1-diméthylaminométhyl-3,5-diméthyl-cyclohexyl)-acétique, sel chlorhydrate ;
l'acide 1α,3β,5β-(1-butylaminométhyl-3,5-diméthyl-cyclohexyl)-acétique, sel chlorhydrate ;
l'acide 1α,3β,5β-{1-[(benzyl-méthyl-amino)-méthyl]-3,5-diméthyl-cyclohexyl}-acétique, sel chlorhydrate ;
l'acide 1α,3β,5β-(3,5-diméthyl-1-méthylaminométhyl-cyclohexyl)-acétique, sel chlorhydrate ;
l'acide 1α,3β,5β-[1-(acétylamino-méthyl)-3,5-diméthyl-cyclohexyl]-acétique ;
l'acide 1α,3β,5β-[1-(isobutylamino-méthyl)-3,5-diméthyl-cyclohexyl]-acétique, sel chlorhydrate ;
l'acide 1α,3β,5β-[3,5-diméthyl-1-(phénéthylamino-méthyl)-cyclohexyl]-acétique, sel chlorhydrate ;
l'acide 1α,3β,5β-{3,5-diméthyl-1-[(3-phényl-propylamino)-méthyl]-cyclohexyl}-acétique, sel chlorhydrate ;
l'acide {1-[(cyclobutylméthyl-amino)-méthyl]-cyclohexyl]-acétique, sel chlorhydrate ;
l'acide 1α,3β,5β-[1-(isopropylamino-méthyl)-3,5-diméthyl-cyclohexyl]-acétique, sel chlorhydrate ;
l'acide 1-aminométhyl-1-cyclohexane-acétique ;
l'acide 1-aminométhyl-1-cyclopentane-acétique ;
l'acide 1-aminométhyl-1'-cyclopentane-acétique, sel de sodium ;
l'acide 1-(hydroxyméthyl)cyclohexane-acétique, sel de sodium ;
l'acide {1-[(2-méthyl-butylamino)-méthyl]-cyclohexyl}-acétique, sel chlorhydrate ;
l'acide {1-[(4,4,4-trifluoro-butylamino)-méthyl]-cyclohexyl}-acétique, sel chlorhydrate ;
acide (1-éthylaminométhyl-cyclohexyl)-acétique, sel chlorhydrate ;
l'acide {1-[(cyclopropylméthyl-amino)-méthyl]-cyclohexyl}-acétique, sel chlorhydrate ;
l'acide {1-[(2-hydroxy-1-méthyl-éthylamino)-méthyl]-cyclohexyl}-acétique, sel chlorhydrate ;
l'acide [1-(isobutylamino-méthyl)-cyclohexyl]-acétique, sel chlorhydrate ;
l'acide (1-propylaminométhyl-cyclohexyl)-acétique, sel chlorhydrate ;
l'acide [1-(isopropylamino-méthyl)-cyclohexyl]-acétique, sel chlorhydrate ;
l'acide (1-cyclohexylaminométhyl-cyclohexyl)-acétique, sel chlorhydrate ;
l'acide [1-(benzylamino-méthyl)-cyclohexyl]-acétique, sel chlorhydrate ;
l'acide ((1R,3R)-3-méthyl-1-propylaminométhyl-cyclohexyl)-acétique, sel chlorhydrate ;
l'acide {1-[(cyclopentylméthyl-amino)-méthyl]-cyclohexyl}-acétique, sel chlorhydrate ;
l'acide {1-[(cyclohexylméthyl-amino)-méthyl]-cyclohexyl}-acétique, sel chlorhydrate ;
l'acide [1-(*tert*-butoxycarbonylamino-méthyl)-cyclohexyl]-acétique ;
l'acide [1-(acétylamino-méthyl)-cyclohexyl]-acétique ;
l'acide ((3R,5S)-1-cyclobutylaminométhyl-3,5-diméthyl-cyclohexyl)-acétique, sel chlorhydrate ;
l'acide {(3R,5S)-3,5-diméthyl-1-[(2-méthyl-butylamino)-méthyl]-cyclohexyl}-acétique, sel chlorhydrate ;
l'acide {(3R,5S)-1-[(2-hydroxy-1-méthyl-éthylamino)-méthyl]-3,5-diméthyl-cyclohexyl}-acétique, sel chlorhydrate ;
l'acide {(3R,5S)-1-[(2,2-diméthoxy-éthylamino)-méthyl]-3,5-diméthyl-cyclohexyl}-acétique, sel chlorhydrate ;
l'acide {(3R,5S)-1-[(cyclopentylméthyl-amino)-méthyl]-3,5-diméthyl-cyclohexyl}-acétique, sel chlorhydrate ;
l'acide {(3R,5S)-1-[(cyclohexylméthyl-amino)-méthyl]-3,5-diméthyl-cyclohexyl}-acétique, sel chlorhydrate ;
l'acide ((3R,5S)-1-cyclohexylaminométhyl-3,5-diméthyl-cyclohexyl)-acétique, sel chlorhydrate ;
l'acide ((3R,5S)-1-carboxyméthyl-3,5-diméthyl-cyclohexyl)-acétique,
l'acide *trans*-((3R,5S)-1-hydroxyméthyl-3,5-diméthyl-cyclohexyl)-acétique, sel chlorhydrate ;
l'acide *cis*-((3R,5S)-1-hydroxyméthyl-3,5-diméthyl-cyclohexyl)-acétique, sel chlorhydrate ;
l'acide (1-diméthylaminométhyl-cyclohexyl)-acétique, sel chlorhydrate ;
l'acide (1-butylaminométhyl-cyclohexyl)-acétique, sel chlorhydrate ;
l'acide {1-[(2,2-diméthoxy-éthylamino)-méthyl]-cyclohexyl}-acétique, sel chlorhydrate ;
l'acide (1-méthylaminométhyl-cyclohexyl)-acétique, sel chlorhydrate ;
l'acide {1-[(benzyl-méthyl-amino)-méthyl]-cyclohexyl}-acétique, sel chlorhydrate ;
l'acide [1-(phénéthylamino-méthyl)-cyclohexyl]-acétique, sel chlorhydrate ;
l'acide {1-[(3-phényl-propylamino)-méthyl]-cyclohexyl)-acétique, sel chlorhydrate ;
l'acide ((3R,5S)-1-hydroxyméthyl-3,5-diméthyl-cyclohexyl)-acétique, sel de sodium ;
l'acide ((3R,5S)-1-éthylaminométhyl-3,5-diméthyl-cyclohexyl)-acétique, sel chlorhydrate ;
l'acide (1-aminométhyl-4-éthyl-cyclohexyl)-acétique, sel chlorhydrate ;
l'acide (1-aminométhyl-4-propyl-cyclohexyl)-acétique, sel chlorhydrate ;
l'acide ((3R,5S)-3,5-diméthyl-1-propylaminométhyl-cyclohexyl)-acétique, sel chlorhydrate ;
l'acide [(1R,3R)-1-(benzylamino-méthyl)-3-méthyl-cyclohexyl]-acétique, sel chlorhydrate ;
l'acide {(1R,3R)-1-[(benzyl-méthyl-amino)-méthyl]-3-méthyl-cyclohexyl}-acétique, sel chlorhydrate ;
ou
l'acide ((1R,3R)-3-méthyl-1-méthylaminométhyl-cyclohexyl)-acétique, sel chlorhydrate.

10. Utilisation d'un composé répondant aux formules I, II et/ou III : 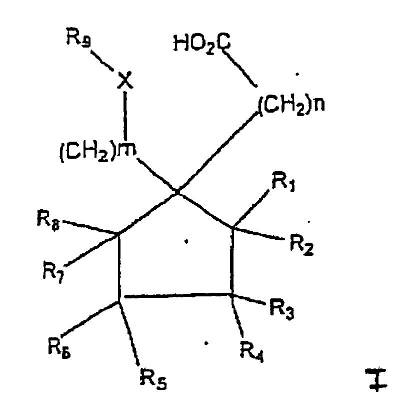 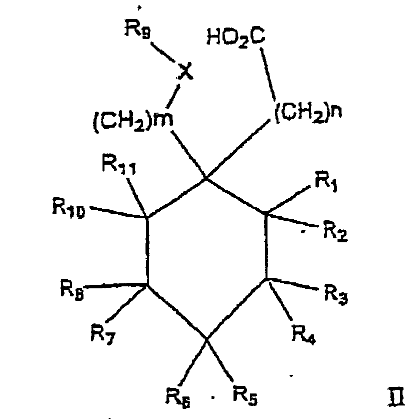 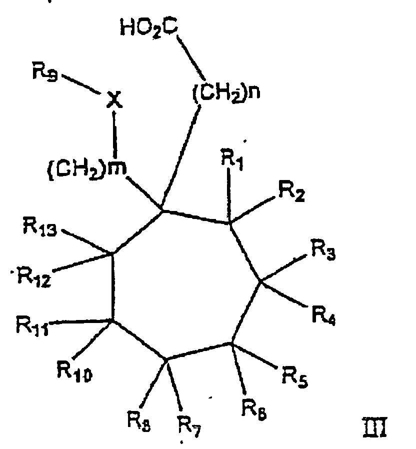 dans lesquelles
R₉ représente un atome d'hydrogène ; un groupe alkyle ; un groupe cycloalkyle ; un groupe alkyle substitué contenant un atome d'halogène, un groupe amine, un groupe alcoxy, un groupe cycloalkyle ou un groupe hydroxyle ; un groupe allyle ; un groupe alcynyle ; un groupe alcanoyle ; un groupe alcoxyalcanoyle ; un groupe sulfonyle ; un groupe phényle ; un groupe benzyle ; ou un groupe arylalkyle ;
et avec cette réserve que R₉ ne peut représenter un atome d'hydrogène ou un groupe alcanoyle pour des composés répondant à la formule II ;
m et n représentent, indépendamment l'un de l'autre, un entier de 1-3 ;
R₁ - R₈ et R₁₀ - R₁₄ représentent indépendamment les uns des autres un atome d'hydrogène, un groupe alkyle, ou un groupe alkyle substitué ; et
X représente un groupe NR₁₄, un atome d'oxygène ou un atome de soufre ;
lorsqu'il y a plus d'un stéréo-isomère, chaque centre chiral peut présenter, de manière indépendante, la configuration R ou la configuration S ; ou d'un de ses sels, de ses esters, de ses promédicaments ou de ses amides pharmaceutiquement acceptables
pour la préparation d'un produit pharmaceutique destiné à inhiber l'aminotransférase dépendant d'acides aminés à chaînes ramifiées.

11. Utilisation selon la revendication 10, dans laquelle
m et n sont égaux à 1 ;
X représente un groupe NR₁₄ ;
R₉ représente un atome d'hydrogène ;
R₄ représente un groupe méthyle ;
R₄ et R₅ représentent un groupe méthyle ;
R₈ représente un groupe méthyle ;
R₁₀ représente un groupe méthyle ;
R₇ et R₈ représentent un groupe méthyle ;
R₄ et R₈ représentent un groupe méthyle ;
R₁ - R₈ et R₁₀ - R₁₃ représentent un atome d'hydrogène ;
R₉ représente un groupe alkyle ;
R₉ représente un groupe benzyle ;
R₉ représente un groupe arylalkyle ;
R₉ représente un groupe cycloalkyle ;
R₁₄ représente un groupe alkyle ;
R₁-R₈ représentent un atome d'hydrogène ;
R₁ - R₈ et R₁₀ - R₁₁ représentent un atome d'hydrogène ;
R₁ - R₂ et R₇ - R₈ représentent un atome d'hydrogène ;
ou bien R₂ représente un groupe méthyle.

12. Utilisation selon la revendication 10, dans laquelle
R₃ représente un groupe alkyle, R₁ - R₂ et R₄ - R₁₁ et R₁₄ représentent un atome d'hydrogène et m et n sont égaux à 1, et X représente un groupe NR₁₄ ;
R₃ et R₁₁ représentent un groupe alkyle, R₁ - R₂ et R₄ - R₁₀ et R₁₄ représentent un atome d'hydrogène, m et n sont égaux à 1, et X représente un groupe NR₁₄ ;
R₃ et R₁₁ représentent un groupe alkyle, R₁ - R₂ et R₄ - R₁₀ et R₁₄ représentent un atome d'hydrogène, m et n sont égaux à 1, R₉ représente un groupe alkyle, et X représente un groupe NR₁₄ ;
R₁ - R₁₁ et R₁₄ représentent un atome d'hydrogène, m et n sont égaux à 1, et X représente un atome d'oxygène.

13. Utilisation selon la revendication 10, dans laquelle le composé est choisi parmi le groupe constitué par :
l'acide (1-allylaminométhyl-cyclohexyl)-acétique ;
l'acide (1-prop-2-ynylaminométhyl-cyclohexyl)-acétique ;
l'acide {1-[(2,2,2-trifluoro-éthylamino)-méthyl]-cyclohexyl}-acétique ;
l'acide {1-[(3,3,3-trifluoro-propylamino)-méthyl]-cyclohexyl}-acétique ;
l'acide 1α,3β,5β-(1-allylaminométhyl-3,5-diméthyl-cyclohexyl)-acétique ;
l'acide 1α,3β,5β-(3,5-diméthyl-1-prop-2-ynylaminométhyl-cyclohexyl)-acétique ;
l'acide 1α,3β,5β-{3,5-diméthyl-1-[(2,2,2-trifluoro-éthylamino)-méthyl]-cyclohexyl}-acétique ;
l'acide 1α,3β,5β-{3,5-diméthyl-1-[(3,3,3-trifluoro-propylamino)-méthyl]-cyclohexyl}-acétique ;
l'acide trans-((1R,3R)-1-allylaminométhyl-3-méthyl-cyclohexyl)-acétique ;
l'acide trans-((1R,3R)-3-méthyl-1-prop-2-ynylaminométhyl-cyclohexyl)-acétique ;
l'acide trans-{(1R,3R)-3-méthyl-1-[(2,2,2-trifluoro-éthylamino)-méthyl]-cyclohexyl}-acétique ;
l'acide trans-{(1R,3R)-3-méthyl-1-[(3,3,3-trifluoro-propylamino)-méthyl]-cyclohexyl}-acétique ;
l'acide trans-{(1R,3R)-3-méthyl-1-[(4,4,4-trifluoro-butylamino)-méthyl]-cyclohexyl}-acétique ;
l'acide 1α,3β,5β-{3,5-diméthyl-1-[(4,4,4-trifluoro-butylamino)-méthyl]-cyclohexyl}-acétique ;
l'acide 1α,3β,5β-{1-[(cyclopropylméthyl-amino)-méthyl]-3,5-diméthyl-cyclohexyl}-acétique ;
l'acide trans-{(1R,3R)-1-[(cyclopropylméthyl-amino)-méthyl]-3-méthyl-cyclohexyl}-acétique ;
l'acide trans-((1R,3R)-3-méthy)-1-méthylaminométhyl-cyclohexyl)-acétique ;
l'acide trans-((1R,3R)-1-éthylaminométhyl-3-méthyl-cyclohexyl)-acétique ;
l'acide trans-((1R,3R)-3-méthyl-1-propylaminométhyl-cyclohexyl)-acétique ;
l'acide trans-((1R,3R)-1-butylaminométhyl-3-méthyl-cyclohexyl)-acétique ;
l'acide trans-((1R,3R)-1-hydroxyméthyl-3-méthyl-cyclohexyl)-acétique ;
l'acide 1α,3β,5β-{1-[(hydroxyméthyl-amino)-méthyl]-3,5-diméthyl-cyclohexyl)-acétique ;
l'acide 1α,3β,5β-(1-aminométhyl-3,5-diéthyl-cyclohexyl)-acétique, chlorhydrate ;
l'acide trans-(1R,3R)-(1-aminométhyl-3-méthyl-cyclohexyl)-acétique, chlorhydrate ;
l'acide (1-aminométhyl-2-méthyl-cyclohexyl)-acétique, chlorhydrate ;
l'acide (1-aminométhyl-3,3-diméthyl-cyclohexyl)-acétique, chlorhydrate ;
l'acide (±)-(trans)-(1-aminométhyl-3,4-diméthyl-cyclopentyl)-acétique, chlorhydrate ;
l'acide (cis/trans)-(3R)-(1-aminométhyl-3-méthyl-cyclopentyl)-acétique, chlorhydrate ;
l'acide (+)-(trans)-(1-aminométhyl-3,4-diméthyl-cyclopentyl)-acétique, chlorhydrate ;
l'acide (+)-(trans)-(1-aminométhyl-3,4-diméthyl-cyclopentyl)-acétique, chlorhydrate ;
l'acide 1α,3β,5β-(1-aminométhyl-3,5-diméthyl-cyclohexyl)-acétique, chlorhydrate ;
l'acide 1α,3β,5β-(3,5-diméthyl-1-propylaminométhyl-cyclohexyl)-acétique, sel chlorhydrate ;
l'acide 1α,3β,5β-(1-éthylaminométhyl-3,5-diméthyl-cyclohexyl)-acétique, sel chlorhydrate ;
l'acide 1α,3β,5β-[(1-benzylamino-méthyl)-3,5-diméthyl-cyclohexyl]-acétique, sel chlorhydrate ;
l'acide 1α,3β,5β-(1-diméthylaminométhyl-3,5-diméthyl-cyclohexyl)-acétique, sel chlorhydrate ;
l'acide 1α,3β,5β-(1-butylaminométhyl-3,5-diméthyl-cyclohexyl)-acétique, sel chlorhydrate ;
l'acide 1α,3β,5β-{1-[(benzyl-méthyl-amino)-méthyl]-3,5-diméthyl-cyclohexyl}-acétique, sel chlorhydrate ;
l'acide 1α,3β,5β-(3,5-diméthyl-1-méthylaminométhyl-cyclohexyl)-acétique, sel chlorhydrate ;
l'acide 1α,3β,5β-[1-(acétylamino-méthyl)-3,5-diméthyl-cyclohexyl]-acétique ;
l'acide 1α,3β,5β-[1-(isobutylamino-méthyl)-3,5-diméthyl-cyclohexyl]-acétique, sel chlorhydrate ;
l'acide 1α,3β,5β-[3,5-diméthyl-1-(phénéthylamino-méthyl)-cyclohexyl]-acétique, sel chlorhydrate ;
l'acide 1α,3β,5β-{3,5-diméthyl-1-[(3-phényl-propylamino)-méthyl]-cyclohexyl}-acétique, sel chlorhydrate ;
l'acide {1-[(cyclobutylméthyl-amino)-méthyl]-cyclohexyl]-acétique, sel chlorhydrate ;
l'acide 1α,3β,5β-[1-(isopropylamino-méthyl)-3,5-diméthyl-cyclohexyl]-acétique, sel chlorhydrate ;
l'acide 1-aminométhyl-1-cyclohexane-acétique ;
l'acide 1-aminométhyl-1-cyclopentane-acétique ;
l'acide 1-aminométhyl-1'-cyclopentane-acétique, sel de sodium ;
l'acide (hydroxyméthyl)cyclohexane-acétique, sel de sodium ;
l'acide {1-[(2-méthyl-butylamino)-méthyl]-cyclohexyl}-acétique, sel chlorhydrate ;
l'acide {1-[(4,4,4-trifluoro-butylamino)-méthyl]-cyclohexyl}-acétique, sel chlorhydrate ;
acide (1-éthylaminométhyl-cyclohexyl)-acétique, sel chlorhydrate ;
l'acide {1-[(cyclopropylméthyl-amino)-méthyl]-cyclohexyl}-acétique, sel chlorhydrate ;
l'acide {1-[(2-hydroxy-1-méthyl-éthylamino)-méthyl]-cyclohexyl}-acétique, sel chlorhydrate ;
l'acide [1-(isobutylamino-méthyl)-cyclohexyl]-acétique, sel chlorhydrate ;
l'acide (1-propylaminométhyl-cyclohexyl)-acétique, sel chlorhydrate ;
l'acide [1-(isopropylamino-méthyl)-cyclohexyl]-acétique, sel chlorhydrate ;
l'acide (1-cyclohexylaminométhyl-cyclohexyl)-acétique, sel chlorhydrate ;
l'acide [1-(benzylamino-méthyl)-cyclohexyl]-acétique, sel chlorhydrate ;
l'acide ((1R,3R)-3-méthyl-1-propylaminométhyl-cyclohexyl)-acétique, sel chlorhydrate ;
l'acide {1-[(cyclopentylméthyl-amino)-méthyl]-cyclohexyl}-acétique, sel chlorhydrate ;
l'acide {1-[(cyclohexylméthyl-amino)-méthyl]-cyclohexyl}-acétique, sel chlorhydrate ;
l'acide [1-(*tert*-butoxycarbonylamino-méthyl)-cyclohexyl]-acétique ;
l'acide [1-(acétylamino-méthyl)-cyclohexyl]-acétique ;
l'acide ((3R,5S)-1-cyclobutylaminométhyl-3,5-diméthyl-cyclohexyl)-acétique, sel chlorhydrate ;
l'acide {(3R,5S)-3,5-diméthyl-1-[(2-méthyl-butylamino)-méthyl]-cyclohexyl}-acétique, sel chlorhydrate ;
l'acide {(3R,5S)-1-[(2-hydroxy-1-méthyl-éthylamino)-méthyl]-3,5-diméthyl-cyclohexyl}-acétique, sel chlorhydrate ;
l'acide {(3R,5S)-1-[(2,2-diméthoxy-éthylamino)-méthyl]-3,5-diméthyl-cyclohexyl}-acétique, sel chlorhydrate ;
l'acide {(3R,5S)-1-[(cyclopentylméthyl-amino)-méthyl]-3,5-diméthyl-cyclohexyl}-acétique, sel chlorhydrate ;
l'acide {(3R,5S)-1-[(cyclohexylméthyl-amino)-méthyl]-3,5-diméthyl-cyclohexyl}-acétique, sel chlorhydrate ;
l'acide ((3R,5S)-1-cyclohexylaminométhyl-3,5-diméthyl-cyclohexyl)-acétique, sel chlorhydrate ;
l'acide ((3R,5S)-1-carboxyméthyl-3,5-diméthyl-cyclohexyl)-acétique,
l'acide *trans*-((3R,5S)-1-hydroxyméthyl-3,5-diméthyl-cyclohexyl)-acétique, sel chlorhydrate ;
l'acide *cis*-((3R,5S)-1-hydroxyméthyl-3,5-diméthyl-cyclohexyl)-acétique, sel chlorhydrate ;
l'acide (1-diméthylaminométhyl-cyclohexyl)-acétique, sel chlorhydrate ;
l'acide (1-butylaminométhyl-cyclohexyl)-acétique, sel chlorhydrate ;
l'acide {1-[(2,2-diméthoxy-éthylamino)-méthyl]-cyclohexyl}-acétique, sel chlorhydrate ;
l'acide (1-méthylaminométhyl-cyclohexyl)-acétique, sel chlorhydrate ; l'acide {1-[(benzyl-méthyl-amino)-méthyl]-cyclohexyl}-acétique, sel chlorhydrate ;
l'acide [1-(phénéthylamino-méthyl)-cyclohexyl]-acétique, sel chlorhydrate ;
l'acide {1-[(3-phényl-propylamino)-méthyl]-cyclohexyl}-acétique, sel chlorhydrate ;
l'acide ((3R,5S)-1-hydroxyméthyl-3,5-diméthyl-cyclohexyl)-acétique, sel de sodium ;
l'acide ((3R,5S)-1-éthylaminométhyl-3,5-diméthyl-cyclohexyl)-acétique, sel chlorhydrate ;
l'acide (1-aminométhyl-4-éthyl-cyclohexyl)-acétique, sel chlorhydrate ;
l'acide (1-aminométhyl-4-propyl-cyclohexyl)-acétique, sel chlorhydrate ;
l'acide ((3R,5S)-3,5-diméthyl-1-propylaminométhyl-cyclohexyl)-acétique, sel chlorhydrate ;
l'acide [(1R,3R)-1-(benzylamino-méthyl)-3-méthyl-cyclohexyl]-acétique, sel chlorhydrate ;
l'acide {(1R,3R)-1-[(benzyl-méthyl-amino)-méthyl]-3-méthyl-cyclohexyl}-acétique, sel chlorhydrate ;
ou
l'acide ((1R,3R)-3-méthyl-1-méthylaminométhyl-cyclohexyl)-acétique, sel chlorhydrate.

14. Utilisation d'un composé répondant à la formule IV selon la revendication 1, pour la préparation d'un produit pharmaceutique destiné au traitement de troubles neurologiques, de la dépression, de l'anxiété, de la peur panique, de la manie, de troubles bipolaires, de maladies inflammatoires, du glaucome, de la douleur ou de lésions gastro-intestinales.
